# EUROPEAN PATENT APPLICATION

(11) **EP 2 462 937 A1**
(43) Date of publication of application: **13.06.2012**
(21) Application number: 11194974.9
(22) Date of filing: 24.05.2006
(51) Int. Cl.: A61K 31/70

(54) **Modulation of LMW-PTPase Expression**

(30) Priority: 24.05.2005 US 68440005 P; 01.12.2005 US 74220705 P
(62) Divisional of application: 06771192.9
(71) Applicant: Isis Pharmaceuticals, Inc., Carlsbad, CA 92010 (US)
(72) Inventor: Pandey, Sanjay, K., Carlsbad, CA 92010 (US); McKay, Robert, Poway, CA 92064 (US); Bhanot, Sanjay, Carlsbad, CA California 92009 (US); Yu, Xing-Xian, Carlsbad, CA 92010 (US)
(74) Representative: Chapman, Desmond Mark

(57) **Abstract**

Disclosed herein are compounds, compositions and methods for modulating the expression of LMW-PTPase in a cell, tissue or animal. Also provided are methods of target validation. Also provided are uses of disclosed compounds and compositions in the manufacture of a medicament for treatment of diseases and disorders. Also provided are methods for the prevention, amelioration and/or treatment of diabetes, insulin resistance, insulin deficiency, hypercholesterolemia, hyperglycemia, dyslipidemia, hyperlipidemia, hypertriglyceridemia, and hyperfattyacidemia. In some embodiments, the diabetes is type II diabetes by administration of antisense compounds targeted to LMW-PTPase.

## Description

### FIELD OF THE INVENTION

Disclosed herein are compounds, compositions and methods for modulating the expression of LMW-PTPase in a cell, tissue or animal.

### BACKGROUND OF THE INVENTION

Considerable attention has been devoted to the characterization of tyrosine kinases and tyrosine phosphatases and their associations with disease states (Zhang, Crit Rev. Biochem. Mol. Biol., 1998, 33, 1-52). LMW-PTPase [also known ACP1; Acid phosphatase 1, soluble; Bf isoform; Bs isoform; HAAP; HCPTP; Cytoplasmic Phosphotyrosyl Protein Phosphatase; MGC3499; RCAF; Red cell acid phosphatase 1, isozyme F; Red cell acid phosphatase 1, isozyme S; acid phosphatase of erythrocyte adipocyte acid phosphatase; low molecular weight phosphotyrosine protein phosphatase; red cell acid phosphatase 1] was originally isolated as an acid phosphatases from red blood cells and was subsequently found to be expressed in many additional tissues, including placenta, brain, kidney, liver, and leukocytes (Bryson et al., Genomics, 1995, 30, 133-140; Dissing and Sveasmark, Biochim Biophys. Acta., 1990, 1041,232-242; Hopkinson et al., Nature, 1963, 199, 969-971; Wo et al., J. Bio. Chem., 1992, 267, 10856-10865). The LMW-PTPase locus was mapped to chromosome 2 at 2p23-p25 (Bryson et al., Genomics, 1995, 30, 133-140; Magenis et al., Birth. Defects Orig- Artic. Ser., 1976, 12, 326-327; Wakita et al., Hum. GeneL, 1985, 71, 259-260) and found to contain seven exons spanning 18 kilobases [Emanuel et al., Am. J. Med. Genet., 1979, 4, 167-172; Junien et al., Hum. Genet., 1979, 48, 17-21).

LMW-PTPase interacis directly with insulin stimulated insulin receptors, and negatively modulates metabolic and mitogenic insulin signaling (Chiarugi et al., Biochem. Biophys. Res- Commun-, 1997,23 8, 676-682)- A recombinant form of one LMW-PTPase isoforma, HAAPβ, dephospherylates the adipocyte lipid binding protein (ALBP), which may be a substrate for insulin receptor kinase (Shekels et al., Protein Sci., 1992, 1,710-721). Together, these findings suggest a role for LMW-PTPase in regulating insulin signaling. LMW-PTPasc also modulates flavin mononucleotide (FMN) levels, and dephosphorylates Band 3, the erythrocyte anion transporter. These functions regulate red blood cell metabolism and Integrity and account for the association between LMW-PTPase and diseases such as hemolytic favism, a disease characterized by an acute idiosyncratic hemolytic response to molecules derived from fava beans (Bottani et al., Arch. Immunol. Ther. Exp. (Warsz), 2002, 50, 95-104).

The most common genetic polymorphisms of LMW-PTPase (also known as ACP1) result in the occurrence of three alleles (ACP1*A, ACP1*B and ACP1*C) and account for six different genotypes, each of which exhibits strong variations in total enzymatic activity (Golden and Sensabaugh, Hum. Genet., 1986, 72, 340-343; Hopkinson et al., Nature, 1963, 199, 969-971). Numerous rare alleles have been reported. including ACP1 *D, E, F, G, H,1, K, M, R, TIC1, GUA, and a silent allude, ACP1*Q0 (Miller et al., Hum. Hered, 1987, 37, 371-375). Alternative splicing accounts for two isoforms which have been labeled fast (F) and slow (S), based on their electrophoretic mobility (Dissing, Biochem. Genet., 1987, 25, 901-91-8). The F and S isoforms exhibit different enzymatic properties, which, coupled with differences in the ratios of these isozymes, results in variations in activity modulation (Bottini et al., Hum. Genet., 1995, 96, 629-637).

Protein tyrosine phosphatases are signaling molecules that regulate a variety of cellular processes, including cell growth and differentiation, cell cycle progression and growth factor signaling. For example, a number of protein tyrosine phosphatases have been implicated as negative regulators of insulin signaling (Zhang, Crit. Rev. Biochem. Mol. Biol., 1998, 33, 1-52). LMW-PTPase is a phosphotyrosine phosphatase that is involved in multiple signal transduction pathways. For example, LMW-PTPase interacts directly with insulin stimulated insulin receptors, and negatively modulates metabolic and mitogenic insulin signaling (Chiarugi et al., Biochem. Biophys. Res. Commun., 1997, 238, 676-682). A recombinant form of one LMW-PTPase isoforma, HAAPβ, dephosphorylates the adipocyte lipid binding protein (ALBP), which may be a substrate for insulin receptor kinase (Shekels et al., Protein Sci., 1992, 1, 710-721). Together, these findings suggest a role for LMW-PTPase in regulating insulin signaling. LMW-PTPase also modulates flavin mononucleotide (FMN) levels, and dephosphorylates Band 3, the erythrocyte anon transporter. These functions regulate red blood cell metabolism and integrity and account for the association between LMW-PTPase and diseases such as hemolytic favism, a disease characterized by an acute idiosyncratic hemolytic response to molecules derived from fava beans (Bottini et al., Arch. Immunol. Ther. Exp. (Warsz), 2002, 50, 95-104).

The most common genetic polymorphisms of LMW-PTPase (also known as ACP1) result in the occurrence of three alleles (ACP1*A, ACP1*B and ACP1*C) and account for six different genotypes, each of which exhibits strong variations in total enzymatic activity (Golden and Sensabaugh, Hum. Genet., 1986, 72, 340-343; Hopkinson et al., Nature, 1963, 199, 969-971). Numerous rare alleles have been reported, including ACP1*D, E, F, G, H, I, K, M, R, TIC1, GUA, and a silent allele, ACP1*Q0 (Miller et al., Hum. Hered., 1987, 37, 371-375). Alternative splicing accounts for two isoforms which have been labeled fast (F) and slow (S), based on their electrophoretic mobility (Dissing, Biochem. Genet., 1987, 25, 901-918). The F and S isoforms exhibit different enzymatic properties, which, coupled with differences in the ratios of these isozymes, results in variations in activity modulation (Bottini et al., Hum. Genet., 1995, 96, 629-637). LMW-PTPase genotypes, and consequently isoform levels and total enzymatic activity, show correlation to a number of disease states. (See, e.g., Bottini et al., Arch. Immunol. Ther. Exp. (Warsz), 2002, 50, 95-104). These findings, together with the evidence that LMW-PTPase participates in insulin signaling, support a role for LMW-PTPase in metabolic disorders such as diabetes.

Given the genetic evidence for the involvement of LMW-PTPase in human disease, pharmacological modulation of LMW-PTPase activity and/or expression is an appropriate point of therapeutic intervention in these and other pathological conditions. Currently, there are no known therapeutic agents which effectively inhibit the synthesis of LMW-PTPase. Consequently, there remains a long felt need for agents capable of effectively inhibiting LMW-PTPase function.

Antisense technology is an effective means for reducing the expression of LMW-PTPase and is uniquely useful in a number of therapeutic, diagnostic, and research applications. Generally, the principle behind antisense technology is that an antisense compound hybridizes to a target nucleic acid and effects the modulation of gene expression activity, or function, such as transcription or translation. The modulation of gene expression can be achieved by, for example, target RNA degradation or occupancy-based inhibition. An example of modulation of target RNA function by degradation is RNase H-based degradation of the target RNA upon hybridization with a DNA-like antisense compound. Another example of modulation of gene expression by target degradation is RNA interference (RNAi) using small interfering RNAs (siRNAs). RNAi is a form of antisense-mediated gene silencing involving the introduction of double stranded (ds)RNA-like oligonucleotides leading to the sequence-specific reduction of targeted endogenous mRNA levels. This sequence-specificity makes antisense compounds extremely attractive as tools for target validation and gene functionalization, as well as therapeutics to selectively modulate the expression of genes involved in diseases.

### SUMMARY OF THE INVENTION

Disclosed herein are antisense compounds targeted to and hybridizable with a nucleic acid molecule encoding LMW-PTPase and which modulate the expression of LMW-PTPase. In a preferred embodiment the nucleic acid molecule encoding LMW-PTPase has a nucleotide sequence that is substantially similar to one or more of GenBank Accession Nos.: NM_004300.2, NM_007099.2, NM_177554.1, and NT_022327.13_ (SEQ ID NOS: 3-6, respectively), presented in table 1, below and incorporated herein by reference. In a further aspect, the antisense compounds are targeted to and hybridizable with a region of a nucleic acid molecule encoding LMW-PTPase. Still further, the antisense compounds are targeted to and hybridizable with a segment of a nucleic acid molecule encoding LMW-PTPase. Still further the antisense compounds are targeted to and hybridizable with a site of a nucleic acid molecule encoding LMW-PTPase.

Further disclosed herein are active target segments comprising segments of a nucleic acid molecule encoding LMW-PTPase, the active target segments being accessible to antisense hybridization, and so, suitable for antisense modulation. In one embodiment, the active target segments have been discovered herein using empirical data that is presented below, wherein at least two chimeric oligonucleotides are shown to hybridize within the active target segment and reduce expression of the target nucleic acid (hereinafter, "active antisense compound"). The at least two active antisense compounds are preferably separated by about 60 nucleobases on the nucleic acid molecule encoding LMW-PTPase. In another embodiment, antisense compounds are designed to target the active target segments and modulate expression of the nucleic acid molecule encoding LMW-PTPase.

In one aspect there are herein provided antisense compounds comprising sequences 12 to 35 nucleotides in length comprising at least two chemical modifications selected from a modified internucleoside linkage, a modified nucleobase or a modified sugar. Provided herein are chimeric oligonucleotides comprising a deoxynucleotide mid-region flanked on each of the 5' and 3' ends by wing regions, each wing region comprising at least one high affinity nucleotide.

In one embodiment there is herein provided chimeric oligonucleotides comprising ten deoxynucleotide mid-regions flanked on each of the 5' and 3' ends with wing regions comprising five 2'-O-(2-methoxyethyl) nucleotides and wherein each internucleoside linkage of the chimeric oligonucleotid is a phosphorothioate. In another embodiment there is herein provided chimeric oligonucleotides comprising fourteen deoxynucleotide mid-regions flanked on each of the 5' and 3' ends with wing regions comprising three locked nucleic acid nucleotides and wherein each internucleoside linkage of the chimeric oligonucleotide is a phosphorothioate. In a further embodiment there are hererin provided chimeric oligonucleotides comprising fourteen deoxynucleotide mid-regions flanked on each of the 5' and 3' ends by wing regions comprising two 2'-O-(2-methoxyethyl) nucleotides and wherein each internucleoside linkage of the chimeric oligonucleotide is a phosphorothioate. In a further embodiment, the antisense compounds may comprise at least one 5-methylcytosine.

Further provided are methods of modulating the expression of LMW-PTPase in cells, tissues or animals comprising contacting said cells, tissues or animals with one or more of the compounds or compositions of the present invention. For example, in one embodiment, the compounds can be used to inhibit the expression of LMW-PTPase in cells, tissues or animals. In this aspect of the invention cells are analyzed for indicators of a decrease in expression of LMW-PTPase mRNA and/or protein by direct measurement of mRNA and/or protein levels, and/or indicators of a disease or condition, such as glucose levels, lipid levels, weight, or a combination thereof.

One embodiment provides methods of lowering glucose and triglycerides. Glucose may be blood, plasma or serum glucose. Triglycerides may be blood, plasma, or serum triglycerides. Another embodiment provides methods of improving insulin sensitivity. Another embodiment provides methods of lowering cholesterol. In some embodiments, cholesterol is LDL or VLDL cholesterol. An embodiment provides methods of improving glucose tolerance.

Other embodiments are directed to methods of ameliorating or lessening the severity of a condition in an animal comprising contacting said animal with an effective amount of an antisense compound so that expression of LMW-PTPase is inhibited and measurement of one or more physical indicator of said condition indicates a lessening of the severity of said condition. In some embodiments, the conditions include, but are not limited to, diabetes, insulin resistance, insulin deficiency, hypercholesterolemia, hyperglycemia, dyslipidemia, hyperlipidemia, hypertriglyceridemia, and hyperfattyacidemia. In some embodiments, the diabetes is type II diabetes. In another embodiment, the condition is metabolic syndrome. In another embodiment, the condition is prediabetes. In another embodiment the condition is steatosis. In one embodiment, the steatosis is steatohepatitis. In another embodiment, the steatosis is NASH. In another embodiment, the condition is a cardiovascular disease. In another embodiment, the cardiovascular disease is coronary heart disease. In another embodiment, the condition is a cardiovascular risk factor.

In another embodiment, there is provided a method of decreasing hepatic glucose output in an animal comprising administering an oligomeric compound of the invention. In one embodiment, the present invention provides a method of decreasing hepatic glucose-6-phosphatase expression comprising administering an oligomeric compound of the invention. Another aspect of the present invention is a method of reducing LMW-PTPase expression in liver, fat, or in both tissues.

Also provided are methods of ameliorating or lessening the severity of a condition in an animal comprising contacting said animal with an oligomeric compound of the invention in combination with a glucose-lowering, lipid-lowering, or anti-obesity agent to achieve an additive therapeutic effect.

Also provided are methods for the prevention, amelioration, and/or treatment of diabetes, type II diabetes, prediabetes, insulin resistance, insulin deficiency, hypercholesterolemia, hyperglycemia, dyslipidemia, hyperlipidemia, hypertriglyceridemia, metabolic syndrome, hyperfattyacidemia, steatosis, steatohepatitis, NASH, cardiovascular disease, coronary heart disease, a cardiovascular risk factor or combinations thereof comprising administering at least one compound of the instant invention to an individual in need of such intervention.

The invention also provides a method of use of the compositions of the instant invention for the preparation of a medicament for the prevention, amelioration, and/or treatment disease, especially a disease associated with and including at least one indicator of diabetes, type II diabetes, prediabetes, insulin resistance, insulin deficiency, hypercholesterolemia, hyperglycemia, dyslipidemia, hyperlipidemia, hypertriglyceridemia, metabolic syndrome, hyperfattyacidemia, steatosis, steatohepatitis, NASH, cardiovascular disease, coronary heart disease, a cardiovascular risk factor or combinations thereof.

### DETAILED DESCRIPTION OF THE INVENTION

LMW-PTPase is shown to effect *in* vivo glucose levels, triglyceride levels, cholesterol levels, insulin sensitivity and glucose tolerance, therefore, LMW-PTPase is is indicated in diseases and conditions related thereto and including, but not limited to , diabetes, type II diabetes, obesity, insulin resistance, insulin deficiency, hypercholesterolemia, hyperglycemia, hyperlipidemia, hypertriglyceridemia, hyperfattyacidemia, liver steatosis, steatohepatitis, non-alcoholic steatohepatitis, metabolic syndrome, cardiovascular disease and coronary heart disease. Provided herein are antisense compounds for the prevention, amelioration, and /or treatment of diseases and conditions relating to LMW-PTPase function. As used herein, the term "prevention" means to delay or forestall onset or development of a condition or disease for a period of time from hours to days, preferably weeks to months. As used herein, the term "amelioration" means a lessening of at least one indicator of the severity of a condition or disease. The severity of indicators may be determined by subjective or objective measures which are known to those skilled in the art. As used herein, "treatment" means to administer a composition of the invention to effect an alteration or improvement of the disease or condition. Prevention, amelioration, and/or treatment may require administration of multiple doses at regular intervals, or prior to exposure to an agent to alter the course of the condition or disease.

Disclosed herein are antisense compounds, including antisense oligonucleotides and other antisense compounds for use in modulating the expression of nucleic acid molecules encoding LMW-PTPase. This is accomplished by providing antisense compounds that hybridize with one or more target nucleic acid molecules encoding LMW-PTPase. As used herein, the terms "target nucleic acid" and "nucleic acid molecule encoding LMW-PTPase " have been used for convenience to encompass RNA (including pre-mRNA and mRNA or portions thereof) transcribed from DNA encoding LMW-PTPase, and also cDNA derived from such RNA. In a preferred embodiment, the target nucleic acid is an mRNA encoding LMW-PTPase.

### Target Nucleic Acids

"Targeting" an antisense compound to a particular target nucleic acid molecule can be a multistep process. The process usually begins with the identification of a target nucleic acid whose expression is to be modulated. For example, the target nucleic acid can be a cellular gene (or mRNA transcribed from the gene) whose expression is associated with a particular disorder or disease state, or a nucleic acid molecule from an infectious agent. As disclosed herein, the target nucleic acid encodes LMW-PTPase and has a polynucleotide sequence that is substantially similar to one or more of SEQ ID NOS: 1-4.

It is also known in the art that alternative RNA transcripts can be produced from the same genomic region of DNA. These alternative transcripts are generally known as "variants." More specifically, "pre-mRNA variants" are transcripts produced from the same genomic DNA that differ from other transcripts produced from the same genomic DNA in either their start or stop position and contain both intronic and exonic sequence. Variants can result in mRNA variants including, but not limited to, those with alternate splice junctions, or alternate initiation and termination codons. Variants in genomic and mRNA sequences can result in disease. Antisense compounds targeted to such variants are within the scope of the instant invention.

In accordance with the present invention are compositions and methods for modulating the expression of LMW-PTPase. Table 1 lists the GenBank accession numbers of sequences corresponding to nucleic acid molecules encoding LMW-PTPase (nt = nucleotide), the date the version of the sequence was entered in GenBamk, and the corresponding SEQ ID NO in the instant application, when assigned, each of which is incorporated herein by reference.

**Table 1 Gene Targets**

| **Species** | **Genbank #** | **Genbank Date** | **SEQ ID NO** |
|---|---|---|---|
| Human | M83653.1 | Apr 27 1993 | 1 |
| Human | M83654.1 | Apr 27 1993 | 2 |
| Human | NM_004300.2 | Apr 24 2003 | 3 |
| Human | NM_007099.2 | Apr 24 2003 | 4 |
| Human | NM_177554.1 | Apr 24 2003 * | 5 |
| Human | nucleotides 254496 to 268683 of NT 022327.13 | Oct 7 2003 | 6 |
| Human | U25847.1 | Jan 5 1996 | 7 |
| Human | U25848.1 | Jan 5 1996 | 8 |
| Human | U25849.1 | Jan 5 1996 | 9 |
| Human | Y16846.1 | Jun 16 1998 | 10 |
| Mouse | BF167197.1 | Oct 27 2000 | 11 |
| Mouse | NM_021330.1 | Oct 23 2000 | 12 |
| Mouse | the complement of nucleotides 6757610 to 6776640 of NT_039548.2 | Oct 30 2003 | 13 |
| Mouse | Y17343.1 | Ju181998 | 14 |
| Mouse | Y17344.1 | Jul 8 1998 | 355 |
| Mouse | Y17345.1 | Jul 8 1998 | 15 |
| Rat | NM_021262.2 | Jan 1 2004 | 16 |
| Rat | the complement of nucleotides 905000 to 921000 of NW_047759.1 | Sep 22 2003 | 17 |
| Rat | XM_343044.1 | Sep 22 2003 | 18 |

| | | | |
|---|---|---|---|
| *NM_177554.1 was permanently suppressed because it is a nonsense-mediated mRNA decay (NMD) candidate. | | | |

### Modulation of Target Expression

Modulation of expression of a target nucleic acid can be achieved through alteration of any number of nucleic acid (DNA or RNA) functions. "Modulation" means a perturbation of function, for example, either an increase (stimulation or induction) or a decrease (inhibition or reduction) in expression. As another example, modulation of expression can include perturbing splice site selection of pre-mRNA processing. "Expression" includes all the functions by which a gene's coded information is converted into structures present and operating in a cell. These structures include the products of transcription and translation. "Modulation of expression" means the perturbation of such functions. The functions of RNA to be modulated can include translocation functions, which include, but are not limited to, translocation of the RNA to a site of protein translation, translocation of the RNA to sites within the cell which are distant from the site of RNA synthesis, and translation of protein from the RNA. RNA processing functions that can be modulated include, but are not limited to, splicing of the RNA to yield one or more RNA species, capping of the RNA, 3' maturation of the RNA and catalytic activity or complex formation involving the RNA which may be engaged in or facilitated by the RNA. Modulation of expression can result in the increased level of one or more nucleic acid species or the decreased level of one or more nucleic acid species, either temporally or by net steady state level. One result of such interference with target nucleic acid function is modulation of the expression of LMW-PTPase. Thus, in one embodiment modulation of expression can mean increase or decrease in target RNA or protein levels. In another embodiment modulation of expression can mean an increase or decrease of one or more RNA splice products, or a change in the ratio of two or more splice products.

The effect of antisense compounds of the present invention on target nucleic acid expression can be tested in any of a variety of cell types provided that the target nucleic acid is present at measurable levels. The effect of antisense compounds of the present invention on target nucleic acid expression can be routinely determined using, for example, PCR or Northern blot analysis. Cell lines are derived from both normal tissues and cell types and from cells associated with various disorders (e.g. hyperproliferative disorders). Cell lines derived from multiple tissues and species can be obtained from American Type Culture Collection (ATCC, Manassas, VA) and other public sources, and are well known to those skilled in the art. Primary cells, or those cells which are isolated from an animal and not subjected to continuous culture, can be prepared according to methods known in the art, or obtained from various commercial suppliers. Additionally, primary cells include those obtained from donor human subjects in a clinical setting (i.e. blood donors, surgical patients). Primary cells prepared by methods known in the art.

### Assaying Modulation of Expression

Modulation of LMW-PTPase expression can be assayed in a variety of ways known in the art. LMW-PTPase mRNA levels can be quantitated by, e.g., Northern blot analysis, competitive polymerase chain reaction (PCR), or real-time PCR. RNA analysis can be performed on total cellular RNA or poly(A)+ mRNA by methods known in the art. Methods of RNA isolation are taught in, for example, Ausubel, F.M. et al., Current Protocols in MalecularBiology, Volume 1, pp. 4.1.1-4.2.9 and 4.5.1-4.5.3, John Wiley & Sons, Inc., 1993**.**

Northern blot analysis is routine in the art and is taught in, for example, Ausubel, F.M. et al., Current Protocols in Molecular Biology, Volume 1, pp. 4.2.1-4.2.9, John Wiley & Sons, Inc., 1996. Real-time quantitative (PCR) can be conveniently accomplished using the commercially available ABI PRISM™ 7700 Sequence Detection System, available from PE-Applied Biosystems, Foster City, CA and used according to manufacturer's instructions. The method of analysis of modulation of RNA levels is not a limitation of the instant invention.

Levels of a protein encoded by LMW-PTPase can be quantitated in a variety of ways well known in the art, such as immunoprecipitation, Western blot analysis (immunoblotting), ELISA or fluorescence-activated cell sorting (FACS). Antibodies directed to a protein encoded by LMW-PTPase can be identified and obtained from a variety of sources, such as the MSRS catalog of antibodies (Aerie Corporation, Birmingham, MI), or can be prepared via conventional antibody generation methods. Methods for preparation of polyclonal antisera are taught in, for example, Ausubel, F.M. et al., Current Protocols in Molecular Biology, Volume 2, pp. 11.12.1-11.12.9, John Wiley & Sons, Inc., 1997. Preparation of monoclonal antibodies is taught in, for example, Ausubel, F.M. et al., Current Protocols in Molecular Biology, Volume 2, pp. 11.4.1-11.11.5, John Wiley & Sons, Inc., 1997.

Immunoprecipitation methods are standard in the art and can be found at, for example, Ausubel, F.M. et al., Current Protocols in Molecular Biology, Volume 2, pp. 10. 16.1-10.16.11, John Wiley & Sons, Inc., 1998. Western blot (immunoblot) analysis is standard in the art and can be found at, for example, Ausubel, F.M. et al., Current Protocols in Molecular Biology, Volume 2, pp. 10.8.1-10.8.21, John Wiley & Sons, Inc., 1997.

### Active Target Segments

The locations on the target nucleic acid defined by having at least two active antisense compounds targeted thereto are referred to as "active target segments." An active target segment is defined by one of the at least two active antisense compounds hybridizing at the 5' end of the active target segment and the other hybridizing at the 3' end of the active target segment. Additional active antisense compounds may hybridize within this defined active target segment. The compounds are preferably separated by no more than about 60 nucleotides on the target sequence, more preferably no more than about 30 nucleotides on the target sequence, even more preferably the compounds are contiguous, most preferably the compounds are overlapping. There may be substantial variation in activity (e.g., as defined by percent inhibition) of the antisense compounds within an active target segment. Active antisense compounds are those that modulate the expression of their target RNA. In one of the assays provided herein, active antisense compounds inhibit expression of their target RNA at least 10%, preferably 20%. In a preferred embodiment, at least about 50%, preferably about 70% of the oligonucleotides targeted to the active target segment modulate expression of their target RNA at least 40%. In a more preferred embodiment, the level of inhibition required to define an active antisense compound is defined based on the results from the screen used to define the active target segments. One ordinarily skilled in the art will readily understand that values received from any single assay will vary in comparison to other similar assays due to assay-to-assay conditions.

### Hybridization

As used herein, "hybridization" means the pairing of complementary strands of antisense compounds to their target sequence. While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases). For example, the natural base adenine is complementary to the natural nucleobases thymidine and uracil which pair through the formation of hydrogen bonds. The natural base guanine is complementary to the natural base 5-methyl cytosine and the artificial base known as a G-clamp. Hybridization can occur under varying circumstances.

An antisense compound is specifically hybridizable when there is a sufficient degree of complementarity to avoid non-specific binding of the antisense compound to non-target nucleic acid sequences under conditions in which specific binding is desired, i.e., under physiological conditions in the case of *in vivo* assays or therapeutic treatment, and under conditions in which assays are performed in the case of *in vitro* assays.

As used herein, "stringent hybridization conditions" or "stringent conditions" refers to conditions under which an antisense compound will hybridize to its target sequence, but to a minimal number of other sequences. Stringent conditions are sequence-dependent and will be different in different circumstances, and "stringent conditions" under which antisense compounds hybridize to a target sequence are determined by the nature and composition of the antisense compounds and the assays in which they are being investigated.

### Complementarity

"Complementarity," as used herein, refers to the capacity for precise pairing between two nucleobases on either two oligomeric compound strands or an antisense compound with its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be a complementary position. The antisense compound and the further DNA or RNA are complementary to each other when a sufficient number of complementary positions in each molecule are occupied by nucleobases which can hydrogen bond with each other. Thus, "specifically hybridizable" and "complementary" are terms which are used to indicate a sufficient degree of precise pairing or complementarity over a sufficient number of nucleobases such that stable and specific binding occurs between the antisense compound and a target nucleic acid.

Those in the art understand that for an antisense compound to be active it need not be 100% complementary to the target nucleic acid site wherein it hybridizes. Often, once an antisense compound has been identified as an active antisense compound, the compounds are routeinly modified to include mismatched nucleobases compared to the sequence of the target nucleic acid site. The art teaches methods for introducing mismatches into an antisense compound without substantially altering its activity. Antisense compounds may be able to tolerate up to about 20% mismatches without significant alteration of activity, particularly so when a high affinity modification accompanies the mismatches.

### Identity

Antisense compounds, or a portion thereof, may have a defined percent identity to a SEQ ID NO, or a compound having a specific compound number. As used herein, a sequence is identical to the sequence disclosed herein if it has the same nucleobase pairing ability. For example, a RNA which contains uracil in place of thymidine in the disclosed sequences of the instant invention would be considered identical as they both pair with adenine. Similarly, a G-clamp modified heterocyclic base would be considered identical to a cytosine or a 5-Me cytosine in the sequences of the instant application as it pairs with a guanine. This identity may be over the entire length of the oligomeric compound, or in a portion of the antisense compound (e.g., nucleobases 1-20 of a 27-mer may be compared to a 20-mer to determine percent identity of the oligomeric compound to the SEQ ID NO.) It is understood by those skilled in the art that an antisense compound need not have an identical sequence to those described herein to function similarly to the antisense compound described herein. Shortened versions of antisense compound taught herein, or non-identical versions of the antisense compound taught herein fall within the scope of the invention. Non-identical versions are those wherein each base does not have the same pairing activity as the antisense compounds disclosed herein. Bases do not have the same pairing activity by being shorter or having at least one abasic site. Alternatively, a non-identical version can include at least one base replaced with a different base with different pairing activity (e.g., G can be replaced by C, A, or T). Percent identity is calculated according to the number of bases that have identical base pairing corresponding to the SEQ ID NO or antisense compound to which it is being compared. The non-identical bases may be adjacent to each other, dispersed through out the oligonucleotide, or both.

For example, a 16-mer having the same sequence as nucleobases 2-17 of a 20-mer is 80% identical to the 20-mer. Alternatively, a 20-mer containing four nucleobases not identical to the 20-mer is also 80% identical to the 20-mer. A 14-mer having the same sequence as nucleobases 1-14 of an 18-mer is 78% identical to the 18-mer. Such calculations are well within the ability of those skilled in the art.

The percent identity is based on the percent of nucleobases in the original sequence present in a portion of the modified sequence. Therefore, a 30 nucleobase antisense compound comprising the full sequence of the complement of a 20 nucleobase active target segment would have a portion of 100% identity with the complement of the 20 nucleobase active target segment, while further comprising an additional 10 nucleobase portion. In the context of the invention, the complement of an active target segment may constitute a single portion. In a preferred embodiment, the oligonucleotides of the instant invention are at least about 80%, more preferably at least about 85%, even more preferably at least about 90%, most prefereably at least 95% identical to at least a portion of the complement of the active target segments presented herein.

It is well known by those skilled in the art that it is possible to increase or decrease the length of an antisense compound and/or introduce mismatch bases without eliminating activity. For example, in Woolf et al. (Proc. Natl. Acad. Sci. USA 89:7305-7309, 1992, incorporated herein by reference), a series of ASOs 13-25 nucleobases in length were tested for their ability to induce cleavage of a target RNA in an oocyte injection model. ASOs 25 nucleobases in length with 8 or 11 mismatch bases near the ends of the ASOs were able to direct specific cleavage of the target mRNA, albeit to a lesser extent than the ASOs that contained no mismatches. Similarly, target specific cleavage was achieved using a 13 nucleobase ASOs, including those with 1 or 3 mismatches. Maher and Dolnick (Nuc. Acid. Res. 16:3341-3358,1988, incorporated herein by reference) tested a series of tandem 14 nucleobase ASOs, and a 28 and 42 nucleobase ASOs comprised of the sequence of two or three of the tandem ASOs, respectively, for their ability to arrest translation of human DHFR in a rabbit reticulocyte assay. Each of the three 14 nucleobase ASOs alone were able to inhibit translation, albeit at a more modest level than the 28 or 42 nucleobase ASOs.

### Therapeutics

Antisense compounds of the invention can be used to modulate the expression of LMW-PTPase in an animal, such as a human. In one non-limiting embodiment, the methods comprise the step of administering to said animal in need of therapy for a disease or condition associated with LMW-PTPase an effective amount of an antisense compound that inhibits expression of LMW-PTPase. A disease or condition associated with LMW-PTPase includes, but is not limited to, diabetes, type II diabetes, obesity, insulin resistance, insulin deficiency, hypercholesterolemia, hyperglycemia, hyperlipidemia, hypertriglyceridemia, hyperfattyacidemia, liver steatosis, steatohepatitis, non-alcoholic steatohepatitis, metabolic syndrome, cardiovascular disease and coronary heart disease. The diseases or conditions are associated with clinical indicators that include, but are not limited to blood glucose levels, blood lipid levels, hepatic lipid levels, insulin levels, cholesterol levels, transaminase levels, electrocardiogram, glucose uptake, gluconeogenesis, insulin sensitivity, body weight and combinations thereof. In one embodiment, the antisense compounds of the present invention effectively inhibit the levels or function of LMW-PTPase RNA. Because reduction in LMW-PTPase mRNA levels can lead to alteration in LMW-PTPase protein products of expression as well, such resultant alterations can also be measured. Antisense compounds of the present invention that effectively inhibit the level or function of LMW-PTPase RNA or protein products of expression are considered an active antisense compounds. In one embodiment, the antisense compounds of the invention inhibit the expression of LMW-PTPase causing a reduction of RNA by at least 30%, by at least 40%, by at least 50%, by at least 60%, by at least 70%, by at least 75%, by at least 80%, by at least 85%, by at least 90%, by at least 95%, by at least 98%, by at least 99%, or by 100%.

For example, the reduction of the expression of LMW-PTPase can be measured in a bodily fluid, tissue or organ of the animal. Methods of obtaining samples for analysis, such as body fluids (e.g., blood), tissues (e.g., biopsy), or organs, and methods of preparation of the samples to allow for analysis are well known to those skilled in the art. Methods for analysis of RNA and protein levels are discussed above and are well known to those skilled in the art. The effects of treatment can be assessed by measuring biomarkers associated with the LMW-PTPase expression in the aforementioned fluids, tissues or organs, collected from an animal contacted with one or more compounds of the invention, by routine clinical methods known in the art. These biomarkers include but are not limited to: liver transaminases, bilirubin, albumin, blood urea nitrogen, creatine and other markers of kidney and liver function; glucose levels, triglyceride levels, insulin levels, fatty acid levels, cholesterol levels, electrocardiogram, glucose uptake, gloconeogenesis, insulin sensitivity and body weight, and other markers of diabetes, type II diabetes, obesity, insulin resistance, insulin deficiency, hypercholesterolemia, hyperglycemia, hyperlipidemia, hypertriglyceridemia, hyperfattyacidemia, liver steatosis, steatohepatitis, non-alcoholic steatohepatitis, metabolic syndrome, cardiovascular disease and coronary heart disease. Additionally, the effects of reatment can be assessed using non-invasive indicators of improved disease state or condition, such as electrocardiogram, body weight, and the like.

The antisense compounds of the present invention can be utilized in pharmaceutical compositions by adding an effective amount of a compound to a suitable pharmaceutically acceptable diluent or carrier. Acceptable carriers and dilutents are well known to those skilled in the art. Selection of a dilutent or carrier is based on a number of factors, including, but not limited to, the solubility of the compound and the route of administration. Such considerations are well understood by those skilled in the art. In one aspect, the compounds of the present invention inhibit the expression of LMW-PTPase. The compounds of the invention can also be used in the manufacture of a medicament for the treatment of diseases and disorders related to LMW-PTPase expression by restoring glucose levels, triglyceride levels, insulin levels, fatty acid levels, cholesterol levels, glucose uptake, gloconeogenesis and insulin sensitivity to non-diesase state profiles.

Methods whereby bodily fluids, organs or tissues are contacted with an effective amount of one or more of the antisense compounds or compositions of the invention are also contemplated. Bodily fluids, organs or tissues can be contacted with one or more of the compounds of the invention resulting in modulation of LMW-PTPase expression in the cells of bodily fluids, organs or tissues.

### Kits, Research Reagents, and Diagnostics

The antisense compounds of the present invention can be utilized for diagnostics, and as research reagents and kits. Furthermore, antisense compounds, which are able to inhibit gene expression with specificity, are often used by those of ordinary skill to elucidate the function of particular genes or to distinguish between functions of various members of a biological pathway.

For use in kits and diagnostics, the antisense compounds of the present invention, either alone or in combination with other compounds or therapeutics, can be used as tools in differential and/or combinatorial analyses to elucidate expression patterns of a portion or the entire complement of genes expressed within cells and tissues. Methods of gene expression analysis are well known to those skilled in the art.

### Antisense Compounds

The term "antisense compound" refers to a polymeric structure capable of hybridizing to a region of a nucleic acid molecule. As is used herein, the term "active antisense compound" is an antisense compound that has been shown to hybridize with the target nucleic acid and modulate it expression. Generally, antisense compounds comprise a plurality of monomeric subunits linked together by internucleoside linking groups and/or internucleoside linkage mimetics. Each of the monomeric subunits comprises a sugar, abasic sugar, modified sugar, or a sugar mimetic, and except for the abasic sugar includes a nucleobase, modified nucleobase or a nucleobase mimetic. Preferred monomeric subunits comprise nucleosides and modified nucleosides. An antisense compound is at least partially complementary to the region of a target nucleic acid molecule to which it hybridizes and which modulates (increases or decreases) its expression. This term includes oligonucleotides, oligonucleosides, oligonucleotide analogs, oligonucleotide mimetics, antisense compounds, antisense oligomeric compounds, and chimeric combinations of these. An "antisense oligonucleotide" is an antisense compound that is a nucleic acid-based oligomer. An antisense oligonucleotide can, in some cases, include one or more chemical modifications to the sugar, base, and/or internucleoside linkages. Nonlimiting examples of antisense compounds include antisense compounds, antisense oligonucleotides, external guide sequence (EGS) oligonucleotides, alternate splicers, and siRNAs. As such, these compounds can be introduced in the form of single-stranded, double-stranded, circular, branched or hairpins and can contain structural elements such as internal or terminal bulges or loops. In some embodiments it is desirous to take advantage of alternate antisense mechanisms (such as RNAi). Antisense compounds that use these alternate mechanisms may optionally comprise a second compound which is complementary to the antisense compound. In other words, antisense double-stranded compounds can be two strands hybridized to form double-stranded compounds or a single strand with sufficient self complementarity to allow for hybridization and formation of a fully or partially double-stranded compound. The compounds of the instant invention are not auto-catalytic. As used herein, "auto-catalytic" means a compound has the ability to promote cleavage of the target RNA in the absence of accessory factors, e.g. proteins.

In one embodiment of the invention, double-stranded antisense compounds encompass short interfering RNAs (siRNAs). As used herein, the term "siRNA" is defined as a double-stranded compound having a first and second strand, each strand having a central portion and two independent terminal portions. The central portion of the first strand is complementary to the central portion of the second strand, allowing hybridization of the strands. The terminal portions are independently, optionally complementary to the corresponding terminal portion of the complementary strand. The ends of the strands may be modified by the addition of one or more natural or modified nucleobases to form an overhang

Each strand of the siRNA duplex may be from about 12 to about 35 nucleobases. In a preferred embodiment, each strand of the siRNA duplex is about 17 to about 25 nucleobases. The two strands may be fully complementary (i.e., form a blunt ended compound), or include a 5' or 3' overhang on one or both strands. Double-stranded compounds can be made to include chemical modifications as discussed herein.

In one embodiment of the invention, the antisense compound comprises a single stranded oligonucleotide. In some embodiments of the invention the antisense compound contains chemical modifications. In a preferred embodiment, the antisense compound is a single stranded, chimeric oligonucleotide wherein the modifications of sugars, bases, and internucleoside linkages are independently selected.

The antisense compounds may comprise a length from about 12 to about 35 nucleobases (i.e. from about 12 to about 35 linked nucleosides). In other words, a single-stranded compound of the invention comprises from about 12 to about 35 nucleobases, and a double-stranded antisense compound of the invention (such as a siRNA, for example) comprises two strands, each of which is independently from about 12 to about 35 nucleobases. This includes oligonucleotides 15 to 35 and 16 to 35 nucleobases in length. Contained within the antisense compounds of the invention (whether single or double stranded and on at least one strand) are antisense portions. The "antisense portion" is that part of the antisense compound that is designed to work by one of the aforementioned antisense mechanisms. One of ordinary skill in the art will appreciate that about 12 to about 35 nucleobases includes 12, 13, 14, 15, 16, 17, 18, 19, 20, 21, 22, 23, 24, 25, 26, 27, 28, 29, 30, 31, 32, 33, 34, or 35 nucleobases. For convenience we describe antisense compounds, but one ordinarily skilled in the art will understand that analogues and mimetics can have a length within this same range.

Antisense compounds about 12 to 35 nucleobases in length, preferably about 15 to 35 nucleobases in length, comprising a stretch of at least eight (8), preferably at least 12, more preferably at least 15 consecutive nucleobases selected from within the active target regions are considered to be suitable antisense compounds as well.

Modifications can be made to the antisense compounds of the instant invention and may include conjugate groups attached to one of the termini, selected nucleobase positions, sugar positions or to one of the internucleoside linkages. Possible modifications include, but are not limited to, 2'-fluoro (2'-F), 2'-OMethyl (2'-OMe), 2'-Methoxy ethoxy (2'-MOE) sugar modifications, inverted abasic caps, deoxynucleobases, and bicyclice nucleobase analogs such as locked nucleic acids (LNA.sup.TM) and ENA.

### Chemical Modifications

As is known in the art, a nucleoside is a base-sugar combination. The base portion of the nucleoside is normally a heterocyclic base (sometimes referred to as a "nucleobase" or simply a "base"). The two most common classes of such heterocyclic bases are the purines and the pyrimidines. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. In forming oligonucleotides, the phosphate groups covalently link adjacent nucleosides to one another to form a linear polymeric compound. Within oligonucleotides, the phosphate groups are commonly referred to as forming the internucleoside backbone of the oligonucleotide. The normal linkage or backbone of RNA and DNA is a 3' to 5' phosphodiester linkage. It is often preferable to include chemical modifications in oligonucleotides to alter their activity. Chemical modifications can alter oligonucleotide activity by, for example: increasing affinity of an antisense oligonucleotide for its target RNA, increasing nuclease resistance, and/or altering the pharmacokinetics of the oligonucleotide. The use of chemistries that increase the affinity of an oligonucleotide for its target can allow for the use of shorter oligonucleotide compounds.

The term "nucleobase" or "heterocyclic base moiety" as used herein, refers to the heterocyclic base portion of a nucleoside. In general, a nucleobase is any group that contains one or more atom or groups of atoms capable of hydrogen bonding to a base of another nucleic acid. In addition to "unmodified" or "natural" nucleobases such as the purine nucleobases adenine (A) and guanine (G), and the pyrimidine nucleobases thymine (T), cytosine (C) and uracil (U), many modified nucleobases or nucleobase mimetics known to those skilled in the art are amenable to the present invention. The terms modified nucleobase and nucleobase mimetic can overlap but generally a modified nucleobase refers to a nucleobase that is fairly similar in structure to the parent nucleobase, such as for example a 7-deaza purine or a 5-methyl cytosine, whereas a nucleobase mimetic would include more complicated structures, such as for example a tricyclic phenoxazine nucleobase mimetic. Methods for preparation of the above noted modified nucleobases are well known to those skilled in the art.

Antisense compounds may also contain one or more nucleosides having modified sugar moieties. The furanosyl sugar ring of a nucleoside can be modified in a number of ways including, but not limited to, addition of a substituent group, bridging of two non-geminal ring atoms to form a bicyclic nucleic acid (BNA) and substitution of an atom or group such as -S-, -N(R)- or -C(R₁)(R₂) for the ring oxygen at the 4'-position. Modified sugar moieties are well known and can be used to alter, typically increase, the affinity of the antisense compound for its target and/or increase nuclease resistance. A representative list of preferred modified sugars includes but is not limited to bicyclic modified sugars (BNA's), including LNA and ENA (4'-(CH₂)₂-O-2' bridge); and substituted sugars, especially 2'-substituted sugars having a 2'-F, 2'-OCH₂ or a 2'-o(CH₂)₂OCH₃ substituent group. Sugars can also be replaced with sugar mimetic groups among others. Methods for the preparations of modified sugars are well known to those skilled in the art.

Internucleoside linking groups link the nucleosides or otherwise modified monomer units together thereby forming an antisense compound. The two main classes of internucleoside linking groups are defined by the presence or absence of a phosphorus atom. Representative phosphorus containing internucleoside linkages include, but are not limited to, phosphodiesters, phosphotriesters, methylphosphonates, phosphoramidate, and phosphorothioates. Representative non-phosphorus containing internucleoside linking groups include, but are not limited to, methylenemethylimino (-CH.sub.2-N(CH.sub.3)-O-CH.sub.2-), thiodiester (-O-C(O)-S-), thionocarbamate (-O-C(O)(NH)-S-); siloxane (-O-Si(H).sub.2-O-); and N,N'-dimethylhydrazine (-CH.sub.2-N(CH.sub.3)-N(CH.sub.3)-). Antisense compounds having non-phosphorus internucleoside linking groups are referred to as oligonucleosides. Modified internucleoside linkages, compared to natural phosphodiester linkages, can be used to alter, typically increase, nuclease resistance of the antisense compound. Internucleoside linkages having a chiral atom can be prepared racemic, chiral, or as a mixture. Representative chiral internucleoside linkages include, but are not limited to, alkylphosphonates and phosphorothioates. Methods of preparation of phosphorous-containing and non-phosphorous-containing linkages are well known to those skilled in the art.

As used herein the term "mimetic" refers to groups that are substituted for a sugar, a nucleobase, and/ or internucleoside linkage. Generally, a mimetic is used in place of the sugar or sugar-internucleoside linkage combination, and the nucleobase is maintained for hybridization to a selected target. Representative examples of a sugar mimetic include, but are not limited to, cyclohexenyl or morpholino. Representative examples of a mimetic for a sugar-intemucleoside linkage combination include, but are not limited to, peptide nucleic acids (PNA) and morpholino groups linked by uncharged achiral linkages. In some instances a mimetic is used in place of the nucleobase. Representative nucleobase mimetics are well known in the art and include, but are not limited to, tricyclic phenoxazine analogs and universal bases (Berger et al., Nuc Acid Res. 2000, 28:2911-14, incorporated herein by reference). Methods of synthesis of sugar, nucleoside and nucleobase mimetics are well known to those skilled in the art.

As used herein the term "nucleoside" includes, nucleosides, abasic nucleosides, modified nucleosides, and nucleosides having mimetic bases and/or sugar groups.

In the context of this disclosure, the term "oligonucleotide" refers to an oligomeric compound which is an oligomer or polymer of ribonucleic acid (RNA) or deoxyribonucleic acid (DNA). This term includes oligonucleotides composed of naturally- and non-naturally-occurring nucleobases, sugars and covalent internucleoside linkages, possibly further including non-nucleic acid conjugates.

Provided are compounds having reactive phosphorus groups useful for forming internucleoside linkages including for example phosphodiester and phosphorothioate internucleoside linkages. Methods of preparation and/or purification of precursors or antisense compounds of the instant invention are not a limitation of the compositions or methods of the invention. Methods for synthesis and purification of DNA, RNA, and the antisense compounds are well known to those skilled in the art.

As used herein the term "chimeric antisense compound" refers to an antisense compound, having at least one sugar, nucleobase and/or internucleoside linkage that is differentially modified as compared to the other sugars, nucleobases and internucleoside linkages within the same oligomeric compound. The remainder of the sugars, nucleobases and internucleoside linkages can be independently modified or unmodified. In general a chimeric oligomeric compound will have modified nucleosides that can be in isolated positions or grouped together in regions that will define a particular motif. Any combination of modifications and or mimetic groups can comprise a chimeric oligomeric compound.

Chimeric antisense compounds typically contain at least one region modified so as to confer increased resistance to nuclease degradation, increased cellular uptake, and/or increased binding affinity for the target nucleic acid. An additional region of the oligomeric compound may serve as a substrate for enzymes capable of cleaving RNA:DNA or RNA:RNA hybrids. By way of example, RNase H is a cellular endonuclease that cleaves the RNA strand of an RNA:DNA duplex. Activation of RNase H, therefore, results in cleavage of the RNA target, thereby greatly enhancing the efficiency of inhibition of gene expression. Consequently, comparable results can often be obtained with shorter antisense compounds when chimeras are used, compared to for example phosphorothioate deoxyoligonucleotides hybridizing to the same target region. Cleavage of the RNA target can be routinely detected by gel electrophoresis and, if necessary, associated nucleic acid hybridization techniques known in the art.

Certain chimeric as well as non-chimeric antisense compounds can be further described as having a particular motif. As used herein, the term "motif" refers to the orientation of modified sugar moieties and/or sugar mimetic groups in an antisense compound relative to like or differentially modified or unmodified nucleosides. As used herein, the terms "sugars", "sugar moieties" and "sugar mimetic groups' are used interchangeably. Such motifs include, but are not limited to, gapped motifs, alternating motifs, fully modified motifs, hemimer motifs, blockmer motifs, and positionally modified motifs. The sequence and the structure of the nucleobases and type of internucleoside linkage is not a factor in determining the motif of an antisense compound.

As used herein, the term "gapped motif" refers to an antisense compound comprising a contiguous sequence of nucleosides that is divided into 3 regions, an internal region (gap) flanked by two external regions (wings). The regions are differentiated from each other at least by having differentially modified sugar groups that comprise the nucleosides. In some embodiments, each modified region is uniformly modified (e.g. the modified sugar groups in a given region are identical); however, other motifs can be applied to regions. For example, the wings in a gapmer could have an alternating motif. The nucleosides located in the gap of a gapped antisense compound have sugar moieties that are different than the modified sugar moieties in each of the wings.

As used herein, the term "alternating motif" refers to an antisense compound comprising a contiguous sequence of nucleosides comprising two differentially sugar modified nucleosides that alternate for essentially the entire sequence of the antisense compound, or for essentially the entire sequence of a region of an antisense compound.

As used herein, the term "fully modified motif" refers to an antisense compound comprising a contiguous sequence of nucleosides wherein essentially each nucleoside is a sugar modified nucleoside having uniform modification.

As used herein, the term "hemimer motif" refers to a sequence of nucleosides that have uniform sugar moieties (identical sugars, modified or unmodified) and wherein one of the 5'-end or the 3'-end has a sequence of from 2 to 12 nucleosides that are sugar modified nucleosides that are different from the other nucleosides in the hemimer modified antisense compound.

As used herein, the term "blockmer motif" refers to a sequence of nucleosides that have uniform sugars (identical sugars, modified or unmodified) that is internally interrupted by a block of sugar modified nucleosides that are uniformly modified and wherein the modification is different from the other nucleosides. Methods of preparation of chimeric oligonucleotide compounds are well known to those skilled in the art.

As used herein, the term "positionally modified motif" comprises all other motifs. Methods of preparation of positionally modified oligonucleotide compounds are well known to those skilled in the art.

The compounds described herein contain one or more asymmetric centers and thus give rise to enantiomers, diastereomers, and other stereoisomeric configurations that may be defined, in terms of absolute stereochemistry, as (R) or (S), .alpha. or .beta., or as (D) or (L) such as for amino acids et al. This is meant to include all such possible isomers, as well as their racemic and optically pure forms.

In one aspect, antisense compounds are modified by covalent attachment of one or more conjugate groups. Conjugate groups may be attached by reversible or irreversible attachments. Conjugate groups may be attached directly to antisense compounds or by use of a linker. Linkers may be mono- or bifunctional linkers. Such attachment methods and linkers are well known to those skilled in the art. In general, conjugate groups are attached to antisense compounds to modify one or more properties. Such considerations are well known to those skilled in the art.

### Oligomer Synthesis

Oligomerization of modified and unmodified nucleosides can be routinely performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713).

Antisense compounds can be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives. The invention is not limited by the method of antisense compound synthesis.

### Oligomer Purification and Analysis

Methods of oligonucleotide purification and analysis are known to those skilled in the art. Analysis methods include capillary electrophoresis (CE) and electrospray-mass spectroscopy. Such synthesis and analysis methods can be performed in multi-well plates. The compositions and methods disclosed herein not limited by the method of oligomer purification.

### Salts, prodrugs and bioequivalents

The antisense compounds may comprise any pharmaceutically acceptable salts, esters, or salts of such esters, or any other functional chemical equivalent which, upon administration to an animal including a human, is capable of providing (directly or indirectly) the biologically active metabolite or residue thereof. Accordingly, for example, the disclosure is also drawn to prodrugs and pharmaceutically acceptable salts of the antisense compounds, pharmaceutically acceptable salts of such prodrugs, and other bioequivalents.

The term "prodrug" indicates a therapeutic agent that is prepared in an inactive or less active form that is converted to an active form (i.e., drug) within the body or cells thereof by the action of endogenous enzymes, chemicals, and/or conditions. In particular, prodrug versions of the oligonucleotides of the invention are prepared as SATE ((S-acetyl-2-thioethyl) phosphate) derivatives according to the methods disclosed in WO 93/24510 or WO 94/26764. Prodrugs can also include antisense compounds wherein one or both ends comprise nucleobases that are cleaved (e.g., phosphodiester backbone linkages) to produce the smaller active compound.

The term "pharmaceutically acceptable salts" refers to physiologically and pharmaceutically acceptable salts of the antisense compounds: i.e., salts that retain the desired biological activity of the parent compound and do not impart undesired toxicological effects thereto. Sodium salts of antisense oligonucleotides are useful and are well accepted for therapeutic administration to humans. In another embodiment, sodium salts of dsRNA compounds are also provided.

### Formulations

The antisense compounds may also be admixed, encapsulated, conjugated or otherwise associated with other molecules, molecule structures or mixtures of compounds.

The antisense compounds may also include pharmaceutical compositions and formulations. The pharmaceutical compositions of the present invention may be administered in a number of ways depending upon whether local or systemic treatment is desired and upon the area to be treated.

The pharmaceutical formulations, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers, finely divided solid carriers, or both, and then, if necessary, shaping the product (e.g., into a specific particle size for delivery).

A "pharmaceutical carrier" or "excipient" can be a pharmaceutically acceptable solvent, suspending agent or any other pharmacologically inert vehicle for delivering one or more nucleic acids to an animal and are known in the art. The excipient may be liquid or solid and is selected, with the planned manner of administration in mind, so as to provide for the desired bulk, consistency, etc., when combined with a nucleic acid and the other components of a given pharmaceutical composition.

### Combinations

Compositions provided herein can contain two or more antisense compounds. In another related embodiment, compositions can contain one or more antisense compounds, particularly oligonucleotides, targeted to a first nucleic acid and one or more additional antisense compounds targeted to a second nucleic acid target. Alternatively, compositions can contain two or more antisense compounds targeted to different regions of the same nucleic acid target. Two or more combined compounds may be used together or sequentially. Compositions of the instant invention can also be combined with other non-antisense compound therapeutic agents.

### Nonlimiting disclosure and incorporation by reference

While certain compounds, compositions and methods have been described with specificity in accordance with certain embodiments, the following examples serve only as illustrations of the compounds and methods and are not intended to limit the claims of the invention. Each of the references, GenBank accession numbers, and the like recited in the present application is incorporated herein by reference in its entirety.

### Example 1: Cell Types and Transfection Methods

Cell types- The effect of oligomeric compounds on target nucleic acid expression was tested in one or more of the following cell types.

*A549:* The human lung carcinoma cell line A549 was obtained from the American Type Culture Collection (Manassas, VA). A549 cells were routinely cultured in DMEM, high glucose (Invitrogen Life Technologies, Carlsbad, CA) supplemented with 10% fetal bovine serum, 100 units per ml penicillin, and 100 micrograms per ml streptomycin (Invitrogen Life Technologies, Carlsbad, CA). Cells were routinely passaged by trypsinization and dilution when they reached approximately 90% confluence. Cells were seeded into 96-well plates (Falcon-Primaria #3872) at a density of approximately 5000 cells/well for use in oligomeric compound transfection experiments.

*b.END_{:}* The mouse brain endothelial cell line b.END was obtained from Dr. Werner Risau at the Max Plank Institute (Bad Nauheim, Germany). b.END cells were routinely cultured in DMEM, high glucose (Invitrogen Life Technologies, Carlsbad, CA) supplemented with 10% fetal bovine serum (Invitrogen Life Technologies, Carlsbad, CA). Cells were routinely passaged by trypsinization and dilution when they reached approximately 90% confluence. Cells were seeded into 96-well plates (Falcon-Primaria #353872, BD Biosciences, Bedford, MA) at a density of approximately 3000 cells/well for use in oligomeric compound transfection experiments.

*A10:* The rat aortic smooth muscle cell line A10 was obtained from the American Type Culture Collection (Manassas, VA). A10 cells were routinely cultured in DMEM, high glucose (American Type Culture Collection, Manassas, VA) supplemented with 10% fetal bovine serum (Invitrogen Life Technologies, Carlsbad, CA). Cells were routinely passaged by trypsinization and dilution when they reached approximately 80% confluence. Cells were seeded into 96-well plates (Falcon-Primaria #3872) at a density of approximately 2500 cells/well for use in oligomeric compound transfection experiments.

*Primary Mouse Hepatocytes:* Primary mouse hepatocytes were prepared from CD-1 mice purchased from Charles River Labs. Primary mouse hepatocytes were routinely cultured in Hepatocyte Attachment Media supplemented with 10% fetal bovine serum, 1% penicillin/ streptomycin, 1% antibiotic-antimitotic (Invitrogen Life Technologies, Carlsbad, CA) and 10nM bovine insulin (Sigma-Aldrich, St. Louis, MO). Cells were seeded into 96-well plates (Falcon-Primaria #353872, BD Biosciences, Bedford, MA) coated with 0.1mg/ml collagen at a density of approximately 10,000 cells/well for use in oligomeric compound transfection experiments.

*Primary Rat Hepatocytes:* Primary rat hepatocytes are prepared from Sprague-Dawley rats purchased from Charles River Labs (Wilmington, MA) and are routinely cultured in DMEM, high glucose (Invitrogen Life Technologies, Carlsbad, CA) supplemented with 10% fetal bovine serum (Invitrogen Life Technologies, Carlsbad, CA), 100 units per mL penicillin, and 100 micro.g/mL streptomycin (Invitrogen Life Technologies, Carlsbad, CA). Cells are seeded into 96-well plates (Falcon-Primaria #353872, BD Biosciences, Bedford, MA) at a density of 4000-6000 cells/well treatment with the oligomeric compounds of the invention.

For Northern blotting or other analysis, cells may be seeded onto 100 mm or other standard tissue culture plates and treated similarly, using appropriate volumes of medium and oligonucleotide.

Treatment with oligomeric compounds: When cells reach appropriate confluency, they are treated with oligonucleotide using a transfection method as described.

*Lipofectin*™ When cells reached 65-75% confluency, they were treated with oligonucleotide. Oligonucleotide was mixed with LIPOFECTIN™ Invitrogen Life Technologies, Carlsbad, CA) in Opti-MEM™-1 reduced serum medium (Invitrogen Life Technologies, Carlsbad, CA) to achieve the desired concentration of oligonucleotide and a LIPOFECTIN™ concentration of 2.5 or 3 µg/mL per 100 nM oligonucleotide. This transfection mixture was incubated at room temperature for approximately 0.5 hours. For cells grown in 96-well plates, wells were washed once with 100 µL OPTI-MEM™-1 and then treated with 130 µL of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture was replaced with fresh culture medium. Cells were harvested 16-24 hours after oligonucleotide treatment.

*CYTOFECTIN*™: When cells reached 65-75% confluency, they were treated with oligonucleotide. Oligonucleotide was mixed with CYTOFECTIN™ (Gene Therapy Systems, San Diego, CA) in OPTI-MEM-1.sup.TM reduced serum medium (Invitrogen Life Technologies, Carlsbad, CA) to achieve the desired concentration of oligonucleotide and a CYTOFECTIN™ concentration of 2 or 4 micro.g/mL per 100 nM oligonucleotide. This transfection mixture was incubated at room temperature for approximately 0.5 hours. For cells grown in 96-well plates, wells were washed once with 100 micro.L OPTI-MEM-1.sup.TM and then treated with 130 .micro.L of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture was replaced with fresh culture medium. Cells were harvested 16-24 hours after oligonucleotide treatment.

### Control oligonucleotides

Control oligonucleotides are used to determine the optimal oligomeric compound concentration for a particular cell line. Furthermore, when oligomeric compounds of the invention are tested in oligomeric compound screening experiments or phenotypic assays, control oligonucleotides are tested in parallel with compounds of the invention.

**Table 2 Control oligonucleotides for cell line testing, oligomeric compound screening and phenotypic assays**

| **Compou nd No.** | **Target Name** | **Species of Target** | **Sequence (5' to 3')** | **Motif** | **SEQ ID NO** |
|---|---|---|---|---|---|
| 113131 | CD86 | Human | CGTGTGTCTGTGCTAGTCCC | 5-10-5 | 19 |
| 289865 | forkhead box O1A (rhabdomyosarcoma ) | Human | GGCAACGTGAACAGGTCCAA | 5-10-5 | 20 |
| 25237 | integrin beta 3 | Human | GCCCATTGCTGGACATGC | 4-10-4 | 21 |
| 196103 | integrin beta 3 | Human | AGCCCATTGCTGGACATGCA | 5-10-5 | 22 |
| 148715 | Jagged 2 | Human; Mouse; Rat | TTGTCCCAGTCCCAGGCCTC | 5-10-5 | 23 |
| 18076 | Jun N-Terminal Kinase - 1 | Human | CTTTC^{U}CGTTGGA^{U}C^{U}CCCTGGG | 5-9-6 | 24 |
| 18078 | Jun N-Terminal Kinase - 2 | Human | GTGCG^{u}CG^{u}CGAG^{u}C^{u}CGAAATC | 5-9-6 | 25 |
| 183881 | kinesin-like 1 | Human | ATCCAAGTGCTACTGTAGTA | 5-10-5 | 26 |
| 29848 | none | none | NNNNNNNNNNNNNNNNNNNN | 5-10-5 | 27 |
| 226844 | Notch (Drosophil a) homolog 1 | Human; Mouse | GCCCTCCATGCTGGCACAGG | 5-10-5 | 28 |
| 105990 | Peroxisome proliferat or-activated receptor gamma | Human | AGCAAAAGATCAATCCGTTA | 5-10-5 | 29 |
| 336806 | Raf kinase C | Human | TACAGAAGGCTGGGCCTTGA | 5-10-5 | 30 |
| 15770 | Raf kinase C | Mouse; Murine sarcoma virus; Rat | ATGCATT^{u}CTG^{u}C^{u}C^{u}C^{u}C^{u}CAAGGA | 5-10-5 | 31 |

The concentration of oligonucleotide used varies from cell line to cell line. To determine the optimal oligonucleotide concentration for a particular cell line, the cells are treated with a positive control oligonucleotide at a range of concentrations. Positive controls are shown in Table 2. For human and non-human primate cells, the positive control oligonucleotide is selected from Compound No. 13650, Compound No. 336806, or Compound No. 18078. For mouse or rat cells the positive control oligonucleotide is Compound No. 15770 or Compound No. 15346. The concentration of positive control oligonucleotide that results in 80% inhibition of the target mRNA, for example, human Raf kinase C for Compound No. 13650, is then utilized as the screening concentration for new oligonucleotides in subsequent experiments for that cell line. If 80% inhibition is not achieved, the lowest concentration of positive control oligonucleotide that results in 60% inhibition of the target mRNA is then utilized as the oligonucleotide screening concentration in subsequent experiments for that cell line. If 60% inhibition is not achieved, that particular cell line is deemed as unsuitable for oligonucleotide transfection experiments. The concentrations of antisense oligonucleotides used herein are from 50 nM to 300 nM when the antisense oligonucleotide is transfected using a liposome reagent and 1 .micro.M to 40 .micro.M when the antisense oligonucleotide is transfected by electroporation.

### Example 2: Real-time Quantitative PCR Analysis of LMW-PTPase RNA Levels

Quantitation of LMW-PTPase mRNA levels was accomplished by real-time quantitative PCR using the ABI PRISM™ 7600, 7700, or 7900 Sequence Detection System (PE-Applied Biosystems, Foster City, CA) according to manufacturer's instructions.

Prior to quantitative PCR analysis, primer-probe sets specific to the LMW-PTPase being measured were evaluated for their ability to be "multiplexed" with a GAPDH amplification reaction. After isolation the RNA is subjected to sequential reverse transcriptase (RT) reaction and real-time PCR, both of which are performed in the same well. RT and PCR reagents were obtained from Invitrogen Life Technologies (Carlsbad, CA). RT, real-time PCR was carried out in the same by adding 20 .micro.L PCR cocktail (2.5x PCR buffer minus MgCl.sub.2, 6.6 mM MgCl.sub.2, 375 .micro.M each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 .micro.L total RNA solution (20-200 ng). The RT reaction was carried out by incubation for 30 minutes at 48.deg.C. Following a 10 minute incubation at 95.deg.C to activate the PLATINUM® Taq, 40 cycles of a two-step PCR protocol were carried out: 95.deg.C for 15 seconds (denaturation) followed by 60.deg.C for 1.5 minutes (annealing/extension).

Gene target quantities obtained by RT, real-time PCR were normalized using either the expression level of GAPDH, a gene whose expression is constant, or by quantifying total RNA using RiboGreen^{™} (Molecular Probes, Inc. Eugene, OR). GAPDH expression was quantified by RT, real-time PCR, by being run simultaneously with the target, multiplexing, or separately. Total RNA was quantified using RiboGreen^{™} RNA quantification reagent (Molecular Probes, Inc. Eugene, OR).

170 .micro.L of RiboGreen^{™} working reagent (RiboGreen^{™} reagent diluted 1:350 in 10mM Tris-HCl, 1 mM EDTA, pH 7.5) was pipetted into a 96-well plate containing 30 .micro.L purified cellular RNA. The plate was read in a CytoFluor 4000 (PE Applied Biosystems) with excitation at 485nm and emission at 530nm.

The GAPDH PCR probes have JOE covalently linked to the 5' end and TAMRA or MGB covalently linked to the 3' end, where JOE is the fluorescent reporter dye and TAMRA or MGB is the quencher dye. In some cell types, primers and probe designed to a GAPDH sequence from a different species are used to measure GAPDH expression. For example, a human GAPDH primer and probe set is used to measure GAPDH expression in monkey-derived cells and cell lines.

Probes and primers for use in real-time PCR were designed to hybridize to target-specific sequences. The primers and probes and the target nucleic acid sequences to which they hybridize are presented in Table 3. The target-specific PCR probes have FAM covalently linked to the 5' end and TAMRA or MGB covalently linked to the 3' end, where FAM is the fluorescent dye and TAMRA or MGB is the quencher dye.

**Table 3 LMW-PTPase-specific primers and probes for use in real-time PCR**

| **Target Name** | **Species** | **Sequence Description** | **Sequence (5' to 3')** | **SEQ ID NO** |
|---|---|---|---|---|
| GAPDH | Human | Forward Primer | CAACGGATTTGGTCGTATTGG | 32 |
| GAPDH | Human | Reverse Primer | GGCAACAATATCCACTTTACCAGAGT | 33 |
| GAPDH | Human | Probe | CGCCTGGTCACCAGGGCTGCT | 34 |
| GAPDH | Human | Forward Primer | GAAGGTGAAGGTCGGAGTC | 35 |
| GAPDH | Human | Reverse Primer | GAAGATGGTGATGGGATTTC | 36 |
| GAPDH | Human | Probe | CAAGCTTCCCGTTCTCAGCC | 37 |
| GAPDH | Human | Forward Primer | GAAGGTGAAGGTCGGAGTC | 35 |
| GAPDH | Human | Reverse Primer | GAAGATGGTGATGGGATTTC | 36 |
| GAPDH | Human | Probe | TGGAATCATATTGGAACATG | 38 |
| GAPDH | Mouse | Forward Primer | GGCAAATTCAACGGCACAGT | 39 |
| GAPDH | Mouse | Reverse Primer | GGGTCTCGCTCCTGGAAGAT | 40 |
| GAPDH | Mouse | Probe | AAGGCCGAGAATGGGAAGCTTGTCATC | 41 |
| GAPDH | Rat | Forward Primer | TGTTCTAGAGACAGCCGCATCTT | 42 |
| GAPDH | Rat | Reverse Primer | CACCGACCTTCACCATCTTGT | 43 |
| GAPDH | Rat | Probe | TTGTGCAGTGCCAGCCTCGTCTCA | 44 |

### Example 3: Antisense inhibition of human LMW-PTPase expression by oligomeric compounds

A series of antisense compounds was designed to target different regions of human LMW-PTPase RNA, using published sequences or portions of published sequences as cited in Table 1. The designed antisense compounds are complementary to one or more of the target nucleic acids in Table 1. The start and stop sites on the target nucleic acids for each antisense compound are presented in Tables 4a, b, c and d.

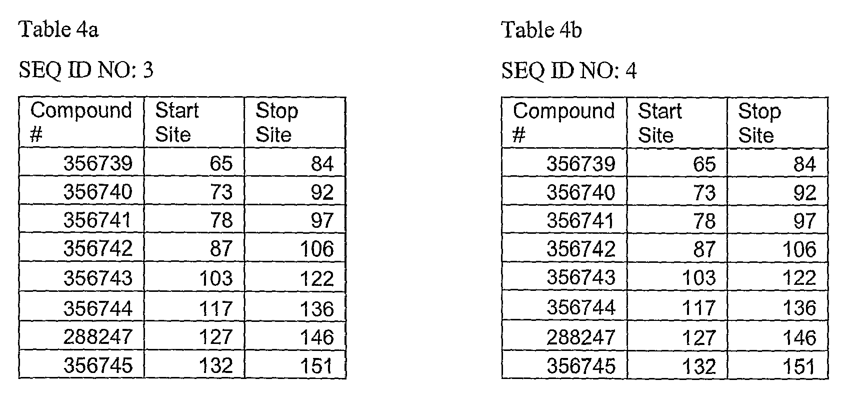

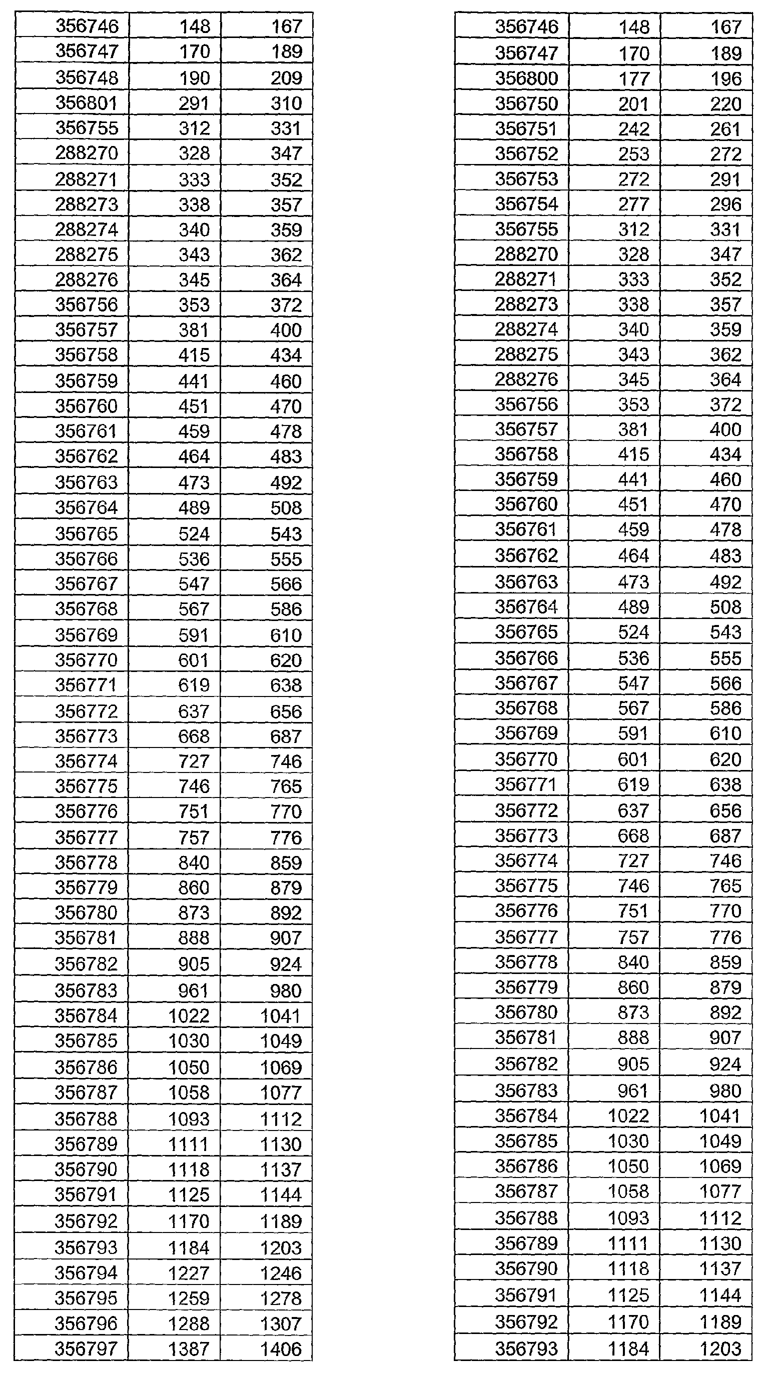

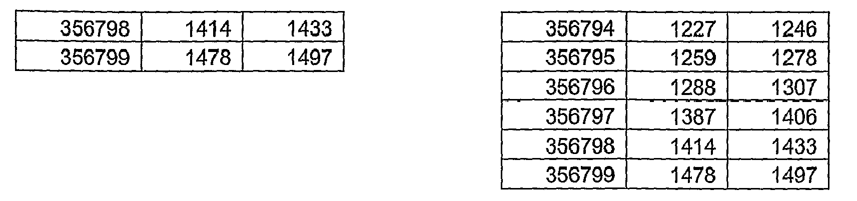

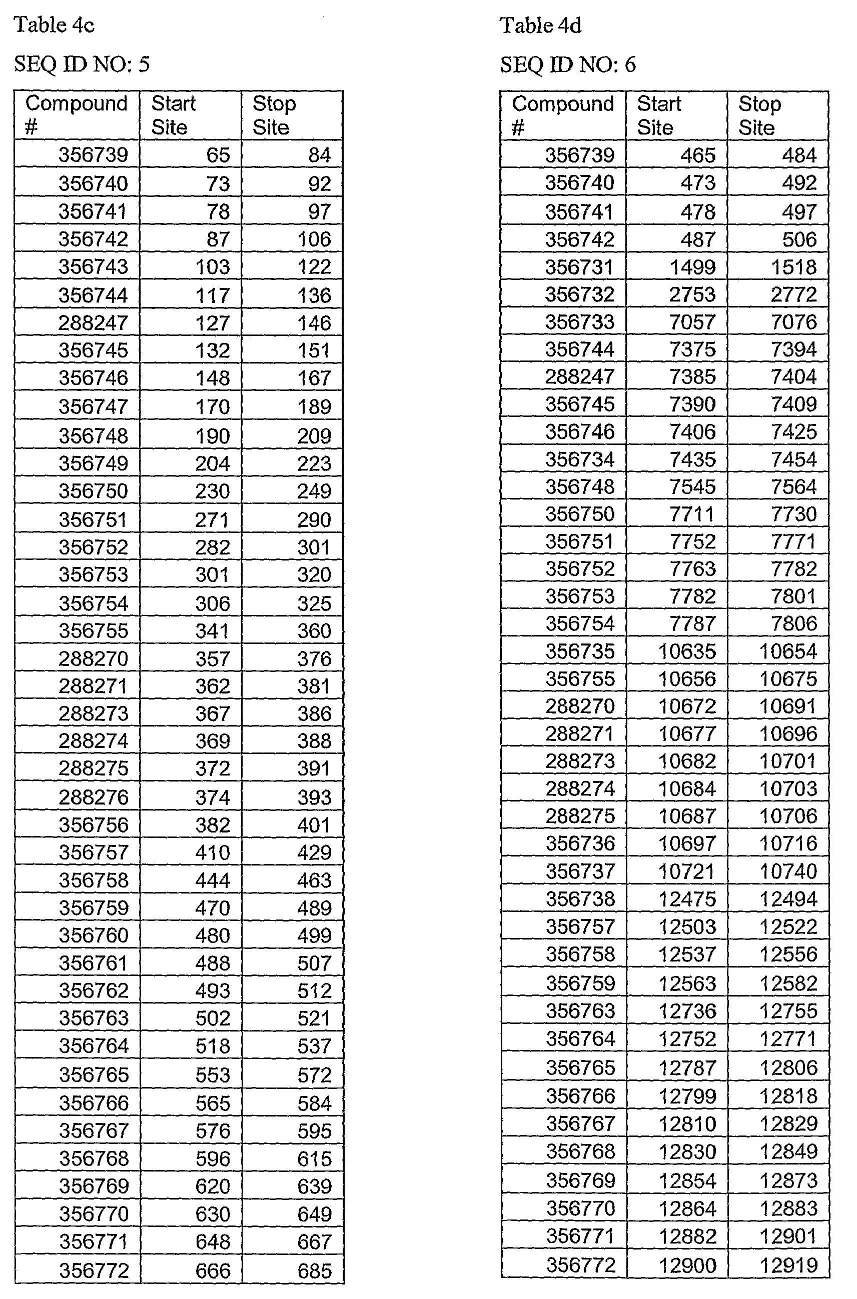

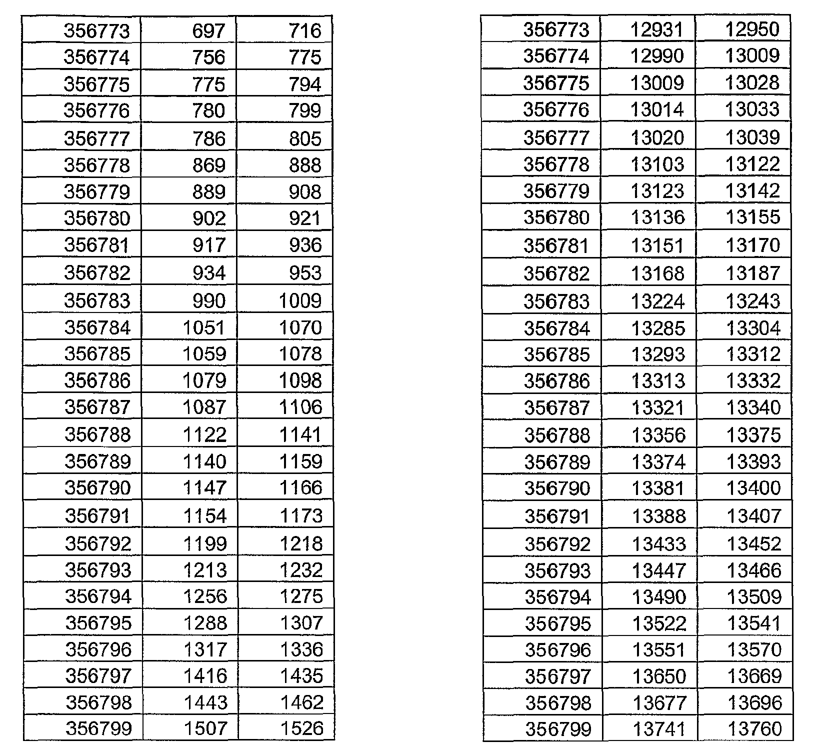

As stated above, antisense oligonucleotides directed to a target or more preferably to an active target segment can be from about 13 to about 80 linked nucleobases. The following Table 4e provides a non-limiting example of such antisense oligonucleotides targeting SEQ ID NO 1.

**Table 4e Antisense Oligonucleotides from about 13 to about 35 Nucleobases**

| Sequence | | | | Length |
|---|---|---|---|---|
| | | CCATGATTTCTTAGGCAGCT | | 20 nucleobases (SEQ ID NO: 76) |
| | AATGCCATGATTTCT | | | 15 nucleobases (SEQ ID NO: 356) |
| | | GCCATGATTTCTTAG | | 15 nucleobases (SEQ ID NO: 357) |
| | ATGCCATGATTTC | | | 13 nucleobases (SEQ ID NO: 358) |
| | AATGCCATGATTTCTTAGGCAGCTC | | | 24 nucleobases (SEQ ID NO: 359) |
| | | | TTTCTTAGGCAGCT | 14 nucleobases (SEQ ID NO: 360) |
| TGTGAATGCCATGATTTCTTAGGCAGCTCACAGCT | | | | 35 nucleobases (SEQ ID NO: 361) |
| | | CCATGATTTCTTAGGCAGCTCAGAGCT | | 27 nucleobases (SEQ ID NO: 362) |
| | | | GATTTCTTAGGCAGCTCACAGCT | 22 nucleobases (SEQ ID NO: 363) |

Antisense oligonucleotides directed to a target or more preferably to an active target segment can also contain mismatched nucleobases when compared to the target sequence. The following Table 4f provides a non-limiting example of such antisense oligonucleotides targeting nucleobases 282 *to* 301 of SEQ ID NO 5. Mismatched nucleobases are underlined. One ordinarily skilled in the art understands that antisense compounds can tolerate mismatches yet still retain their ability to hybridize with a target site and modulate the target nucleic acid through antisense mechanisms.

**Table 4f Antisense Oligonucleotides from about 1-3 Nucleobases Mismatched to the Target Sequence**

| Sequence | Number of mismatches to SEQ ID NO: 5 |
|---|---|
| CCATGATTTCTTAGGCAGCT (SEQ ID NO: 76) | None |
| CCATGATTTCTTAGGCATCT (SEQ ID NO: 364) | One mismatch |
| CCATGATCTCTTAGGCAGCT (SEQ ID NO: 365) | One mismatch |
| CCATGATTTCTTAGGCACAT (SEQ ID NO: 366) | Two mismatches |
| GCATGATTTCTTAGGCCGCT (SEQ ID NO: 367) | Two mismatches |
| CCTTGATACCTTAGGCAGCT (SEQ ID NO: 368) | Three mismatches |

Antisense compounds were designed against one or more of the human LMW-PTPase target nucleic acids sequences published in table 1 and were screened *in vitro* to determine the compound's ability to modulate expression of a target nucleic acid that encodes LMW-PTPase. The compounds shown in Table 5 are all chimeric oligonucleotides ("gapmers") 20 nucleotides in length, composed of a central "gap" region consisting of 10 2'-deoxynucleotides, which is flanked on both sides (5' and 3') by five-nucleotide "wings". The wings are composed of 2'-O-(2-methoxyethyl) nucleotides, also known as 2'-MOE nucleotides. The internucleoside (backbone) linkages are phosphorothioate throughout the oligonucleotide. All cytidine residues are 5-methylcytidines. The compounds were analyzed for their effect on gene target mRNA levels by quantitative real-time PCR as described in other examples herein, using the primer-probe set designed to hybridize to human LMW-PTPase (Table 2). Data are averages from two experiments in which A549 cells were treated with 65 nM of the disclosed oligomeric compounds using LIPOFECTIN™. A reduction in expression is expressed as percent inhibition in Table 5. If present, "N.D." indicates "not determined". The control oligomeric compound used was SEQ ID NO: 25.

**Table 5 Inhibition of human LMW-PTPase mRNA levels by chimeric oligonucleotides having 2'-MOE wings and deoxy gap**

| **Compound No.** | **Target SEQ ID NO** | **Target Site** | **Sequence (5' to 3')** | **% Inhib** | **SEQ ID NO** |
|---|---|---|---|---|---|
| 356801 | 3 | 291 | CTTTGGTAATCTGCCGGGCA | 36 | 54 |
| 288247 | 4 | 127 | ACTGCTTCTGCAATGGGTGA | 67 | 55 |
| 356800 | 4 | 177 | CAATGACCCAATTCTCTGAG | 16 | 56 |
| 288270 | 4 | 328 | TCCATACATAGTATATAATC | 39 | 57 |
| 288271 | 4 | 333 | TTTCATCCATACATAGTATA | 29 | 58 |
| 288273 | 4 | 338 | ATTGCTTTCATCCATACATA | 54 | 59 |
| 288274 | 4 | 340 | AGATTGCTTTCATCCATACA | 51 | 60 |
| 288275 | 4 | 343 | CTCAGATTGCTTTCATCCAT | 63 | 61 |
| 288276 | 4 | 345 | CTCTCAGATTGCTTTCATCC | 56 | 62 |
| 356739 | 5 | 65 | AGCCTGTTCCGCCATCTTCC | 74 | 63 |
| 356740 | 5 | 73 | GACTTGGTAGCCTGTTCCGC | 67 | 64 |
| 356741 | 5 | 78 | GCACGGACTTGGTAGCCTGT | 68 | 65 |
| 356742 | 5 | 87 | ACACAAACAGCACGGACTTG | 35 | 66 |
| 356743 | 5 | 103 | CAAATGTTACCCAGACACAC | 33 | 67 |
| 356744 | 5 | 117 | CAATGGGTGATCGACAAATG | 55 | 68 |
| 356745 | 5 | 132 | TGAAAACTGCTTCTGCAATG | 54 | 69 |
| 356746 | 5 | 148 | TCGGTTACAAGTTTCCTGAA | 64 | 70 |
| 356747 | 5 | 170 | CCAATTCTCTGAGATGTTTT | 68 | 71 |
| 356748 | 5 | 190 | GTTGCCGCGCTGTCTACCCT | 70 | 72 |
| 356749 | 5 | 204 | ATGACCCACCGGAAGTTGCC | 22 | 73 |
| 356750 | 5 | 230 | TCCAGTCAGAAACAGCACCG | 50 | 74 |
| 356751 | 5 | 271 | TAGGCAGCTCACAGCTCTTG | 59 | 75 |
| 356752 | 5 | 282 | CCATGATTTCTTAGGCAGCT | 76 | 76 |
| 356753 | 5 | 301 | TTTATGGGCTGTGTGAATGC | 56 | 77 |
| 356754 | 5 | 306 | CTTGCTTTATGGGCTGTGTG | 70 | 78 |
| 356755 | 5 | 341 | AATCAAATGTGGGAAAATCT | 51 | 79 |
| 356756 | 5 | 382 | ATTCAAATCTCTCAGATTGC | 52 | 80 |
| 356757 | 5 | 410 | TGCAGGTTTTAACTTGATTA | 57 | 81 |
| 356758 | 5 | 444 | GGATCATAGCTCCCAAGTAG | 57 | 82 |
| 356759 | 5 | 470 | GATCTTCAATAATAAGTTGT | 55 | 83 |
| 356760 | 5 | 480 | CCATAATAGGGATCTTCAAT | 26 | 84 |
| 356761 | 5 | 488 | AGTCATTCCCATAATAGGGA | 52 | 85 |
| 356762 | 5 | 493 | GTCAGAGTCATTCCCATAAT | 60 | 86 |
| 356763 | 5 | 502 | CGTCTCAAAGTCAGAGTCAT | 62 | 87 |
| 356764 | 5 | 518 | CACACTGCTGGTACACCGTC | 74 | 88 |
| 356765 | 5 | 553 | GTGGGCCTTCTCCAAGAACG | 43 | 89 |
| 356766 | 5 | 565 | GAACCTGCCTCAGTGGGCCT | 74 | 90 |
| 356767 | 5 | 576 | CAGCAGGGCACGAACCTGCC | 38 | 91 |
| 356768 | 5 | 596 | GGGTCTAGTCAGGCTGGCCG | 67 | 92 |
| 356769 | 5 | 620 | TGAGAAATGCAGGACCTCAG | 80 | 93 |
| 356770 | 5 | 630 | ACACACCGACTGAGAAATGC | 64 | 94 |
| 356771 | 5 | 648 | GGGCCCTGGAACGTGATTAC | 55 | 95 |
| 356772 | 5 | 666 | AACAAAGAGCTGGGCTTTGG | 71 | 96 |
| 356773 | 5 | 697 | CTTTTTAAGGTAAGAAACAG | 25 | 97 |
| 356774 | 5 | 756 | TGAATCAAAGATTTTTATTG | 15 | 98 |
| 356775 | 5 | 775 | AAATACCCCATAAGCTGTCT | 45 | 99 |
| 356776 | 5 | 780 | GCTTAAAATACCCCATAAGC | 61 | 100 |
| 356777 | 5 | 786 | AAGAATGCTTAAAATACCCC | 27 | 101 |
| 356778 | 5 | 869 | CAAGTGAGGTTTTCCTTCAT | 67 | 102 |
| 356779 | 5 | 889 | TAGATGTTGACCTGGGCCTT | 61 | 103 |
| 356780 | 5 | 902 | GTCTCAACAGGCTTAGATGT | 53 | 104 |
| 356781 | 5 | 917 | GACTCGATTATCTAAGTCTC | 57 | 105 |
| 356782 | 5 | 934 | AACCTACTGAAGAGGTAGAC | 76 | 106 |
| 356783 | 5 | 990 | AAGAGAGAGGTAGCACTGGG | 45 | 107 |
| 356784 | 5 | 1051 | CTAATCTAGACTGTGAGCTC | 79 | 108 |
| 356785 | 5 | 1059 | AAACACTTCTAATCTAGACT | 21 | 109 |
| 356786 | 5 | 1079 | CTATGGGTGTGTAGAAATTA | 67 | 110 |
| 356787 | 5 | 1087 | AGTGTGCACTATGGGTGTGT | 51 | 111 |
| 356788 | 5 | 1122 | AAATGTTTCTCTCTTCCCTA | 31 | 112 |
| 356789 | 5 | 1140 | GCCAACGACTGATTCCATAA | 87 | 113 |
| 356790 | 5 | 1147 | TGAAGGTGCCAACGACTGAT | 79 | 114 |
| 356791 | 5 | 1154 | GAAGTATTGAAGGTGCCAAC | 80 | 115 |
| 356792 | 5 | 1199 | GCCAATGGGCTGACCTCCTC | 77 | 116 |
| 356793 | 5 | 1213 | TGGTTCAGATGGGAGCCAAT | 66 | 117 |
| 356794 | 5 | 1256 | AAGTGTCCTTCTTTCTGGAT | 67 | 118 |
| 356795 | 5 | 1288 | CATATTCCTCAACTGACCAT | 31 | 119 |
| 356796 | 5 | 1317 | TTTGGGTTACATGTGCATAT | 73 | 120 |
| 356797 | 5 | 1416 | TGATGAAGAATACTTATTCA | 49 | 121 |
| 356798 | 5 | 1443 | ACATCTGCCTATACATTTAT | 24 | 122 |
| 356799 | 5 | 1507 | TCCCCAGTTTATTTTGAAAT | 37 | 123 |
| 356731 | 6 | 1499 | GGAAGCAACTCATGATCTGG | 63 | 124 |
| 356732 | 6 | 2753 | AATGCATGCCATATAGTAGA | 43 | 125 |
| 356733 | 6 | 7057 | CTAATGATCCAGGAGTGAAT | 39 | 126 |
| 356734 | 6 | 7435 | TGGTACTTACATTCTCTGAG | 23 | 127 |
| 356735 | 6 | 10635 | CTTTGGTAATCTAAAATTGA | 15 | 128 |
| 356736 | 6 | 10697 | ACAGGATTACCTCAGATTGC | 53 | 129 |
| 356737 | 6 | 10721 | GTTGAACAGAAATATTCTTC | 13 | 130 |
| 356738 | 6 | 12475 | ATTCAAATCTCTGTAAAATT | 14 | 131 |

The screen identified active target segments within the human LMW-PTPase mRNA sequence, specifically SEQ ID NOS: 3, 4 and 5. Each active target segment was targeted by at least one active antisense oligonucleotide. These active target regions identified for SEQ ID NO: 3 include nucleotides 1111 to 1189 (Region A) with an average inhibition of 80.5%, nucleotides 489 to 656 (Region B) with an average inhibition of 62.8%, nucleotides 536 to 656 (Region C) with an average inhibition of 64.1%, nucleotides 489 to 610 (Region D) with an average inhibition of 62.6%, nucleotides 1111 to 1203 (Region E) with an average inhibition of 77.6%, nucleotides 840 to 924 (Region F) with an average inhibition of 62.8%, nucleotides 1022 to 1069 (Region G) with an average inhibition of 55.6%, nucleotides 65 to 209 (Region H) with an average inhibition of 59.5%, nucleotides 65 to 136 (Region I) with an average inhibition of 55.2%, nucleotides 117 to 209 (Region J) with an average inhibition of 63.0% and nucleotides 338 to 460 (Region K) with an average inhibition of 55.6%. Over half of the oligonucleotides tested in this region inhibited expression by greater than 63%. Identification of these regions allows for the design of antisense oligonucleotides that modulate the expression of LMW-PTPase.

The active target regions identified for SEQ ID NO: 4 include nucleotides nucleotides 65 to 189 (Region AA) with an average inhibition of 58.4%, nucleotides 65 to 146 (Region AB) with an average inhibition of 56.8%, nucleotides 127to 189 (Region AC) with an average inhibition of 63.2%, nucleotides 338 to 460 (Region AD) with an average inhibition of 55.6%, nucleotides 489 to 610 (Region AE) with an average inhibition of 62.6%, nucleotides 536 to 656 (Region AF) with an average inhibition of 64.1%, nucleotides 489 to 656 (Region AG) with an average inhibition of 62.8%, nucleotides 840 to 924 (Region AH) with an average inhibition of 62.8%, nucleotides 1022 to 1069 (Region AI) with an average inhibition of 55.6%, nucleotides 1111 to 1189 (Region AJ) with an average inhibition of 80.5% and nucleotides 1111 to 1203 (Region AK) with an average inhibition of 77.6%..

Active target regions have also been identified for SEQ ID NO: 5. These active target regions include nucleotides 65 to 136 (Region BA) with an average inhibition of 55.2%, nucleotides 117 to 209 (Region BB) with an average inhibition of 63.0%, nucleotides 65 to 209 (Region BC) with an average inhibition of 59.5%, nucleotides 367 to 489 (Region BD) with an average inhibition of 55.6%, nucleotides 518 to 639 (Region BE) with an average inhibition of 62.6%, nucleotides 565 to 685 (Region BF) with an average inhibition of 64.1%, nucleotides 518 to 685 (Region BG) with an average inhibition of 62.8%, nucleotides 689 to 953 (Region BH) with an average inhibition of 62.8%, nucleotides 1051 to 1098 (Region BI) with an average inhibition of 55.6%, nucleotides 1140 to 1218 (Region BJ) with an average inhibition of 80.53% and nucleotides 1140 to 1232 (Region BK) with an average inhibition of 77.6%.

### Example 4

### Antisense inhibition of mouse LMW-PTPase expression by oligomeric compounds

Antisense compounds were designed against one or more of the mouse LMW-PTPase target nucleic acid sequences cited in Table 1 and were screened *in vitro* to determine the compound's ability to modulate expression of a target nucleic acid that encodes LMW-PTPase. The compounds shown in Table 6 are all chimeric oligonucleotides ("gapmers") 20 nucleotides in length, composed of a central "gap" region consisting of 10 2'-deoxynucleotides, which is flanked on both sides (5' and 3') by five-nucleotide "wings". The wings are composed of 2'-O-(2-methoxyethyl) nucleotides, also known as 2'-MOE nucleotides. The internucleoside (backbone) linkages are phosphorothioate throughout the oligonucleotide. All cytidine residues are 5-methylcytidines. The compounds were analyzed for their effect on gene target mRNA levels by quantitative real-time PCR as described in other examples herein, using the primer-probe set designed to hybridize to mouse LMW-PTPase (Table 2). Data are averages from two experiments in which b.END cells were treated with 75 nM of the disclosed oligomeric compounds using LIPOFECTIN™. A reduction in expression is expressed as percent inhibition in Table 6. The control oligomeric compound used was SEQ ID NO: 25. If present, "N.D." indicates "not determined".

**Table 6 Inhibition of mouse LMW-PTPase mRNA levels by chimeric oligonucleotides having 2'-MOE wings and deoxy gap**

| **Compound No.** | **Target SEQ ID NO** | **Target Site** | **Sequence (5' to 3')** | **% Inhib** | **SEQ ID NO** |
|---|---|---|---|---|---|
| 288290 | 11 | 207 | CTGTCTGACTCAAATGCTTT | 69 | 132 |
| 288291 | 11 | 252 | GGTCAGAGGTTTAGTTAGTC | 80 | 133 |
| 288292 | 11 | 493 | TCCGTCTGCGGTTTTATGTA | 4 | 134 |
| 288293 | 11 | 982 | GTGGTGCTCTGTTGAGGTGT | 0 | 135 |
| 288216 | 12 | 3 | TTGCTTAGTCTATAACTGAC | 0 | 136 |
| 288217 | 12 | 13 | ATGATGGAGATTGCTTAGTC | 0 | 137 |
| 288218 | 12 | 24 | AAATATGCTAAATGATGGAG | 0 | 138 |
| 288219 | 12 | 40 | GCTTCCTGTGCACCAGAAAT | 64 | 139 |
| 288220 | 12 | 48 | CTCACGTTGCTTCCTGTGCA | 0 | 140 |
| 288221 | 12 | 79 | ACTTTGTAATGGGAGTAGAT | 3 | 141 |
| 288222 | 12 | 90 | TATAATGGTAGACTTTGTAA | 0 | 142 |
| 288223 | 12 | 114 | TAGAGAATGCAAGCATATCA | 0 | 143 |
| 288224 | 12 | 123 | TTCAATTAATAGAGAATGCA | 0 | 144 |
| 288225 | 12 | 149 | ACATATACACATGAGTTGTA | 0 | 145 |
| 288226 | 12 | 159 | CTTTGTAATGACATATACAC | 0 | 146 |
| 288227 | 12 | 164 | AAACTCTTTGTAATGACATA | 0 | 147 |
| 288228 | 12 | 179 | TGCTTCCATGAAGCAAAACT | 0 | 148 |
| 288229 | 12 | 189 | GATACTTTCATGCTTCCATG | 0 | 149 |
| 288230 | 12 | 196 | AATATGTGATACTTTCATGC | 0 | 150 |
| 288231 | 12 | 202 | GCCATAAATATGTGATACTT | 0 | 151 |
| 288232 | 12 | 232 | AGACCCTCAATTTCTCTAAT | 14 | 152 |
| 288233 | 12 | 248 | TGCCATGTTTCGGTGCAGAC | 75 | 153 |
| 288234 | 12 | 253 | ACCTCTGCCATGTTTCGGTG | 64 | 154 |
| 288235 | 12 | 266 | TGACTTGGACCCAACCTCTG | 59 | 155 |
| 288236 | 12 | 273 | ACAGCACTGACTTGGACCCA | 75 | 156 |
| 288237 | 12 | 277 | AGGAACAGCACTGACTTGGA | 61 | 157 |
| 288238 | 12 | 281 | ACACACGAACAGCACTGACT | 62 | 158 |
| 288239 | 12 | 289 | TTACCGAGACACACGAACAG | 52 | 159 |
| 288240 | 12 | 293 | AATGTTACCGAGACACACGA | 53 | 160 |
| 288241 | 12 | 296 | GCAAATGTTACCGAGACACA | 56 | 161 |
| 288242 | 12 | 304 | GGTGACCGGCAAATGTTACC | 68 | 162 |
| 288243 | 12 | 306 | TGGGTGACCGGCAAATGTTA | 61 | 163 |
| 288244 | 12 | 309 | CAATGGGTGACCGGCAAATG | 62 | 164 |
| 288245 | 12 | 310 | GCAATGGGTGACCGGCAAAT | 81 | 165 |
| 288246 | 12 | 317 | TGCTTCTGCAATGGGTGACC | 77 | 166 |
| 288247 | 12 | 319 | ACTGCTTCTGCAATGGGTGA | 83 | 55 |
| 288248 | 12 | 321 | ATACTGCTTCTGCAATGGGT | 73 | 167 |
| 288249 | 12 | 323 | GAATACTGCTTCTGCAATGG | 76 | 168 |
| 288250 | 12 | 325 | CTGAATACTGCTTCTGCAAT | 59 | 169 |
| 288251 | 12 | 328 | TTCCTGAATACTGCTTCTGC | 73 | 170 |
| 288252 | 12 | 334 | ACCAGTTTCCTGAATACTGC | 79 | 171 |
| 288253 | 12 | 338 | AGTTACCAGTTTCCTGAATA | 63 | 172 |
| 288254 | 12 | 343 | TCATCAGTTACCAGTTTCCT | 57 | 173 |
| 288255 | 12 | 347 | CTTTTCATCAGTTACCAGTT | 63 | 174 |
| 288256 | 12 | 350 | AACCTTTTCATCAGTTACCA | 67 | 175 |
| 288257 | 12 | 358 | TTATCTGAAACCTTTTCATC | 48 | 176 |
| 288258 | 12 | 360 | AATTATCTGAAACCTTTTCA | 49 | 177 |
| 288259 | 12 | 365 | GGCCCAATTATCTGAAACCT | 57 | 178 |
| 288260 | 12 | 367 | ATGGCCCAATTATCTGAAAC | 43 | 179 |
| 288261 | 12 | 375 | TGCTGTCAATGGCCCAATTA | 62 | 180 |
| 288262 | 12 | 379 | GCGCTGCTGTCAATGGCCCA | 61 | 181 |
| 288263 | 12 | 406 | GGCCGGCCCACGTTCCAGTC | 65 | 182 |
| 288264 | 12 | 493 | GCAAAGTCTTCTTTTGTAAT | 57 | 183 |
| 288265 | 12 | 499 | AATGTGGCAAAGTCTTCTTT | 54 | 184 |
| 288266 | 12 | 505 | TAATCGAATGTGGCAAAGTC | 59 | 185 |
| 288267 | 12 | 510 | GTATATAATCGAATGTGGCA | 80 | 186 |
| 288268 | 12 | 511 | AGTATATAATCGAATGTGGC | 76 | 187 |
| 288269 | 12 | 518 | CATACATAGTATATAATCGA | 52 | 188 |
| 288270 | 12 | 520 | TCCATACATAGTATATAATC | 60 | 57 |
| 288271 | 12 | 525 | TTTCATCCATACATAGTATA | 57 | 58 |
| 288272 | 12 | 526 | CTTTCATCCATACATAGTAT | 57 | 189 |
| 288273 | 12 | 530 | ATTGCTTTCATCCATACATA | 71 | 59 |
| 288274 | 12 | 532 | AGATTGCTTTCATCCATACA | 70 | 60 |
| 288275 | 12 | 535 | CTCAGATTGCTTTCATCCAT | 73 | 61 |
| 288276 | 12 | 537 | CTCTCAGATTGCTTTCATCC | 78 | 62 |
| 288277 | 12 | 538 | TCTCTCAGATTGCTTTCATC | 66 | 190 |
| 288278 | 12 | 540 | GATCTCTCAGATTGCTTTCA | 70 | 191 |
| 288279 | 12 | 545 | ATTGAGATCTCTCAGATTGC | 61 | 192 |
| 288280 | 12 | 549 | TTCTATTGAGATCTCTCAGA | 65 | 193 |
| 288281 | 12 | 572 | GCAGTTTTTAACTTGATTAC | 61 | 194 |
| 288282 | 12 | 626 | AATGATGAGCTGTTTCTGTG | 53 | 195 |
| 288283 | 12 | 636 | AGGGATCTTCAATGATGAGC | 59 | 196 |
| 288284 | 12 | 639 | AATAGGGATCTTCAATGATG | 48 | 197 |
| 288285 | 12 | 663 | CCTGGAAGTCAGAGTCATTG | 82 | 198 |
| 288286 | 12 | 668 | CACCACCTCGAAGTCAGAGT | 81 | 199 |
| 288287 | 12 | 673 | TGGTACACCACCTCGAAGTC | 74 | 200 |
| 288288 | 12 | 678 | ATTGCTGGTACACCACCTCG | 75 | 201 |

### Example 5

### Antisense inhibition of rat LMW-PTPase expression by oligomeric compounds

Antisense compounds were designed against one or more of the rat LMW-PTPase target nucleic acid sequences cited in Table 1 and were screened *in vitro* to determine the compound's ability to modulate expression of a target nucleic acid that encodes LMW-PTPase. The compounds shown in Table 7 are all chimeric oligonucleotides ("gapmers") 20 nucleotides in length, composed of a central "gap" region consisting of 10 2'-deoxynucleotides, which is flanked on both sides (5' and 3') by five-nucleotide "wings". The wings are composed of 2'-O-(2-methoxyethyl) nucleotides, also known as 2'-MOE nucleotides. The internucleoside (backbone) linkages are phosphorothioate throughout the oligonucleotide. All cytidine residues are 5-methylcytidines. The compounds were analyzed for their effect on gene target mRNA levels by quantitative real-time PCR as described in other examples herein, using the primer-probe set designed to hybridize to rat LMW-PTPase (Table 2). Data are averages from two experiments in which A10 cells were treated with 50 nM of the disclosed oligomeric compounds using LIPOFECTIN™. A reduction in expression is expressed as percent inhibition in Table 7. The control oligomeric compound used was SEQ ID NO: 25. If present, "N.D." indicates "not determined".

**Table 7 Inhibition of rat LMW-PTPase mRNA levels by chimeric oligonucleotides having 2'-MOE wings and deoxy gap**

| **Compound No.** | **Target SEQ ID NO** | **Target Site** | **Sequence (5' to 3')** | **% Inhib** | **SEQ ID NO** |
|---|---|---|---|---|---|
| 288289 | 15 | 403 | ACCTCGAAGTCAGAGTCATT | 70 | 202 |
| 288233 | 16 | 24 | TGCCATGTTTCGGTGCAGAC | 74 | 153 |
| 288234 | 16 | 29 | ACCTCTGCCATGTTTCGGTG | 83 | 154 |
| 355621 | 16 | 36 | GGACCCAACCTCTGCCATGT | 89 | 203 |
| 288235 | 16 | 42 | TGACTTGGACCCAACCTCTG | 73 | 155 |
| 288238 | 16 | 57 | ACACACGAACAGCACTGACT | 29 | 158 |
| 288239 | 16 | 65 | TTACCGAGACACACGAACAG | 34 | 159 |
| 288241 | 16 | 72 | GCAAATGTTACCGAGACACA | 50 | 161 |
| 288274 | 16 | 308 | AGATTGCTTTCATCCATACA | 54 | 60 |
| 288286 | 16 | 444 | CACCACCTCGAAGTCAGAGT | 69 | 199 |
| 288287 | 16 | 449 | TGGTACACCACCTCGAAGTC | 81 | 200 |
| 288288 | 16 | 454 | ATTGCTGGTACACCACCTCG | 69 | 201 |
| 355622 | 16 | 461 | CTAAGGCATTGCTGGTACAC | 72 | 204 |
| 355623 | 16 | 466 | AGCACCTAAGGCATTGCTGG | 74 | 205 |
| 355624 | 16 | 471 | CTTGCAGCACCTAAGGCATT | 74 | 206 |
| 355625 | 16 | 476 | AAGGCCTTGCAGCACCTAAG | 83 | 207 |
| 355626 | 16 | 481 | CCAGGAAGGCCTTGCAGCAC | 86 | 208 |
| 355627 | 16 | 486 ' | CTTCTCCAGGAAGGCCTTGC | 79 | 209 |
| 355628 | 16 | 492 | GTGAGTCTTCTCCAGGAAGG | 82 | 210 |
| 355629 | 16 | 504 | TAGGACCAGCTAGTGAGTCT | 74 | 211 |
| 355630 | 16 | 519 | CTCAGTGGTGGTGGTTAGGA | 55 | 212 |
| 355631 | 16 | 556 | GCCACCACCCTTGGGCACAG | 78 | 213 |
| 355632 | 16 | 567 | GGCTAAGGACTGCCACCACC | 78 | 214 |
| 355633 | 16 | 607 | GATATACAGTAAGTCAGCTG | 80 | 215 |
| 355634 | 16 | 625 | ACCTACAATTATTTTAAAGA | 15 | 216 |
| 355635 | 16 | 633 | TGATTTCCACCTACAATTAT | 52 | 217 |
| 355636 | 16 | 638 | ATGCCTGATTTCCACCTACA | 91 | 218 |
| 355637 | 16 | 647 | TCTGAACAAATGCCTGATTT | 82 | 219 |
| 355638 | 16 | 674 | AATGTCTGCCTCAAATGTTT | 73 | 220 |
| 355639 | 16 | 680 | ACCTCAAATGTCTGCCTCAA | 78 | 221 |
| 355640 | 16 | 687 | GAGCCACACCTCAAATGTCT | 89 | 222 |
| 355641 | 16 | 700 | GTCTAAGAATACTGAGCCAC | 87 | 223 |
| 355642 | 16 | 706 | TTGTTAGTCTAAGAATACTG | 52 | 224 |
| 355643 | 16 | 725 | TATGGCGAGGCCAGAGCTTT | 77 | 225 |
| 355644 | 16 | 735 | ATTTTGTAATTATGGCGAGG | 60 | 226 |
| 355645 | 16 | 753 | ACAGTTGCTCGTTCCACTAT | 92 | 227 |
| 355646 | 16 | 759 | TGTTCCACAGTTGCTCGTTC | 95 | 228 |
| 355647 | 16 | 795 | CCTTGTGGGTCATTCTTACT | 71 | 229 |
| 355648 | 16 | 819 | GCTGGGCTCAAAGGCTGATC | 67 | 230 |
| 355649 | 16 | 841 | TTAGACCAGACTACCCAGGC | 80 | 231 |
| 355650 | 16 | 853 | CTCACACTCCAGTTAGACCA | 63 | 232 |
| 355651 | 16 | 871 | CACTGGGTGCTGGCCATGCT | 70 | 233 |
| 355652 | 16 | 890 | GTAAGGCAAGCAAACAGCAC | 40 | 234 |
| 355653 | 16 | 924 | TTGTCACAATAAGAGACAAT | 55 | 235 |
| 355654 | 16 | 931 | GGAGATATTGTCACAATAAG | 85 | 236 |
| 355655 | 16 | 939 | CCATGGATGGAGATATTGTC | 82 | 237 |
| 355656 | 16 | 944 | GGCTGCCATGGATGGAGATA | 79 | 238 |
| 355657 | 16 | 952 | AAATGGAAGGCTGCCATGGA | 80 | 239 |
| 355658 | 16 | 958 | AGTGTTAAATGGAAGGCTGC | 59 | 240 |
| 355659 | 16 | 970 | TTAAACTCTCCCAGTGTTAA | 76 | 241 |
| 355660 | 16 | 976 | CTGGGTTTAAACTCTCCCAG | 80 | 242 |
| 355661 | 16 | 1013 | GGTTCTCCTCTCTCAAATAT | 82 | 243 |
| 355662 | 16 | 1038 | AGTTCCAGGCCCATCATCAC | 61 | 244 |
| 355663 | 16 | 1050 | ATGGCCTGCTGGAGTTCCAG | 67 | 245 |
| 355664 | 16 | 1089 | TTTTTATCTTTCAGACAGGG | 19 | 246 |
| 355665 | 16 | 1103 | GCGATCTGTTAGCATTTTTA | 73 | 247 |
| 355666 | 16 | 1111 | CTGTTGCTCCCATCTGTTAG | 80 | 248 |
| 355667 | 16 | 1125 | TTAACTTCACCAACCTGTTG | 85 | 249 |
| 355668 | 16 | 1169 | CGAAGCTCAAGAAACTACAC | 69 | 250 |
| 355669 | 16 | 1187 | AAGTGGCTCAAATAGGAACC | 31 | 251 |
| 355670 | 16 | 1206 | CTTTCTCTTTAAAGAAGCAA | 24 | 252 |
| 355671 | 16 | 1215 | GCACACTTACTTTCTCTTTA | 84 | 253 |
| 355672 | 16 | 1228 | CACCACTATTTAAGCACACT | 28 | 254 |
| 355673 | 16 | 1237 | ACAAACGCACACCACTATTT | 62 | 255 |
| 355674 | 16 | 1255 | GTTGATGAGAGAACACTTAC | 79 | 256 |
| 355675 | 16 | 1270 | GTAACTTTGTAAAATGTTGA | 46 | 257 |
| 355676 | 16 | 1286 | TTACTCATGCTTGCCTGTAA | 90 | 258 |
| 355677 | 16 | 1312 | GTCCCTTTTCTGAAAATACA | 78 | 259 |
| 355678 | 16 | 1323 | ATAAATTTGAGGTCCCTTTT | 66 | 260 |
| 355679 | 16 | 1331 | ATATCCACATAAATTTGAGG | 68 | 261 |
| 355680 | 16 | 1345 | ATCTTTTCTGACATATATCC | 64 | 262 |
| 355613 | 17 | 3444 | TCTCCAGTGGCAAAGACAAA | 30 | 263 |
| 355614 | 17 | 5834 | AAGCAAGAAACTATGCGGGA | 45 | 264 |
| 355615 | 17 | 8275 | CATACGGTACCTGCCGTGCA | 53 | 265 |
| 355616 | 17 | 8312 | CAATGGCCCACTGTAACACA | 70 | 266 |
| 355617 | 17 | 13156 | GAGTACATTTGAAGTTAAAA | 45 | 267 |
| 355618 | 17 | 13309 | CTCTTGTAATCTACAATTAA | 3 | 268 |
| 355619 | 17 | 13459 | TATACCTGAGTTCAAGGTCA | 57 | 269 |
| 355620 | 18 | 204 | CTCTTGTAATCTGTCTTGCC | 27 | 270 |

### Example 6

### Inhibition of mouse LMW-PTPase mRNA levels by chimeric phosphorothioate oligonucleotides having 2'-MOE wings and a deoxy gap: dose response studies

In a further embodiment, six oligonucleotides were selected for dose-response studies: Compound No. 288285, Compound No. 288276, Compound No. 288268, Compound No. 288286, Compound No. 288267 and Compound No. 288291. Compound No. 129689 (GAGGTCTCGACTTACCCGCT, incorporated herein as SEQ ID NO: 271) and Compound No. 129695 (TTCTACCTCGCGCGATTTAC, incorporated herein as SEQ ID NO: 272, which are not targeted to LMW-PTPase, served as negative controls. Compound No. 129689 and Compound No. 129695 are chimeric oligonucleotides ("gapmers") 20 nucleotides in length, composed of a central "gap" region consisting of ten 2'-deoxynucleotides, which is flanked on both sides (5' and 3' directions) by five-nucleotide "wings". The wings are composed of 2'-O-(2-methoxyethyl) (2'-MOE) nucleotides. The internucleoside (backbone) linkages are phosphorothioate (P=S) throughout the oligonucleotide. All cytidine residues are 5-methylcytidines.

Oligonucleotides were transfected into cells using the CYTOFECTIN™ Reagent (Gene Therapy Systems, San Diego, CA). b.END cells were treated with 12.5, 25, 50 or 100 nM of oligonucleotide. Untreated control cells served as the control to which data were normalized. Quantitative real-time PCR to measure LMW-PTPase levels was performed as described herein.

Data were averaged from 3 experiments and the results are shown in Table 8 as percent inhibition relative to untreated control. Neither control oligonucleotide (Compound No. 129689 or Compound No. 129695) inhibited LMW-PTPase mRNA expression in this experiment.

**Table 8 Inhibition of mouse LMW-PTPase mRNA expression in mouse b.END cells: dose response**

| **Compound No.** | **SEQ ID NO** | **% Inhibition** | | | |
|---|---|---|---|---|---|
| | | **Dose of oligonuleotide (nM)** | | | |
| | | **12.5** | **25** | **50** | **100** |
| 288267 | 186 | 60 | 74 | 88 | 93 |
| 288268 | 187 | 54 | 72 | 80 | 93 |
| 288276 | 62 | 23 | 47 | 68 | 85 |
| 288285 | 198 | 11 | 41 | 67 | 86 |
| 288286 | 199 | 50 | 69 | 86 | 96 |
| 288291 | 133 | 72 | 82 | 90 | 92 |

As demonstrated in Table 8, Compound No. 288267, Compound No. 288268, Compound No. 288276, Compound No. 288285, Compound No. 288286 and Compound No. 288291 inhibited mouse LMW-PTPase mRNA expression in a dose-dependent manner.

### Example 7

### Inhibition of rat LMW-PTPase mRNA levels by chimeric phosphorothioate oligonucleotides having 2'-MOE wings and a deoxy gap:dose response studies

In a further embodiment, seven oligonucleotides were selected for dose-response studies: Compound No. 355621, Compound No. 355636, Compound No. 355676, Compound No. 355626, Compound No. 355654, Compound No. 355640, and Compound No. 355641. Compound No. 15770, Compound No. 129690 (TTAGAATACGTCGCGTTATG, incorporated herein as SEQ ID NO: 273), and Compound No. 141923 (CCTTCCCTGAAGGTTCCTCC, incorporated herein as SEQ ID NO: 274), which are not targeted to LMW-PTPase, served as controls. Compound No. 129690 and Compound No. 141923 are chimeric oligonucleotides ("gapmers") 20 nucleotides in length, composed of a central "gap" region consisting of ten 2'-deoxynucleotides, which is flanked on both sides (5' and 3' directions) by five-nucleotide "wings". The wings are composed of 2'-O-methoxyethyl (2'-MOE) nucleotides. The internucleoside (backbone) linkages are phosphorothioate (P=S) throughout the oligonucleotide. All cytidine residues are 5-methylcytidines.

Rat primary hepatocytes were treated with 12.5, 25, 50, 100 or 200 nM of oligonucleotide, using LIPOFECTIN™ as described herein. Untreated control cells served as the control to which data were normalized. Treatment with the transfection mixture and quantitative real-time PCR to measure rat LMW-PTPase levels were both performed as described herein.

Results of these studies are shown in Table 9. Data are averaged from four experiments and are expressed as percent inhibition relative untreated control. None of the control oligonucleotides tested (Compound No. 141923, Compound No. 15770 or Compound No. 129690) resulted greater than 4% inhibition of rat LMW-PTPase; data from cells treated with Compound No. 15770 is shown in Table 8 and is representative of the control oligonucleotide treatments.

**Table 9 Inhibition of rat LMW-PTPase mRNA expression in rat primary hepatocyte cells: dose response**

| **Compound No.** | **SEQ ID NO** | **% Inhibition** | | | | |
|---|---|---|---|---|---|---|
| | | **Dose of oligonucleotide (nM)** | | | | |
| | | **12.5** | **25** | **50** | **100** | **200** |
| 355621 | 203 | 17 | 29 | 52 | 70 | 82 |
| 355626 | 208 | 6 | 17 | 33 | 48 | 70 |
| 355636 | 218 | 17 | 27 | 43 | 64 | 80 |
| 355640 | 222 | 19 | 31 | 55 | 73 | 84 |
| 355641 | 223 | 14 | 31 | 46 | 65 | 80 |
| 355654 | 236 | 18 | 25 | 42 | 66 | 81 |
| 355676 | 258 | 15 | 24 | 49 | 62 | 77 |
| 15770 | 31 | 0 | 1 | 0 | 4 | 0 |

As demonstrated in Table 9, Compound No. 355621, Compound No. 355626 Compound No. 355636, Compound No. 355640, Compound No. 355641, Compound No. 355654 and Compound No. 355676 inhibited LMW-PTPase mRNA expression in a dose-dependent manner.

### Example 8

### Antisense inhibition of LMW-PTPase expression in vivo: ob/ob mice

Leptin is a hormone produced by fat that regulates appetite. Deficiencies in this hormone in both humans and non-human animals leads to obesity. ob/ob mice have a mutation in the leptin gene which results in obesity and hyperglycemia. As such, these mice are a useful model for the investigation of obesity and diabetes and treatments designed to treat these conditions. ob/ob mice have higher circulating levels of insulin and are less hyperglycemic than db/db mice, which harbor a mutation in the leptin receptor. In accordance with the present invention, the oligomeric compounds of the invention are tested in the ob/ob model of obesity and diabetes.

C57B1/6J-Lep ob/ob mice (Jackson Laboratory, Bar Harbor, ME) are subcutaneously injected with Compound No. 288267 (SEQ ID NO: 186) at a dose of 25 mg/kg two times per week for 4 weeks (n=5). Saline-injected animals serve as controls (n=4). After the treatment period, mice are sacrificed and target levels were evaluated in liver and in fat. RNA isolation and target mRNA expression level quantitation were performed as described by other examples herein. Animals treated with Compound No. 288267 on average showed 90% reduction in liver LMW-PTPase levels as compared to saline treated control animals. LMW-PTPase mRNA levels in epididymal fat were reduced 70% on average in animals treated with Compound No. 288267.

To assess the physiological effects resulting from inhibition of target mRNA, the ob/ob mice were evaluated at the end of the treatment period (day 28) for serum triglycerides and serum glucose levels. These parameters were measured by routine clinical analyzer instruments (e.g. Olympus Clinical Analyzer, Melville, NY). At day 28, the average triglyceride levels measured for saline-treated control animals was 168 mg/dL, while the average for animals treated with Compound No. 288267 was 75 mg/dL. At day 28, glucose was 491 mg/dL for animals treated with saline alone and 258 mg/dL for animals treated with Compound No. 288267. Therefore, treatment with Compound No. 288267 caused substantial decreases in glucose and in triglyceride levels. Therefore, one embodiment of the present invention is a method of lowering glucose by administering an oligomeric compound of the invention, and another embodiment of the present invention is a method of lowering triglycerides by administering an oligomeric compound of the invention. In one embodiment, the triglycerides are blood, plasma, or serum triglycerides. Another embodiment of the current invention is a method of ameliorating or lessening the severity of a condition in an animal. In some embodiments, the condition is diabetes. In some embodiments, the diabetes is type II diabetes. In other embodiments, the condition is metabolic syndrome.

To further assess the effects of inhibition of target mRNA on glucose metabolism, fasted serum glucose was measured via routine clinical analysis. The average fasted serum glucose level for animals treated with Compound No. 288267 was 194 mg/dL, while the average fasted level measured for animals treated with saline alone was 330 mg/dL. Therefore, another embodiment of the present invention is a method of lowering serum glucose.

Insulin levels were also measured after four weeks of treatment using a commercially available kit (e.g. Alpco insulin-specific ELISA kit,Windham, NH). Treatment with Compound No. 288267 caused about a 45% reduction in circulating plasma insulin levels. Decreased insulin levels can indicate improvement in insulin sensitivity. In one embodiment, the present invention provides methods of improving insulin sensitivity. In a further embodiment, decreased insulin levels are indicative of improved insulin sensitivity.

At the end of the study, mice were sacrificed and tissues were weighed. The average liver and spleen weights were not substantially altered by treatment with Compound No. 288267 as compared to saline-treated controls. Epididymal white adipose tissue weight was reduced by about 10% in animals treated with Compound No. 288267. Therefore, another embodiment of the present invention is a method of reducing adiposity in an animal by administering an oligomeric compound of the invention.

### Example 9

### Effect of antisense inhibition of LMW-PTPase on insulin receptor phosphorylation in ob/ob mice

To assess the effects of inhibition of LMW-PTPase on receptor phosphorylation, a bolus of insulin (2U/kg) was administered about 8 to 9 minutes prior to sacrifice to a group of the ob/ob mice treated with Compound No. 288267 (SEQ ID NO: 186) or saline as described in Example 8. Liver samples were pooled and subjected to standard immunoprecipitation and Western blot procedures and analyses. Briefly, tissues were lysed in lysis buffer (150mM NaCl, 50 mM Tris, pH 7.5, 1 % Triton X-100, 0.5% NP-40, 0.25% Nadeoxycholate, 1 mM EDTA, 1 mM EGTA, 1 mM NaOV, 1 mM NaF, and protease inhibitor cocktail I (Calbiochem). The lysates were clarified by centrifugation for 15 min. at 12000 g. The clarified lysates were first incubated with protein agarose A/G beads (1:1 ratio) for 3-4 h at 4.deg.C followed by incubation with anti-phosphotyrosine antibody for another 3-4h at 4.deg.C. The immune complex was then washed with lysis buffer, boiled in Laemmli's sample buffer, and analyzed by Western blot. The membrane was blotted with a commercially available anti-insulin receptor beta (IR-.beta. subunit antibody (Santa Cruz, CA). The signal was detected by using a commercially available HRP-conjugated goat anti-rabbit IgG antibody and ECL.

Quantitation of the resulting bands showed approximately a 4 to 6-fold increase in phosphorylation of the insulin receptor upon insulin stimulation in the samples from animals treated with Compound No. 288267 as compared to that measured for saline-treated controls. These data suggest improved insulin sensitivity upon treatment with antisense oligonucleotides targeted to LMW-PTPase.

### Example 10

### Effect of antisense inhibition of LMW-PTPase on PI3-K activity in ob/ob mice

To further assess the role of LMW-PTPase in the insulin receptor signaling pathway and to assess the effects of antisense inhibition of LMW-PTPase on insulin action, clarified lysates prepared as described in Example 11 from livers or fat tissue of ob/ob mice treated as described in Examples 8 and 9, and were subjected to further analyses.

Phosphatidyl inositol 3-kinase (PI3-K) is an enzyme activated downstream of insulin-receptor stimulation. PI3-kinase activity was measured using methods known in the art (Pandey et al., Biochemistry, 1998, 37, 7006-7014). Briefly, the clarified lysates were subjected to immunoprecipitation with IRS-1/2 antibody (1 ug) for 2 h at 4.deg.C, followed by incubation with protein A/G sepharose for an additional 2h. The immune complexes were washed and subjected to in vitro PI3-Kinase assay using L-.alpha.-phosphatidylinositol (PI) as an exogenous substrate. The phosphorylated lipid was isolated and separated by thin-layer chromatography (TLC). The TLC plate was exposed to Kodak film, and the radioactive spots associated with the product of PI3-K activity (PIP2) was scratched off of the TLC plates and counted in a scintillation counter. Average results from the scintillation counts are shown in Table 10 in arbitrary units for the livers from each treatment group with or without insulin. The antibody used for the immunoprecipitation is shown in the column designated "IP" (IRS-1 or IRS-2).

**Table 10 Effects of antisense inhibition of LMW-PTPase on PI3-K activity in ob/ob mouse liver**

| **Treatment group** | **IP** | **PI3-K activity (arbitrary units)** | |
|---|---|---|---|
| | | **- Insulin** | **+ Insulin** |
| Saline | IRS-1 | 260853 | 257907 |
| Compound No. 288267 | IRS-1 | 291982 | 674202 |
| Saline | IRS-2 | 596 | 677 |
| Compound No. 288267 | IRS-2 | 679 | 1092 |

As shown in Table 10, Compound No. 288267 (SEQ ID NO: 186) caused increases in insulin-stimulated PI3-K activity above the increases observed for animals treated with saline.

Approximate results from the scintillation counts are shown in Table 11 as averages in arbitrary units for adipose tissue from each treatment group with or without insulin. The antibody used for the immunoprecipitation was IRS-1.

**Table 11 Effects of antisense inhibition of LMW-PTPase on PI3-K activity in ob/ob mouse fat tissue**

| | **PI3-K activity (arbitrary units)** | |
|---|---|---|
| **Treatment group** | **- Insulin** | **+ Insulin** |
| Saline | 319 | 365 |
| Compound No. 288267 | 438 | 725 |

As shown in Table 11, Compound No. 288267 caused increases in insulin-stimulated PI3-K activity above the increases observed for animals treated with saline. Taken together, these results demonstrate a novel role for LMW-PTPase in insulin action and show that antisense inhibition of LMW-PTPase improves insulin signaling in ob/ob mice. Therefore another embodiment of the present invention is a method of improving insulin signaling in an animal by administering an oligomeric compound of the invention.

### Example 11

### Effect of antisense inhibition of LMW-PTPase on insulin signaling: in vitro studies

In accord with the present invention, the effects of antisense oligonucleotides targeted to LMW-PTPase on insulin-signaling were investigated in primary hepatocytes cultured from ob/ob mice using methods described herein. The ob/ob primary hepatocytes were treated with Compound No. 288267 (SEQ ID NO: 186) or the control oligonucleotide Compound No. 141923 (SEQ ID NO: 274) by transfection methods described herein. Treated cells were incubated for 10 minutes in the absence or presence of 100 nM insulin. Cells treated with transfection reagent alone served as controls.

Cell lysates were prepared and subjected to immunoprecipitation with anti-phosphotyrosine antibody followed by Western blot analysis using an anti-IR-.beta. antibody (Santa Cruz, CA) via standard methods. Cells treated with Compound No. 288267 showed a larger increase in insulin-stimulated IR-.beta. phosphorylation than was observed for control cells or cells treated with Compound No. 141923.

In a similar experiment using a commercially available antibody which recognizes phosphorylated Akt (Cell Signaling, Boston, MA), cells treated with Compound No. 288267 showed a larger increase in insulin-stimulated Akt phosphorylation than was observed for control cells or cells treated with Compound No. 141923. These data demonstrate that LMW-PTPase plays a role in the insulin signaling pathway.

Antisense compound modulation of LMW-PTPase expression levels was determined using primary mouse hepatocytes by treating the cells with 100 ng of Compound No. 288267 or with vehicle as descreibed above. Following incubation, the cells were lysed in RTL buffer and total RNA was isolated using QIAGEN RNA easy kits (Qiagen, Valencia, CA). RT-PCR was performed as described above. These data showed an approximate 90% reduction in LMW-PTPase mRNA levels compared to control. A corresponding reduction in LMW-PTPase protein levels was seen by western blot analysis of the LMW-PTPase protein. Interestingly, there was no significant reduction in PTP1b levels in the presence of Compound No. 288267 compared to control cells (antibody available from Upstate Cell Signaling Solutions).

To further determine the action of LMW-PTPase antisense compounds on the insulin signaling pathway duplicate cell culture well comprising primary mouse hepatocytes were treated with 100 ng of either Compound No. 288267, Compound No. 288291, control Compound No. 141923, an antisense inhibitor of PTP1b or a combination of Control No. 288291 and the antisense inhibitor of PTP1b. A saline treated control was used as well. For each treatment group, one of the wells was incubated with 5nM of insulin for 10 minutes. Following incubation the cell were analyzed for levels of phosphorylated IR-.beta., phosphoAkt, unphosphorylated Akt, PTP1b or LMW-PTPase using western blot techniques. These data show that IR-.beta. is phosphorylated in the presence of LMW-PTPase antisense compounds, but not in the presence of PTP-1b antisense compounds either alone or combined with the LMW-PTPase antisense compound. Western blot techniques are well known to those of ordinary skill in the art and antibodies are readily available from a number of commercial vendors (e.g., Upstate Cell Signaling Solutions, Charlottesville, VA). (Harlow, E and Lane, D., Antibodies: A Laboratory Manual, (1988) Cold Spring Harbor Press).

### Example 12

### Design of oligomeric compounds targeting human LMW-PTPase

A series of oligomeric compounds was designed to target different regions of human LMW-PTPase, using published sequences cited in Table 1. The compounds are shown in Table 12. All compounds in Table 12 are chimeric oligonucleotides ("gapmers") 20 nucleotides in length, composed of a central "gap" region consisting of 10 2'-deoxynucleotides, which is flanked on both sides (5' and 3') by five-nucleotide "wings". The wings are composed of 2'-O-(2-methoxyethyl) nucleotides, also known as 2'-MOE nucleotides. The internucleoside (backbone) linkages are phosphorothioate throughout the oligonucleotide. All cytidine residues are 5-methylcytidines.

**Table 12 Chimeric oligonucleotides having 2'-MOE wings and deoxy gap targeting human LMW-PTPase**

| **Compound No.** | **Target SEQ ID NO** | **Target Site** | **Sequence (5' to 3')** | **SEQ ID NO** |
|---|---|---|---|---|
| 105809 | 1 | 1 | ACCCCGTTCCGCACGCCCCC | 279 |
| 105811 | 1 | 41 | TAGCCTGTTCCGCCATCTTC | 280 |
| 105812 | 1 | 241 | GCTCATGGGAATGCCGTGCC | 281 |
| 105813 | 1 | 271 | ATCTTCTTTGGTAATCTGCC | 282 |
| 105814 | 1 | 421 | ATAGGGATCTTCAATAATAA | 283 |
| 105815 | 1 | 451 | CACCGTCTCAAAGTCAGAGT | 284 |
| 105816 | 1 | 571 | CCGACTGAGAAATGCAGGAC | 285 |
| 105817 | 1 | 611 | AAGAAAGAGCTGGCTTTGGG | 286 |
| 105818 | 1 | 671 | CAAACACAACTGATTTCCAT | 287 |
| 105819 | 1 | 701 | TGAATCAAACATTTTTATTG | 288 |
| 105820 | 1 | 781 | TTGTTCTACTATTTTTGTAA | 289 |
| 105821 | 1 | 811 | GTGAGGTTTTCCTTCATTGT | 290 |
| 105822 | 1 | 961 | ACTACTGTCAATCCACAAAA | 291 |
| 105823 | 1 | 1051 | TCTTCCCTATCTTTTCAATA | 292 |
| 105824 | 1 | 1091 | GTATTGAAGGTGCCAACGAC | 293 |
| 105825 | 1 | 1171 | TAAGTTTCAGAGGCAAAGTG | 294 |
| 105826 | 1 | 1201 | CATACAAGTGTCCTTCTTTC | 295 |
| 105827 | 1 | 1291 | TTATTTTAAAAAATAAGCCA | 296 |
| 105828 | 1 | 1321 | AAATAATAACAGTTTTGCCA | 297 |

### Example 13

### Design of oligomeric compounds targeting mouse LMW-PTPase

A series of oligomeric compounds was designed to target different regions of mouse LMW-PTPase, using published sequences cited in Table 1. The compounds are shown in Table 13. All compounds in Table 13 are chimeric oligonucleotides ("gapmers") 20 nucleotides in length, composed of a central "gap" region consisting of 10 2'-deoxynucleotides, which is flanked on both sides (5' and 3') by five-nucleotide "wings". The wings are composed of 2'-O-(2-methoxyethyl) nucleotides, also known as 2'-MOE nucleotides. The internucleoside (backbone) linkages are phosphorothioate throughout the oligonucleotide. All cytidine residues are 5-methylcytidines.

**Table 13 Chimeric oligonucleotides having 2'-MOE wings and deoxy gap targeting mouse LMW-PTPase**

| **Compound No.** | **Target SEQ ID NO** | **Target Site** | **Sequence (5' to 3')** | **SEQ ID NO** |
|---|---|---|---|---|
| 349037 | 11 | 246 | AGGTTTAGTTAGTCTAAGAA | 298 |
| 349038 | 11 | 248 | AGAGGTTTAGTTAGTCTAAG | 299 |
| 349039 | 11 | 250 | TCAGAGGTTTAGTTAGTCTA | 300 |
| 349040 | 11 | 254 | AAGGTGAGAGGTTTAGTTAG | 301 |
| 349041 | 11 | 256 | GCAAGGTCAGAGGTTTAGTT | 302 |
| 349042 | 11 | 258 | CCGCAAGGTCAGAGGTTTAG | 303 |
| 349043 | 11 | 296 | TTTGTTCCATATTTGCTTGT | 304 |
| 349044 | 12 | 307 | ATGGGTGACCGGCAAATGTT | 305 |
| 349045 | 12 | 308 | AATGGGTGACCGGCAAATGT | 306 |
| 349046 | 12 | 311 | TGCAATGGGTGACCGGCAAA | 307 |
| 349047 | 12 | 312 | CTGCAATGGGTGACCGGCAA | 308 |
| 349048 | 12 | 313 | TCTGCAATGGGTGACCGGCA | 309 |
| 349049 | 12 | 314 | TTCTGCAATGGGTGACCGGC | 310 |
| 349050 | 12 | 315 | CTTCTGCAATGGGTGACCGG | 311 |
| 349051 | 12 | 316 | GCTTCTGCAATGGGTGACCG | 312 |
| 349052 | 12 | 318 | CTGCTTCTGCAATGGGTGAC | 313 |
| 349053 | 12 | 320 | TACTGCTTCTGCAATGGGTG | 314 |
| 349054 | 12 | 322 | AATACTGCTTCTGCAATGGG | 315 |
| 349055 | 12 | 327 | TCCTGAATACTGCTTCTGCA | 316 |
| 349056 | 12 | 330 | GTTTCCTGAATACTGCTTCT | 317 |
| 349057 | 12 | 332 | CAGTTTCCTGAATACTGCTT | 318 |
| 349058 | 12 | 335 | TACCAGTTTCCTGAATACTG | 319 |
| 349059 | 12 | 337 | GTTACCAGTTTCCTGAATAC | 320 |
| 349060 | 12 | 339 | CAGTTACCAGTTTCCTGAAT | 321 |
| 349061 | 11 | 477 | TGTATGCTCGCTCCTCCTCT | 322 |
| 349062 | 12 | 503 | ATCGAATGTGGCAAAGTCTT | 323 |
| 349063 | 11 | 506 | CCGGCGCGCTCGCTCCGTCT | 324 |
| 349064 | 12 | 507 | TATAATCGAATGTGGCAAAG | 325 |
| 349065 | 12 | 509 | TATATAATCGAATGTGGCAA | 326 |
| 349066 | 12 | 512 | TAGTATATAATCGAATGTGG | 327 |
| 349067 | 12 | 513 | ATAGTATATAATCGAATGTG | 328 |
| 349068 | 12 | 515 | ACATAGTATATAATCGAATG | 329 |
| 349069 | 12 | 517 | ATACATAGTATATAATCGAA | 330 |
| 349070 | 12 | 531 | GATTGCTTTCATCCATACAT | 331 |
| 349071 | 12 | 533 | CAGATTGCTTTCATCCATAC | 332 |
| 349072 | 12 | 536 | TCTCAGATTGCTTTCATCCA | 333 |
| 349073 | 11 | 537 | TTGTCGCCTCCCGCGTCGTG | 334 |
| 349074 | 12 | 539 | ATCTCTCAGATTGCTTTCAT | 335 |
| 349075 | 12 | 541 | AGATCTCTCAGATTGCTTTC | 336 |
| 349076 | 12 | 543 | TGAGATCTCTCAGATTGCTT | 337 |
| 349077 | 11 | 574 | CTCCTGCCACATGTAGTCCG | 338 |
| 349078 | 11 | 643 | CTGCTCGGGCCCTTATTTTC | 339 |
| 349079 | 12 | 665 | CACCTCGAAGTCAGAGTCAT | 340 |
| 349080 | 12 | 667 | ACCACCTCGAAGTCAGAGTC | 341 |
| 349081 | 12 | 669 | ACACCACCTCGAAGTCAGAG | 342 |
| 349082 | 12 | 670 | TACACCACCTCGAAGTCAGA | 343 |
| 349083 | 12 | 671 | GTACACCACCTCGAAGTGAG | 344 |
| 349084 | 12 | 672 | GGTACACCACCTCGAAGTCA | 345 |
| 349085 | 12 | 674 | CTGGTACACCACCTCGAAGT | 346 |
| 349086 | 12 | 675 | GCTGGTACACCACCTCGAAG | 347 |
| 349087 | 12 | 676 | TGCTGGTACACCACCTCGAA | 348 |
| 349088 | 11 | 676 | TACGACCGCGACGGCCGCGT | 349 |
| 349089 | 12 | 677 | TTGCTGGTAGACCAGCTCGA | 350 |
| 349090 | 11 | 745 | CGAAGGTGTTCGTGTTACTC | 351 |
| 349091 | 11 | 824 | GCGTTGGCGCGTCGTCGCTG | 352 |
| 349092 | 11 | 876 | GGGTGGTAGTGGCGGGTGGG | 353 |
| 349093 | 11 | 931 | GTGGCTGGGTGGTGTCGTGT | 354 |

### Example 14

### Antisense inhibition of LMW-PTPase expression in vivo: mouse model of diet-induced obesity

The C57BL/6 mouse strain is reported to be susceptible to hyperlipidemia-induced atherosclerotic plaque formation. When these mice are fed a high-fat diet, they develop diet-induced obesity. Accordingly these mice are a useful model for the investigation of obesity and treatments designed to treat this conditions. In a further embodiment of the present invention, the oligomeric compounds of the invention are tested in a model of diet-induced obesity.

Male C57BL/6 mice received a 60% fat diet for about 12-13 weeks, after which mice were subcutaneously injected with Compound No. 288267 (SEQ ID NO: 186), an antisense oligonucleotide targeted to LMW-PTPase, or the control compound Compound No. 141923 (SEQ ID NO: 274) at a dose of 25 mg/kg two times per week for 6 weeks. Each treatment group was comprised of about 8 to 10 animals. Saline-injected high-fat fed animals serve as a control. As an additional control, mice fed a normal chow diet were treated with saline alone.

Body weight and accumulated food intake were measured throughout the study for each treatment group. No significant alterations in accumulated food intake were observed for the animals fed a high-fat diet, regardless of the treatment. At the end of the study, body composition was assayed by MRI. Treatment with Compound No. 288267 caused about a 12% decrease in fat content of the high-fat fed mice over the treatment period. The fat content of high-fat fed animals treated with saline alone or with the control compound Compound No. 141923 did not decrease over the course of the study. Therefore, another embodiment of the present invention is a method of reducing adiposity in an animal by administering an oligomeric compound targeted to LMW-PTPase.

To assess the physiological effects resulting from inhibition of target mRNA, the diet-induced obese mice that received treatment were further evaluated at beginning of the study (week 0), during the 3rd week of treatment (week 3.5), and at the end of the treatment period (week 6) for plasma triglyceride and plasma cholesterol levels. Triglycerides and cholesterol are measured by routine clinical analyzer instruments (e.g. Olympus Clinical Analyzer, Melville, NY). Average results for each treatment group are shown in Table 14 in mg/dL.

**Table 14 Effects of antisense inhibition of LMW-PTPase on plasma lipid levels**

| **Treatment** | **Cholesterol** | | | **Triglycerides** | | |
|---|---|---|---|---|---|---|
| | **Week 0** | **Week 3.5** | **Week 6** | **Week 0** | **Week 3.5** | **Week 6** |
| Saline, high-fat fed | 176 | 175 | 184 | 83 | 65 | 76 |
| Compound No. 141923 | 182 | 174 | 181 | 78 | 70 | 59 |
| Compound No. 288267 | 176 | 157 | 136 | 79 | 60 | 56 |
| Saline, normal diet | 82 | 68 | 68 | 74 | 76 | 61 |

As shown in Table 14, Treatment with Compound No. 288267 caused a decrease in plasma cholesterol in diet-induced obese mice. Therefore embodiments of the present invention include methods of lowering cholesterol in an animal by administering an oligomeric compound of the invention.

The effects of target inhibition on glucose and insulin metabolism were also evaluated in the diet-induced obese mice treated with the oligomeric compounds of the invention. Plasma glucose was measured at beginning of the study (week 0), during the 3rd week of treatment (week 3.5), and at the end of the treatment period (week 6). Plasma insulin was similarly measured at beginning of the study (week 0), during the 3rd week of treatment (week 3.5), and at the end of the treatment period (week 6). Glucose levels were measured using standard methods (for example, with a YSI glucose analyzer, YSI Scientific, Yellow Springs, OH) and insulin levels were measured using a commercially available kit (for example, an Alpco insulin-specific ELISA kit, Windham, NH). Hypoglycemia was not observed in animals treated with Compound No. 288267. Insulin levels are shown in Table 15 as a percentage of insulin levels measured for high-fat fed animals treated with saline alone.

**Table 15 Effects of antisense inhibition of LMW-PTPase on blood glucose and insulin levels**

| **Treatment** | **Insulin** | | |
|---|---|---|---|
| | **Week 0** | **Week 3.5** | **Week 6** |
| Saline, high-fat fed | 100 | 114 | 179 |
| Compound No. 141923 | 100 | 107 | 164 |
| Compound No. 288267 | 100 | 86 | 100 |
| Saline, normal diet | 21 | 86 | 79 |

As shown in Table 15, treatment with Compound No. 288267 prevented the increase in insulin levels over the course of the study observed in high-fat fed animals treated with saline or with the control compound Compound No. 141923. Therefore, another embodiment of the present invention is a method of improving insulin sensitivity in an animal by administering an oligomeric compound targeted to LMW-PTPase. In one embodiment, improved insulin sensitivity is indicated by a reduction in circulating insulin levels.

Glucose tolerance tests were also administered in fasted mice. During the fifth week of treatment, mice were fasted overnight and then received intraperitoneal injections of glucose at a dose of 1g/kg, and the blood glucose were measured before the glucose challenge and at 30 minute intervals for up to 2 hours. Results are shown in Table 16 for each treatment group.

**Table 16 Effects of antisense inhibition of LMW-PTPase on glucose tolerance**

| **Treatment** | **Glucose (mg/dL)** | | | | |
|---|---|---|---|---|---|
| | **0 min.** | **30 min.** | **60 min.** | **90 min.** | **120 min.** |
| Saline, high-fat fed | 110 | 346 | 264 | 219 | 194 |
| Compound No. 141923 | 112 | 317 | 243 | 210 | 185 |
| Compound No. 288267 | 118 | 277 | 210 | 188 | 168 |
| Saline, normal diet | 100 | 249 | 184 | 151 | 133 |

A plot of the data in Table 16 as glucose level as a function of time allows for comparison of the area under the curve for each treatment group. These data reveal improved glucose tolerance in high-fat fed animals treated with Compound No. 288267. Therefore, another aspect of the present invention is a method of improving glucose tolerance in an animal by administering an oligomeric compound of the invention.

Insulin tolerance tests were also administered in fasted mice. During the fourth week of treatment, mice were fasted for approximately 4 to 5 hours, and then received intraperitoneal injections of insulin at a dose of 0.5U/kg. Glucose levels were measured before the insulin challenge and at 30 minute intervals for up to 2 hours. Results are shown in Table 17.

**Table 17 Effects of antisense inhibition of LMW-PTPase on insulin tolerance**

| **Treatment** | **Glucose (mg/dL)** | | | | |
|---|---|---|---|---|---|
| | **0 min.** | **30 min.** | **60 min.** | **90 min.** | **120 min.** |
| Saline, high-fat fed | 218 | 122 | 123 | 129 | 154 |
| Compound No. 141923 | 208 | 127 | 108 | 116 | 145 |
| Compound No. 288267 | 172 | 104 | 87 | 88 | 117 |

A plot of the data in Table 17 as glucose level as a function of time allows for comparison of the area under the curve for each treatment group. These data reveal improved insulin tolerance in high-fat fed animals treated with Compound No. 288267. Therefore, another aspect of the present invention is a method of improving insulin tolerance in an animal by administering an oligomeric compound of the invention.

### Example 15

### Antisense inhibition of LMW-PTPase expression in a mouse model of diet-induced obesity

Liver and fat tissues from C57BL/6 mice fed a high fat diet and treated as described in Example 14 were further evaluated at the end of the study for target reduction. Analysis of target reduction in tissues was performed as described herein. Treatment with Compound No. 288267 reduced LMW-PTPase gene expression by about 90% in liver and about 75% in fat (white adipose tissue), whereas treatment with Compound No. 141923 did not reduce LMW-PTPase expression in either tissue. Treatment with Compound No. 288267 also caused a significant reduction in hepatic glucose-6-phosphatase expression, suggesting a suppression of hepatic glucose output. Treatment with Compound No. 288267 did not have an effect on the expression of SHP2, SHPTP2 or PTEN expression. Similar results are seen with ob/ob mice. Therefore, another embodiment of the present invention is a method of reducing hepatic glucose output in an animal by administering an oligomeric compound of the invention. Another embodiment of the present invention is a method of modulating genes involved in glucose metabolism by administering an oligomeric compound of the invention. In one embodiment, the modulated gene is glucose-6-phosphatase.

Liver triglyceride levels were also assessed, using a commercially available kit (for example, using the Triglyceride GPO Assay from Roche Diagnostics, Indianapolis, IN). Liver triglyceride content was reduced with Compound No. 288267 treatment as compared to treatment with Compound No. 141923 (about 35 mg/g vs. about 56 mg/g tissue, respectively).

Hepatic steatosis may also be assessed by routine histological analysis of frozen liver tissue sections stained with oil red O stain, which is commonly used to visualize lipid deposits, and counterstained with hematoxylin and eosin, to visualize nuclei and cytoplasm, respectively.

Therefore, another embodiment of the present invention is a method of decreasing hepatic triglyceride accumulation in an animal by administering an oligomeric compound of the invention. Another embodiment of the present invention is a method of treating steatosis in an animal by administering an oligomeric compound of the invention. In one embodiment, the steatosis is steatohepatitis. In one embodiment, the steatosis is NASH.

Another embodiment of the present invention is a method of treating diabetes or metabolic syndrome comprising administering an oligomeric compound of the invention. In some embodiments, the oligomeric compound inhibits LMW-PTPase expression in the liver, in fat, or in both tissues.

The invention also provides the following numbered embodiments:
1. A chimeric antisense compound of 15 to 35 nucleobases comprising at least two chemical modifications and targeted to at least a 12 nucleobase portion of an active target segment of SEQ ID NO: 5, wherein the active target segment is selected from the group consisting of Region BA, Region BB, Region BC, Region BD, Region BE, Region BF, Region BG, Region BH, Region BI, Region BJ and Region BK.
2. The compound of embodiment 1, wherein the compound is targeted to at least a 20 nucleobase portion of an active target segment of SEQ ID NO: 5, wherein the active target segment is selected from the group consisting of Region BA, Region BB, Region BC, Region BD, Region BE, Region BF, Region BG, Region BH, Region BI, Region BJ and Region BK.
3. The compound of embodiment 1, comprising 15 to 30 nucleobases in length.
4. The compound of embodiment 1, comprising 18 to 22 nucleobases in length.
5. The compound of embodiment 1 comprising less than 4 mismatches to the target nucleic acid.
6. The compound of embodiment 1 wherein the chemical modification is selected from a group consisting or at least one modified internucleoside linkage, at least one modified nucleobase, at least one modified sugar and a combination thereof.
7. The compound of embodiment 6 wherein the modified internucleoside linkage comprises a phosphorothioate linkage.
8. The compound of embodiment 6 wherein the modified sugar moiety comprises a high affinity modification comprising a 2'-O-(2-methoxyethyl), a 2-O-methyl, an LNA, an ENA or a combination thereof.
9. The compound of embodiment 1 wherein the chimeric antisense compound comprises deoxynucleotides in a first region, at least one high affinity modified sugar in each of a second region and a third region, which flank the first region on the 5' end and the 3' end, respectively, and at least one phosphorothioate modified internucleoside linkage.
10. The compound of embodiment 9 wherein the first region is ten deoxynucleotides in length, the second and third regions are each five nucleotides in length and comprise five 2'-O-(2-methoxyethyl) nucleotides, and each internucleoside linkage in the chimeric oligonucleotide is a phosphorothioate.
11. A pharmaceutical composition comprising a compound of embodiment 1 and a pharmaceutically acceptable penetration enhancer, carrier, or diluent.
12. A method of lowering triglyceride levels in an animal comprising the step of administering an oligomeric compound of any one of embodiments 1-11.
13. The method of embodiment 12 wherein the triglyceride levels are blood, plasma, or serum triglyceride levels.
14. A method of improving insulin sensitivity in an animal comprising the step of administering an oligomeric compound of any one of embodiments 1-11.
15. A method of improving insulin sensitivity in an animal comprising the step of administering an oligomeric compound of any one of embodiments 1-11, wherein improvement of insulin sensitivity is measured as a reduction in insulin levels.
16. A method of lowering blood glucose levels in an animal comprising the step of administering an oligomeric compound of any one of embodiments 1-11.
17. A method of lowering cholesterol in an animal comprising the step of administering an oligomeric compound of any one of embodiments 1-11.
18. The method of embodiment 17 wherein cholesterol is LDL cholesterol.
19. The method of embodiment 17 wherein cholesterol is VLDL cholesterol.
20. A method of improving glucose tolerance in an animal comprising the step of administering an oligomeric compound of any one of embodiments 1-11.
21. A method of ameliorating or lessening the severity of a condition in an animal comprising contacting said animal with an effective amount of the compound of any one of embodiments 1-11 so that expression of LMW-PTPase is inhibited and measurement of one or more physical indicator of said condition indicates a lessening of the severity of said condition.
22. The method of embodiment 21 wherein the condition is diabetes.
23. The method of embodiment 22 wherein the diabetes is type II.
24. The method of embodiment 21 wherein the condition is obesity.
25. The method of embodiment 24 wherein the obesity is diet-induced.
26. The method of embodiment 21 wherein the condition is obesity and the physical indicator is reduced body fat.
27. The method of embodiment 21 wherein the condition is insulin resistance.
28. The method of embodiment 21 wherein the condition is insulin deficiency.
29. The method of embodiment 21 wherein the condition is hypercholesterolemia.
30. A method for the prevention, amelioration, or treatment of a disease or condition associated with reduced insulin sensitivity comprising administration of the compound of any of embodiments 1-11 to an individual in need of such intervention.
31. A method for designing and synthesizing an antisense compound for inhibiting a nucleic molecule encoding LMW-PTPase, comprising:
   (a) identifying a nucleotide sequence within an active target region to which an to target an antisense compound and
   (b) synthesizing an antisense compound having a complementary nucleotide sequence to the identified nucleotide sequence and with less than 4 mismatched nucleobases.
32. The method of embodiment 31 wherein the active target segment is selected from the group conisiting of Region BA, Region BB, Region BC, Region BD, Region BE, Region BF, Region BG, Region BH, Region BI, Region BJ and Region BK of SEQ ID NO: 5.
33. The method of embodiment 31 wherein the active target segment is selected from the group consisting of Region AA, Region AB, Region AC, Region AD, Region AE, Region AF, Region AG, Region AH, Region AI, Region AJ and Region AK of SEQ ID NO: 4.
34. The method of embodiment 31 wherein the active target segment is selected from the group consisting of Region A, Region B, Region C, Region D, Region E, Region F, Region G, Region H, Region I, Region J and Region K of SEQ ID NO: 3.
35. The method of embodiment 31 wherein antisense compound is a chimeric compound 15 to 35 nucleobases in length and comprising at least one chemical modification.
36. The method of embodiment 35 wherein the chemical modification comprises at least one modified internucleoside linkage, at least one modified nucleobase, at least one modified sugar or a combination thereof.
37. The method of embodiment 36 wherein the modified internucleoside linkage comprises a phosphorothioate linkage.
38. The method of embodiment 36 wherein the modified sugar moiety comprises a high affinity modification comprising a 2'-O-(2-methoxyethyl), a 2-O-methyl, an LNA, an ENA or a combination thereof.
39. The method of embodiment 36 wherein the modified nucleobase comprises a 5-methyl cytosine.
40. The method of embodiment 35 wherein the chimeric antisense compound comprises deoxynucleotides in a first region, at least one high affinity modified sugar in each of a second region and a third region, which flank the first region on the 5' end and the 3' end, respectively, and at least one phosphorothioate modified internucleoside linkage.
41. The method of embodiment 40 wherein the first region is ten deoxynucleotides in length, the second and third regions are each five nucleotides in length and comprise five 2'-O-(2-methoxyethyl) nucleotides, and each internucleoside linkage in the chimeric oligonucleotide is a phosphorothioate.

## Claims

1. An antisense compound targeted to and hybridizable with a nucleic acid molecule encoding LMW-PTPase, wherein the antisense compound comprises a modified sugar and is capable of inhibiting expression of LMW-PTPase.

2. The compound of claim 1, comprising:
(a) 15 to 30 nucleobases in length; or
(b) 18 to 22 nucleobases in length.

3. The compound of claim 1 comprising less than 4 mismatches to the target nucleic acid.

4. The compound of claim 1 wherein the compound further comprises a chemical modification selected from a group consisting of at least one modified internucleoside linkage, at least one modified nucleobase, and a combination thereof.

5. The compound of claim 4 wherein the modified internucleoside linkage comprises a phosphorothioate linkage.

6. The compound of claim 1 wherein the modified sugar moiety comprises a high affinity modification comprising a 2'-O-(2-methoxyethyl), a 2-O-methyl, an LNA, an ENA or a combination thereof.

7. The compound of claim 1 wherein the chimeric antisense compound comprises deoxynucleotides in a first region, at least one high affinity modified sugar in each of a second region and a third region, which flank the first region on the 5' end and the 3' end, respectively, and at least one phosphorothioate modified internucleoside linkage.

8. The compound of claim 7 wherein the first region is ten deoxynucleotides in length, the second and third regions are each five nucleotides in length and comprise five 2'-O-(2-methoxyethyl) nucleotides, and each internucleoside linkage in the chimeric oligonucleotide is a phosphorothioate.

9. A pharmaceutical composition comprising a compound of claim 1 and a pharmaceutically acceptable penetration enhancer, carrier, or diluent.

10. A compound according to any of claims 1-8, for use in therapy.

11. A compound according to any of claims 1-8, for use in:
(i) a method of lowering triglyceride levels in an animal;
(ii) a method of improving insulin sensitivity in an animal;
(iii) a method of lowering blood glucose levels in an animal;
(iv) a method of lowering cholesterol in an animal; or
(v) a method of improving glucose tolerance in an animal.

12. The compound of claim 11 (i), wherein the triglyceride levels are blood, plasma, or serum triglyceride levels.

13. The compound of claim 11 (ii), wherein improvement of insulin sensitivity is measured as a reduction in insulin levels.

14. The compound of claim 11(iv), wherein said cholesterol is:
(a) LDL cholesterol; or
(b) VLDL cholesterol.

15. A compound according to any of claims 1-8, for use in treating:
(i) diabetes, optionally type II diabetes;
(ii) obesity, optionally diet-induced obesity;
(iii) insulin resistance;
(iv) insulin deficiency; or
(v) hypercholesterolemia.


SEQUENCE LISTING

<110> Sanjay K. Pandey
      Robert McKay
      Sanjay Bhanot
      Xing-Xian Yu

<120> COMPOSITIONS AND THEIR USES DIRECTED TO
  LMW-PTPase

<130> RTS-0753WO

<150> 60/684,400
<151> 2005-05-24

<150> 60/742,207
<151> 2005-12-01

<160> 363

<170> FastSEQ for Windows Version 4.0

<210> 1
<211> 1521
<212> DNA
<213> Homo sapiens

<400> 1
gggggcgtgc ggaacggggt gtctcggcgc ctctgcgcgg gaagatggcg gaacaggcta 60
ccaagtccgt gctgtttgtg tgtctgggta acatttgtcg atcacccatt gcagaagcag 120
ttttcaggaa acttgtaacc gatcaaaaca tctcagagaa ttggagggta gacagcgcgg 180
caacttccgg gtatgagata gggaaccccc ctgactaccg agggcagagc tgcatgaaga 240
ggcacggcat tcccatgagc cacgttgccc ggcagattac caaagaagat tttgccacat 300
ttgattatat actatgtatg gatgaaagca atctgagaga tttgaataga aaaagtaatc 360
aagttaaaac ctgcaaagct aaaattgaac tacttgggag ctatgatcca caaaaacaac 420
ttattattga agatccctat tatgggaatg actctgactt tgagacggtg taccagcagt 480
gtgtcaggtg ctgcagagcg ttcttggaga aggcccactg aggcaggttc gtgccctgct 540
gcggccagcc tgactagacc ccaccctgag gtcctgcatt tctcagtcgg tgtgtaatca 600
cgttccaggg cccaaagcca gctctttgtt cagttgactt actgtttctt accttaaaaa 660
gtaattgtag atggaaatca gttgtgtttg gcaggagaat caataaaaat gtttgattca 720
gacagcttat ggggtatttt aagcattctt agactagttg aacatctcac tttgccccag 780
ttacaaaaat agtagaacaa gcaacataaa acaatgaagg aaaacctcac ttgaaggccc 840
aggtcaacat ctaagcctgt tgagacttag ataatcgagt ctacctcttc agtaggtttg 900
tgtggatggc ctggaggcag gtgcttgctc cccagtgcta cctctctctt ccctagggcc 960
ttttgtggat tgacagtagt cccctccgta gagctcacag tctagattag aagtgtttta 1020
atttctacac acccatagtg cacacttgta tattgaaaag atagggaaga gagaaacatt 1080
tatggaatca gtcgttggca ccttcaatac ttcatgattt ttgtcgagtt tacttcatga 1140
ggaggtcagc ccattggctc ccatctgaac cactttgcct ctgaaactta attacatcca 1200
gaaagaagga cacttgtatg ctagtctatg gtcagttgag gaatatgact gtttttatat 1260
gcacatgtaa cccaaatgtc caatataaat tggcttattt tttaaaataa ttttaaaagt 1320
tgggaaaagt gttattattt ggcatgctta aatattgaat aagtattctt catcagcatt 1380
taataaatgt ataggcagat gtaaggtaat ttctgtgtat tttgagataa tgtcaaaatc 1440
atgaatattt caaaataaac tggggagtta taaaaataca actagagata taaaaaaaaa 1500
aaaaaaaaaa aaaaaaaacc c                                           1521

<210> 2
<211> 1468
<212> DNA
<213> Homo sapiens

<400> 2
caggctacca agtccgtgct gtttgtgtgt ctgggtaaca tttgtcgatc acccattgca 60
gaagcagttt tcaggaaact tgtaaccgat caaaacatct cagagaattg ggtcattgac 120
agcggtgctg tttctgactg gaacgtgggc cggtccccag acccaagagc tgtgagctgc 180

ctaagaaatc atggcattca cacagcccat aaagcaagac agattaccaa agaagatttt 240
gccacatttg attatatact atgtatggat gaaagcaatc tgagagattt gaatagaaaa 300
agtaatcaag ttaaaacctg caaagctaaa attgaactac ttgggagcta tgatccacaa 360
aaacaactta ttattgaaga tccctattat gggaatgact ctgactttga gacggtgtac 420
cagcagtgtg tcaggtgctg cagagcgttc ttggagaagg cccactgagg caggttcgtg 480
ccctgctgcg gccagcctga ctagacccca ccctgaggtc ctgcatttct cagtcggtgt 540
gtaatcacgt tccagggccc aaagccagct ctttgttcag ttgacttact gtttcttacc 600
ttaaaaagta attgtagatg gaaatcagtt gtgtttggca ggagaatcaa taaaaatgtt 660
tgattcagac agcttatggg gtattttaag cattcttaga ctagttgaac atctcacttt 720
gccccagtta caaaaatagt agaacaagca acataaaaca atgaaggaaa acctcacttg 780
aaggcccagg tcaacatcta agcctgttga gacttagata atcgagtcta cctcttcagt 840
aggtttgtgt ggatggcctg gaggcaggtg cttgctcccc agtgctacct ctctcttccc 900
tagggccttt tgtggattga cagtagtccc ctccgtagag ctcacagtct agattagaag 960
tgttttaatt tctacacacc catagtgcac acttgtatat tgaaaagata gggaagagag 1020
aaacatttat ggaatcagtc gttggcacct tcaatacttc atgatttttg tcgagtttac 1080
ttcatgagga ggtcagccca ttggctccca tctgaaccac tttgcctctg aaacttaatt 1140
acatccagaa agaaggacac ttgtatgcta gtctatggtc agttgaggaa tatgactgtt 1200
tttatatgca catgtaaccc aaatgtccaa tataaattgg cttatttttt aaaataattt 1260
taaaagttgg gaaaagtgtt attatttggc atgcttaaat attgaataag tattcttcat 1320
cagcatttaa taaatgtata ggcagatgta aggtaatttc tgtgtatttt gagataatgt 1380
caaaatcatg aatatttcaa aataaactgg ggagttataa aaatacaact agagatataa 1440
aaaaaaaaaa aaaaaaaaaa aaaaaccc 1468

<210> 3
<211> 1549
<212> DNA
<213> Homo sapiens

<400> 3
tggcagtgcg cctgcgccgc gtcggcgtgc ggaacgccgc ggtgtctcgg cgcctctgcg 60
cgcgggaaga tggcggaaca ggctaccaag tccgtgctgt ttgtgtgtct gggtaacatt 120
tgtcgatcac ccattgcaga agcagttttc aggaaacttg taaccgatca aaacatctca 180
gagaattgga gggtagacag cgcggcaact tccgggtatg agatagggaa cccccctgac 240
taccgagggc agagctgcat gaagaggcac ggcattccca tgagccacgt tgcccggcag 300
attaccaaag aagattttgc cacatttgat tatatactat gtatggatga aagcaatctg 360
agagatttga atagaaaaag taatcaagtt aaaacctgca aagctaaaat tgaactactt 420
gggagctatg atccacaaaa acaacttatt attgaagatc cctattatgg gaatgactct 480
gactttgaga cggtgtacca gcagtgtgtc aggtgctgca gagcgttctt ggagaaggcc 540
cactgaggca ggttcgtgcc ctgctgcggc cagcctgact agaccccacc ctgaggtcct 600
gcatttctca gtcggtgtgt aatcacgttc cagggcccaa agcccagctc tttgttcagt 660
tgacttactg tttcttacct taaaaagtaa ttgtagatgg aaatcagttg tgtttggcag 720
gagaatcaat aaaaatcttt gattcagaca gcttatgggg tattttaagc attcttagac 780
tagttgaaca tctcactttg ccccagttac aaaaatagta gaacaagcaa cataaaacaa 840
tgaaggaaaa cctcacttga aggcccaggt caacatctaa gcctgttgag acttagataa 900
tcgagtctac ctcttcagta ggtttgtgtg gatggcctgg agggcaggtg ccctctgctc 960
cccagtgcta cctctctctt ccctagggcc ttttgtggat tgacagtagt cccctccgta 1020
ggagctcaca gtctagatta gaagtgtttt aatttctaca cacccatagt gcacacttgt 1080
atattgaaaa gatagggaag agagaaacat ttatggaatc agtcgttggc accttcaata 1140
cttcatgatt tttgtcgagt ttacttcatg aggaggtcag cccattggct cccatctgaa 1200
ccactttgcc tctgaaactt aattacatcc agaaagaagg acacttgtat gctagtctat 1260
ggtcagttga ggaatatgac tgtttttata tgcacatgta acccaaatgt ccaatataaa 1320
ttggcttatt ttttaaaata attttaaaag ttgggaaaag tgttattatt tggcatgctt 1380
aaatattgaa taagtattct tcatcagcat ttaataaatg tataggcaga tgtaaggtaa 1440
tttctgtgta ttttgagata atgtcaaaat catgaatatt tcaaaataaa ctggggagtt 1500
ataaaaatac aactagagat ataaaaaaaa aaaaaaaaaa aaaaaaaaa 1549

<210> 4
<211> 1549
<212> DNA
<213> Homo sapiens

<400> 4
tggcagtgcg cctgcgccgc gtcggcgtgc ggaacgccgc ggtgtctcgg cgcctctgcg 60
cgcgggaaga tggcggaaca ggctaccaag tccgtgctgt ttgtgtgtct gggtaacatt 120
tgtcgatcac ccattgcaga agcagttttc aggaaacttg taaccgatca aaacatctca 180

gagaattggg tcattgacag cggtgctgtt tctgactgga acgtgggccg gtccccagac 240
ccaagagctg tgagctgcct aagaaatcat ggcattcaca cagcccataa agcaagacag 300
attaccaaag aagattttgc cacatttgat tatatactat gtatggatga aagcaatctg 360
agagatttga atagaaaaag taatcaagtt aaaacctgca aagctaaaat tgaactactt 420
gggagctatg atccacaaaa acaacttatt attgaagatc cctattatgg gaatgactct 480
gactttgaga cggtgtacca gcagtgtgtc aggtgctgca gagcgttctt ggagaaggcc 540
cactgaggca ggttcgtgcc ctgctgcggc cagcctgact agaccccacc ctgaggtcct 600
gcatttctca gtcggtgtgt aatcacgttc cagggcccaa agcccagctc tttgttcagt 660
tgacttactg tttcttacct taaaaagtaa ttgtagatgg aaatcagttg tgtttggcag 720
gagaatcaat aaaaatcttt gattcagaca gcttatgggg tattttaagc attcttagac 780
tagttgaaca tctcactttg ccccagttac aaaaatagta gaacaagcaa cataaaacaa 840
tgaaggaaaa cctcacttga aggcccaggt caacatctaa gcctgttgag acttagataa 900
tcgagtctac ctcttcagta ggtttgtgtg gatggcctgg agggcaggtg ccctctgctc 960
cccagtgcta cctctctctt ccctagggcc ttttgtggat tgacagtagt cccctccgta 1020
ggagctcaca gtctagatta gaagtgtttt aatttctaca cacccatagt gcacacttgt 1080
atattgaaaa gatagggaag agagaaacat ttatggaatc agtcgttggc accttcaata 1140
cttcatgatt tttgtcgagt ttacttcatg aggaggtcag cccattggct cccatctgaa 1200
ccactttgcc tctgaaactt aattacatcc agaaagaagg acacttgtat gctagtctat 1260
ggtcagttga ggaatatgac tgtttttata tgcacatgta acccaaatgt ccaatataaa 1320
ttggcttatt ttttaaaata attttaaaag ttgggaaaag tgttattatt tggcatgctt 1380
aaatattgaa taagtattct tcatcagcat ttaataaatg tataggcaga tgtaaggtaa 1440
tttctgtgta ttttgagata atgtcaaaat catgaatatt tcaaaataaa ctggggagtt 1500
ataaaaatac aactagagat ataaaaaaaa aaaaaaaaaa aaaaaaaaa 1549

<210> 5
<211> 1578
<212> DNA
<213> Homo sapiens

<400> 5
tggcagtgcg cctgcgccgc gtcggcgtgc ggaacgccgc ggtgtctcgg cgcctctgcg 60
cgcgggaaga tggcggaaca ggctaccaag tccgtgctgt ttgtgtgtct gggtaacatt 120
tgtcgatcac ccattgcaga agcagttttc aggaaacttg taaccgatca aaacatctca 180
gagaattgga gggtagacag cgcggcaact tccggtgggt cattgacagc ggtgctgttt 240
ctgactggaa cgtgggccgg tccccagacc caagagctgt gagctgccta agaaatcatg 300
gcattcacac agcccataaa gcaagacaga ttaccaaaga agattttgcc acatttgatt 360
atatactatg tatggatgaa agcaatctga gagatttgaa tagaaaaagt aatcaagtta 420
aaacctgcaa agctaaaatt gaactacttg ggagctatga tccacaaaaa caacttatta 480
ttgaagatcc ctattatggg aatgactctg actttgagac ggtgtaccag cagtgtgtca 540
ggtgctgcag agcgttcttg gagaaggccc actgaggcag gttcgtgccc tgctgcggcc 600
agcctgacta gaccccaccc tgaggtcctg catttctcag tcggtgtgta atcacgttcc 660
agggcccaaa gcccagctct ttgttcagtt gacttactgt ttcttacctt aaaaagtaat 720
tgtagatgga aatcagttgt gtttggcagg agaatcaata aaaatctttg attcagacag 780
cttatggggt attttaagca ttcttagact agttgaacat ctcactttgc cccagttaca 840
aaaatagtag aacaagcaac ataaaacaat gaaggaaaac ctcacttgaa ggcccaggtc 900
aacatctaag cctgttgaga cttagataat cgagtctacc tcttcagtag gtttgtgtgg 960
atggcctgga gggcaggtgc cctctgctcc ccagtgctac ctctctcttc cctagggcct 1020
tttgtggatt gacagtagtc ccctccgtag gagctcacag tctagattag aagtgtttta 1080
atttctacac acccatagtg cacacttgta tattgaaaag atagggaaga gagaaacatt 1140
tatggaatca gtcgttggca ccttcaatac ttcatgattt ttgtcgagtt tacttcatga 1200
ggaggtcagc ccattggctc ccatctgaac cactttgcct ctgaaactta attacatcca 1260
gaaagaagga cacttgtatg ctagtctatg gtcagttgag gaatatgact gtttttatat 1320
gcacatgtaa cccaaatgtc caatataaat tggcttattt tttaaaataa ttttaaaagt 1380
tgggaaaagt gttattattt ggcatgctta aatattgaat aagtattctt catcagcatt 1440
taataaatgt ataggcagat gtaaggtaat ttctgtgtat tttgagataa tgtcaaaatc 1500
atgaatattt caaaataaac tggggagtta taaaaataca actagagata taaaaaaaaa 1560
aaaaaaaaaa aaaaaaaa 1578

<210> 6
<211> 14188
<212> DNA
<213> Homo sapiens

<220>
<223> Oligomeric Compound

<400> 6
   caggtacccc tctcctgcgg ccccgtcccc tataggtaac ctttacctcc cgcggctcct 60
   tcccctccaa gcgacccgca gccccgcccc ctccaggtga ctcccccccg ccaacccccg 120
   ccaccacaca cacacacccc ctcgccttcc gcggccctac tccctccagg tgaccccacc 180
   cccgcagctc ctcctcctcc ggacaacctg cagccccgcc cctgcaggtg aacggcggcc 240
   cagtccctgc aggtgacccg cggaccctcc ccgcccgtcc tactaccgtc ataggaccgc 300
   ctccgcaggc gcactggagc cgattgcgca ggcgtggctc tcacacgcgc tgccctgttg 360
   gcgttggtgc gggactgcgc aggcgcgcgg ggcaagaggg tggcagtgcg cctgcgccgc 420
   gtcggcgtgc ggaacgccgc ggtgtctcgg cgcctctgcg cgcgggaaga tggcggaaca 480
   ggctaccaag tccgtgctgt ttgtgtgtct gggtaagagg gcgccgactt actcatgttc 540
   tgacgtcctc tggagagttg gatcgggctt gtgcgctgta ggttgtgccg ccggcctagg 600
   aaccatgagg gggaggaggc caggactggg gaggcctagg gtgttctagg agtgtgccgc 660
   agcgcccctg ttccccatcc gccccgtgca cccgcccagc ctgcccgcta aacctgggtc 720
   ccctcgcgcc tgccatatat ccggggctct tggcatctgc agccacagcc cctacaagcc 780
   aaggtacttt ctttgcgact actagaatcc ctctcgttcc cctccaatgg gcccaggtag 840
   gcctggaggt tgtcggactg atgtggtctc ttaggggcct cctgagtcct atgacaacat 900
   tgttttgttt gctagtttcc ttagcccatg cttagatagg gatctgagtt aaagggagat 960
   gaagttttac ggtttcgggt ttcttgattg taaaagccaa gcaaaggaga cctggaggtc 1020
   cctatataac tttggcccat gaaaaatgaa agtgtatttt attaatggct tttcaggaac 1080
   tttccagctt aaattatttt cactcctaga atttcaacac ttagcttgcc ttacttcaaa 1140
   cataatttat tttccgtttg gtcttcaggg atcttccttt ggtgtagcac tacaaggtta 1200
   tattcctcgg tgcctccacc ctgagaggaa ggcctaaatt gtcagtagtc cggttactta 1260
   aaaccttctg tgagtttgca tttcctgcag tttctgttta tttttacaga ctcctcactt 1320
   tatctcctgt accttttcct gctcacaccc tcgggacact gccttccaga acctttcgtt 1380
   gttcggtagc acttcacatg cttactgagc cttcatcgct gggtgcccat tatgcatttt 1440
   gcctggaaag cctttttatt ttataaagtc aaacctaaat atttcctcct ttgctttccc 1500
   agatcatgag ttgcttcctt atcgacatct tttattacac aaattttact ataatttgtg 1560
   taatatagta aaagatgtaa tacagaaaag gtgtgattcc ttatcgacat cttttattac 1620
   acaaatttta ctataattat ttgatagtct gttgaaatcc cagctgagtt taattttgtc 1680
   gatggcaata actgtggctt atttttcatt ttgttttcag ctttaggaca atatatgata 1740
   aacgctgact aggttttgta gaagttaact tctgtgaaag acgaattaga aggcttacct 1800
   tgtttcacat tggtagtggg catcatttga tttttaagac tcattgaagg ctgaatgatg 1860
   aaatcaataa gtttattaaa cagtagtttg gttaaggatg gttactaaat aatattccat 1920
   tcccttgtat agttttggtt ggtattgtcg attatgctgg cataatgcat catggttgct 1980
   gttaatgggg aagtgtatcc ttgcatatac agtagtccca ctttagctgt aggggataca 2040
   ttccaagact ccccagtgga tcccgaaact gtgggtagta ccaaacccaa ttgctgtcaa 2100
   ttggaacatg tgtctgctcg tttcttacac ccacaaatgt aataccgttg ccaccttaac 2160
   taagcattta aaactgtggt cgcaactttt gcagtttgag gcaagacagc agacgatcag 2220
   gaatttcttt ttccttgttt acattttcat ggatagaaga tttgttcgta ctgtagatct 2280
   tagtaacctc ggtataagtt ttttttttct ttccataaga agtcaaaaac tttcaccttt 2340
   ttacttaagg gtagtgctta tggcttctct ttgccgtatc tgaattgcta gcttcactac 2400
   tcttatgatt tcgggccatt attagtaaac taagggtgac tcgaactcag gcactgtgat 2460
   agcatcacaa tggatctgat aagctagagg gcaataggtg gatagacagc atggatatgc 2520
   tggacaaaga cgtgtttcac ttctggggca ggacggaatg ggatggcatg agaatttatc 2580
   ctgctactca gaacagtgca caatttaaaa cttaggaatt atttgtggaa ttcttcattt 2640
   aatattttcc aactgaagct gaccatgggg taaccatgga aagcaaaacc ttggaaaaga 2700
   ggggactatt gtatttcaat acaattcagt gattttttgt tgttgttgag catctactat 2760
   atggcatgca ttcctctaag tggtgtggtg aacacaacag acaaaaatcc cagcctctct 2820
   ggagctcacg ttctctcagt caccagcaag tattaattga tgtgctcaat cggttgatgt 2880
   gaagattaag aaaaatagag cagaaaaggg gaatgggagt ggagtgcagt tttgagtggg 2940
   atggtcaggg aaggccccac ctgatagatg acttttgagt aaagacttga aggaggtgag 3000
   gaagcaagcc taggatattt tggagaagca tcctgaaccc agaggaaagc aggtaaaagt 3060
   tttgaaggga gaatactggc catggtcatg aaccgcaggg aagctgcagt ggctgggcct 3120
   agtggccaga tagtttttct cagtcaggat tcctcatcta tacctgaaaa tttagcagat 3180
   aatttgagcg cctgttttct cagtgcttcc attgtagatg tatagggaga agttaattcc 3240
   ttatatacac tggtccttta gggcaggagt ctgcaaactc tttgtttaaa gaaccagatt 3300
   ataattattt tagattggct tcacatgaca tacagaaagg gtgtagcaca aagtagccat 3360
   ccatatgatg aacctggctg tgttctagtg gatcttcatt tgtggacatc agaattggat 3420
   ggaactctgt ataattttca catgtcacaa ggtactctac ttttagattt tttccaacta 3480
   ctcaacaatg taaaaattat tcttaccgct gtgggctgta cataaaggca gtggaccatg 3540
   agccgatttt cagacccctg ttttaggtcc agatcttggg aggccatgtg aagacttggg 3600
   cttactctga ataaacattg gagggttttg agcagaggtg ggaagtgatc aaatttaagt 3660
   ttaaaaaaga agagtttcag ctgttgtttt gagatgaaac tggaggtgga gcatgccttt 3720
   actggttgca ttctgaggga atttgagcgg taaatggcat tttacttggt agccatatag 3780
   aggacagtaa gagttgtatg cttttgaaat gtaaaaaact ttgttcattc aaagcatttg 3840
   tttggaccct attgagattg aagtgtagaa aagcatcaga ggtttttcat tggtgaagct 3900
   aatgatggaa aataattgag ctccttacta cagcacaatt atgaagggtg ctttatgttt 3960
   ttgcaataga gacttattag tgaagatgat gcaggagcca tgacccagcc agcatggttg 4020
   gtggagatag cacatgctgg tggagtcact ttacttctgg gctacaccca tactgggtgc 4080
   tgggtctctt accaggtata gcagacacag ccccaccctc ccagagccta tctgcctttt 4140
   atgagtctcc atccttgaat tgaagatgct gaagaagggt ataaaatagt ctgcctaatt 4200
   tggaaaagac aaacttgaga ctttacagat tgaaacattt tttaaaggca tttgaagatg 4260
   gaatgttctg tttttagaac ttgtttgata ggcgaaacct gaagtacatg cagtgtttca 4320
   ctgatgaccg gggtgctcca ttttttcata acaaaatttg gtggcatcaa ctttcgaaat 4380
   caacagggca agctactgct agcctttagg acagttaata atgcaagttt tcatattgtt 4440
   gtggggaaag taagctgtta ttttacagtt aaagataatt atttgtaaac gtgtggggct 4500
   agtctttatt atatttttag atgctgagat actaggcatc tgttgttcta cagatccaaa 4560
   ataataattg tcttaatttg gaatacaatg tttgctgtta aaaatattcc cagggtatat 4620
   ggtgacaagt aaaagagtta cacacccatg ggaagccttt caataattaa aatagtgctg 4680
   atgacaatac ctgtttagaa aactaaacaa aatttagaat gtcgaaagga aaaacttcac 4740
   aacacaaatt agcatataga gggtattcaa ggctaatttg ttgtctttcc ctttatggtt 4800
   tgtaggtaag attttttact gctgtggaaa ctacagtctc tgtgggaaaa aagtacgtaa 4860
   tatgcttagg tcttacaatg tagaaactga aggctgtagt aaggcagtga gttggttttc 4920
   tgaactaggg aattagctgt gttttacaac actagaagat cctcattgta tttgtttatc 4980
   ataaacagaa gaccctaata gtgataaaga gtaaactctt agttagtgct gcctattctc 5040
   acagttcctc tttttcgtat ctgtttccta aattcagctc ctaatgtctg gactcagtgt 5100
   ggtgctttgg cattaaggag actggaaggc ctcagagcag ccccagaagc aaggtcgtag 5160
   aaaccatcat ttatcttccc cagcgcagat catagaaact agaactcctc tcctgcaaag 5220
   caagccataa aacctagaaa gatcactctc tcccttttgc cttctcttga agacagcatt 5280
   tcagaggggc cctgcctcat tcctggggga ggaaatgctg tggccctgct gggtttccct 5340
   cagtctgcta ccattggatc atttcctttt gtccagtgcc atttctacac agctgtgcat 5400
   tggtcatcta agcatcaaaa ccgtttccct gggtctttgg gtcttcattt ctgaaggctc 5460
   ttgttaacct gtcttttgtt acaggagtat cttccatgac ctttatgatg ggtgaggaaa 5520
   gctgccacac ctttccactc tgacactgtc ttgtatttgc tccttcatac gagcctctta 5580
   caaggtctct ccaattcagt catgtgctct tctagtgttt ccttagctgt gccggtaatt 5640
   ttggtaactt gtaggaaggg tctgtgtttt aaacaacttt ctgtcttcag gttagggcac 5700
   agtgcttggc acatagagtt ggcatacaaa atatgtttgt tgaataagtc atattaccct 5760
   tttccatagg actttctatt tctcctcata gtggctacgc aaagttcaaa tgttggccca 5820
   gtgttttagg gctcctgtat accagcccgt cagtttccag acactggctc tctgtacctg 5880
   aagcatcgca gcctcacctg cttagtgcca caatccctct gagttaaatg cctttccctt 5940
   cctctgtcca ttaagatcct gatcatcctt ttaatatcat ctctaatctt tcttttttct 6000
   ggtaagaaat tttcttatat cttgtttttg ctttgtagca tcgttctttt tctgtgctgc 6060
   tgtagtgtat tgtttatcag cattcatttt cccactttta ttataataaa tataataaat 6120
   aaagtggaga tagagttcag ctgaatggcc agtgattcaa cccattgtgt ctccctccga 6180
   aaaccccaat aaaacctcag agcactatgg ctctgaggag cttcctgggt tggcagtgct 6240
   ctttgaatat tgtctcatgt tgatgacagg agagtaaggt gtcccagagg acagtggagc 6300
   tacctgttag ggaacctccc agaatgtgcc ctataggtca cttgctttgg ctgttttgtt 6360
   tgtttgtttg tttttgtttt tgttgctgtt actataatga aattgtaatg gtaagtgttc 6420
   tgctttcctg agtttgggga gtaattctag ggaattatca aacctgaggg agccctgggg 6480
   aacctggatt tctcacttgc tggtctaaag taaggatggc cctgatgtcg acatcgtggt 6540
   ctggagagtg gtgtccctga ccttgggctg ctaatgcctg gatctgtatc cctgggctca 6600
   gggctttgca tgtgatcgct cagtaaatgt tggtgttgaa tcgctgttta tgcttaaatg 6660
   catgtttgtg tgtttgtgta ttgttttgtg aaacagtagt catccttatg tgtgatatac 6720
   ttgaaaatac atacagttga cccttcagcc acaggggttt gaactttgaa ggatttattc 6780
   gaggaatttt tttaatcaag cacagatgga aaataccgtg ttgtctgaat gagaaaccca 6840
   tctgtatgga gggctgactt ttcctatact tgggctccac attctcaggg gccaactata 6900
   ggacctgagt atgcacgttt tggtatactg ggtaggggtg ggagggtgtc ctgaaccagc 6960
   tcctgagtct actgagggac agaccactgt attttatttt tattctgttg tatttcattt 7020
   taaaataagc tggttgcaac ccacttcatt ttcgtgattc actcctggat cattagtagc 7080
   aactctttaa acaataattt ttgttgaaat tgtttttctg atatctttgt catctgtact 7140
   tggagtactt ggctggttgg tgtcaaatca ctgaacttga gcctgatagg ctgttctcca 7200
   gcagggtcta ccctcactgg ttggacaaag gcgtcaaagg atagtgttgg cctttgtttt 7260
   tcctgaggga tagcacagcc cccgtgtgtc aggtgtgtaa agaaggggag ctggcatgtg 7320
   cccttccatc caacctaacc ctgtttcccc acccctccct ttttttaaag gtaacatttg 7380
   tcgatcaccc attgcagaag cagttttcag gaaacttgta accgatcaaa acatctcaga 7440
   gaatgtaagt accattcatt atcttaaaga ggccaacctg aactcctctg ggcaggaaat 7500
   tgcaaaaaaa aaaaaaaaat tccatgtttc ttcccctgca gtggagggta gacagcgcgg 7560
   caacttccgg gtatgagata gggaaccccc ctgactaccg agggcagagc tgcatgaaga 7620
   ggcacggcat tcccatgagc cacgttgccc ggcaggtacc gtccttggac ttgaagttgt 7680
   gtgttttgtg tttcagtggg tcattgacag cggtgctgtt tctgactgga acgtgggccg 7740
   gtccccagac ccaagagctg tgagctgcct aagaaatcat ggcattcaca cagcccataa 7800
   agcaagacag gtagacaagc tcttgttcaa tttctaatat atagagtcca gtaacttgag 7860
   aagtagcgaa aggattaacc agacttgtat attaatgaat gtgtttattt agggtgagct 7920
   taaccagcta tggtgtgtcc attttgtttc acttctggtt gcacggtgtt gaaagacttg 7980
   cctgactttg gaatttactt attaaaatgc acataaaagc taggtaattt ataatgagag 8040
   agcctgactg tgagctgggg ctgagcggtg ctctgtcttc tgttccttcc tgcataattt 8100
   ttattaaaca tttaggccat agtaatcatc ctgctgatat tgcaagtttg ttgctagaat 8160
   gaggttatat aatatataca aaaacatttt ttcaactgta aagtgcctta gtaatatagg 8220
   gtaataccag caacattatg gatatataat tatagtctat tgggccacac ttaagtttgg 8280
   agtctaataa agtcacaatc aaattctgca atttcaattg aagataacct tgtctttata 8340
   ttatgaatta gaagctaaag ttgatttttc taagagttct ttatttaaat gaagtactct 8400
   gggactgacc ttttcggaaa tggaatcttc attggtcagg tgattcaaca tttttataca 8460
   atttatccat cctcatctct tcaggatttg cataccttgc cagtttctac tggccattgt 8520
   tgaaaataca tttatttgga gaagtccaaa gccaaggggc tcatggggct gtgaagtcct 8580
   tcttgctgca tcgtcctgtg gtagaaggtg gaggagtcaa gagagtgccc cagagtgagt 8640
   gagagcgaga actagaaaaa cgggaagagg gaaacagagg agagagagag agaggaccca 8700
   tcagtgtcag gagcccactc ccaagatagt ggcattaata aagatcctgc gtctcactat 8760
   tgttgcattg gggatgaagt ttccaacaaa ggaactttgg gggacacatc caaaccataa 8820
   cataggattt aaataatttt acagagttca agagttctgc tactgaaccg tttgagatcc 8880
   ctgttctgag gtctcatcac tttccagttt tagcaggaag agaagtggca agtggcagga 8940
   gtctgcagat tggggcctgc accttttttt gaggcacctt ttttatgaac agtgtttgtt 9000
   ggaacacagc cagactcact cattcacctg tagttcgcgg ctgcttttgt gctagagcag 9060
   cacagccaga gcggcctggt ggccacaaag cccgactgtc tctgcagcac tggtttttta 9120
   tatggcgttt ctagttgttt ttagtagtag gggtgatttg ccacaagctg tttcatttat 9180
   agctgcaagt ggaaatcctt tattgtgcat ttgttacata agttatagct gtttctttct 9240
   caattattgt aacatctaaa aattatgtaa cagttactaa gtcttttttt atgaaaaatt 9300
   tacaattagt ccagaattta aaacaagttt agtattaaat cctagggaat ttctcacata 9360
   actaatttgg agaaataata taatactgtt caggcatggt ggctcacact tataatccta 9420
   ataacacttt gggaggctca acattgcttg agcctaggag gtcaagacca gcctgagcaa 9480
   catagtgaga cccatctcta ccaaaaaaat tttaaaaatt agctgggtgc tatgacatat 9540
   gcctgtagtc ccagctgttt gggaggctga ggtaggaggt tggcttgaac tcaagaagtt 9600
   gaggctgcag tgagctatga tcacaccact gtaccccagt gttggtgaca gagtgagacc 9660
   ttttctctaa aaacaaaaag aaaaaattca gtattgtact tattctcaat tttgaaataa 9720
   gtcaatgtca tgggagtgtt gacgaaatac agtcttttct agctactgga agtgcatact 9780
   aaaagccaaa gtttctcatt ttttagaaga acaggtatac actttccacc tttgttccgc 9840
   agtacgtatg tttatgtttc ttattctgcc attatttata gtagtcgaaa ttcacaaaat 9900
   aatcttttca tttgtatttt aagtaggtgt gtttatagat tatatcagaa aacaatcata 9960
   agctttcagg ttacaatcag tagaaatact gagtggcatt ctcttctgtg tgaggtttaa 10020
   attatggata gtcaatcaaa ataaaggagg actgtcagtt atcactcctg tgtttctttc 10080
   ttagtgacca cctctgacca cccaacttaa tacttttact cctccctgcc ccaaagctgt 10140
   atgtatctcc tctctgcttt atttcctcca gggcatctgt gcctcctgtg tgttgcctgc 10200
   ccccatgtgg aagggcagct ccatgggacg cggctctgtc cacttagctt ggtgtttgtt 10260
   actgggcccc aggatgttca gtgcttgtgg aatgaaaaat agagtagaat aatgaagggg 10320
   attaaatatg tgacatttta gtatgttgac tgtattatac actgctacta aggaattgaa 10380
   gccgatgtat aaacattgtg tctacacatt atactatttt atgattattg tatggaactt 10440
   tataatacaa aatttctttg cagtacaatt ttgagaaata ggttaactct attttaactt 10500
   aaaagtaccc taaaaattat ttaattgttt attagtgagt aacaggctca aatacagcag 10560
   tttttttttt cttttagtat tgctttgcat cctctaggct tgaatggtat aaacactgtg 10620
   ttttgacttc ttattcaatt ttagattacc aaagaagatt ttgccacatt tgattatata 10680
   ctatgtatgg atgaaagcaa tctgaggtaa tcctgttttt gaagaatatt tctgttcaac 10740
   tctcagttca gcagtgggcc aagtaatttg ttgtccagat ttactttttc tattttaaag 10800
   gttttaatag tcagtgatgg tcaccatatg tagaattatt ttatttagaa cagcaaaaaa 10860
   cttagtaatc tagaattgtc tcttaggtat ttacaaggaa atacacctta gaaaagggaa 10920
   agtctagttg ttaatagcat gatttaattg gaaaactagc ttggctgtta tagttttgta 10980
   tcaagcatat tgttctgcat gagaacgatt ttgatatttt tgctgtaatg attcctgggc 11040
   agggcagtgt tttattgatt ctcaggtcaa agctctgcaa ccataggttc ctattattat 11100
   cccatcttgt aaaagaagga agtgagaaac ggtaatgtta ggttatgtcc ccgagttcac 11160
   acagctagca tgtggtggat gtcatgggat ttaagcctag gctcagtgag gaagggccct 11220
   gctgtatgca cccctgcctc agtcaccacc ctcctgacct gccccagtcc catgttcaag 11280
   gcgtcatcgg gaacggatca taatttaatg tcactccagg attctgactt gcatcactac 11340
   ttattcataa atatctcatg tttttgatga agataaacta tttgtgcttt taattacttt 11400
   tgtacaaatg aacagatagg gatgcaccag tttcaattat tttttattaa ttaggacctt 11460
   cattaaaatg catgtatttt ttaattttta tttttcagtc cttattctgt tctcattgtt 11520
   tttgctgaca ccatagccca taggcatcgt aaaaacacct ttttaggaga ccccatttgc 11580
   tttgactggc actgagcacc gtgtttcttt gcgtgtgggg caagtggagt tcctgcagcc 11640
   tcacatttgc acgtgctgtt cctctgcctg gaagctcctc cttgcttctt gggtcattcg 11700
   gctcctattt gcccctcgtg ggtcagctta aattcatttc ttgatagagg tattctgctg 11760
   ccctgtcatt aggccagatt gtgcattaga cttaggcctg cctgcagttc tcattcctgc 11820
   acctaatcct gagctgtaaa acttcgtgca tgggagctct tggctgttcc gttcccacat 11880
   aaagcacgta cctgactcat caactcatct gtcgcaggtg ctcagtcgaa atttgttaaa 11940
   taaatggagg cgcttagtta agtttgcctt tttcttttgt agcaatgtga aagctaaggg 12000
   tggaagtcta gagtgaagct gagttttcag cttgggcaga ggcttaagga ataaaagatg 12060
   gaagagtaat taaggagtgg gtcgtgctgt gggaagtgct gtgtcaagac acctgaggat 12120
   ggaattggta gccctctgtt ttgggactgg ttcagatggt gttttgggtt cctttctatg 12180
   acctttatct ctgaaacttg attgtcttaa aatgtatttt gtgggagaaa atataattat 12240
   tgtatatttt gtgtaacaga atcagtgaga ataagctgct gtcgcaaact gtcttgccta 12300
   gagagagggc agtggcatac agctgcagaa ggggccacac gggcctgctg agtgttccgt 12360
   ttcatttcaa attaggtcat tctgtcttga ttttgatatg gatgtttcag aacaccctag 12420
   cagatgtccc tgtttaactt gaaaccatag atcagaaaac taagttcata tttcaatttt 12480
   acagagattt gaatagaaaa agtaatcaag ttaaaacctg caaagctaaa attgaactac 12540
   ttgggagcta tgatccacaa aaacaactta ttattgaaga tccctattat gtaagtacag 12600
   ttcacgtttt agggctaata tgaagaccca acacatttgt atcctgccat attaaataac 12660
   agatgagatt gtgttaagga tgtttttgtt atgcaggttt tgccattttc ttctttttcc 12720
   tgtccattta ggggaatgac tctgactttg agacggtgta ccagcagtgt gtcaggtgct 12780
   gcagagcgtt cttggagaag gcccactgag gcaggttcgt gccctgctgc ggccagcctg 12840
   actagacccc accctgaggt cctgcatttc tcagtcggtg tgtaatcacg ttccagggcc 12900
   caaagcccag ctctttgttc agttgactta ctgtttctta ccttaaaaag taattgtaga 12960
   tggaaatcag ttgtgtttgg caggagaatc aataaaaatc tttgattcag acagcttatg 13020
   gggtatttta agcattctta gactagttga acatctcact ttgccccagt tacaaaaata 13080
   gtagaacaag caacataaaa caatgaagga aaacctcact tgaaggccca ggtcaacatc 13140
   taagcctgtt gagacttaga taatcgagtc tacctcttca gtaggtttgt gtggatggcc 13200
   tggagggcag gtgccctctg ctccccagtg ctacctctct cttccctagg gccttttgtg 13260
   gattgacagt agtcccctcc gtaggagctc acagtctaga ttagaagtgt tttaatttct 13320
   acacacccat agtgcacact tgtatattga aaagataggg aagagagaaa catttatgga 13380
   atcagtcgtt ggcaccttca atacttcatg atttttgtcg agtttacttc atgaggaggt 13440
   cagcccattg gctcccatct gaaccacttt gcctctgaaa cttaattaca tccagaaaga 13500
   aggacacttg tatgctagtc tatggtcagt tgaggaatat gactgttttt atatgcacat 13560
   gtaacccaaa tgtccaatat aaattggctt attttttaaa ataattttaa aagttgggaa 13620
   aagtgttatt atttggcatg cttaaatatt gaataagtat tcttcatcag catttaataa 13680
   atgtataggc agatgtaagg taatttctgt gtattttgag ataatgtcaa aatcatgaat 13740
   atttcaaaat aaactgggga gttataaaaa tacaactaga gatataaatc tggtgtctgc 13800
   ctgttttttt attgacaggt aaggaagcat ttgaaaacat tgtattgtcc tttttacatg 13860
   atctgtgatg ttaggtgtgc atattttatc ttcaggataa ggaagtaaaa tgatgaaaat 13920
   aagcacattg ctgccccatg tcatcatgtc tagtttctga aggaagtttg aggttctaca 13980
   gctggaactg tccttgccca gtaccatcct ctggacatgt tgcctctacg ctgtgccctc 14040
   ctgtgcagtg atgcacagct gcctggccag gacatctgca ctcctgacct cagtatcatg 14100
   agggtcccag gcagtgccat ggggaggaga cacgtttgat ccttgactcc ctagtatctg 14160
   gaggataagc tgttgccaga tgaaagtg 14188

<210> 7
<211> 10191
<212> DNA
<213> Homo sapiens

<220>
<221> misc_feature
<222> 289
<223> n = A,T,C or G

<400> 7
   aagctttctc tgctccctcg gaacaggaaa gccctttatt tattcctgcc cttcctcttc 60
   tccaactgaa gagtgtgatc ttggtacaga gcagttccac acatgccaca ctggtctgat 120
   cttttagagt aaaaaatggg atcttctcag agaaaatttt tttaaaaact tcatgtgctt 180
   aggtctcaga gcagagaatt ttgctcttat catgacgcca ctcactggca aacaagctgg 240
   cgcacaggga cttgtgaggc tctcagcaga gtcagggtag cagttattnc cttgactgct 300
   caacttctat gtttgtgttg acatgccaga tccttatttc tgcggaatga gaaatctctt 360
   gagaataaag ttttcctaaa cagaacaaaa gtatcaaatc acttttgatg agcaaacatt 420
   attttaaact tttttttaac ttacaaaaga tgaggggtaa catttactag ttttacagaa 480
   attttcttta aacagaacat tagtgaacca aataaccaat tctcctcaaa catgatagac 540
   taaacatcag tcgctgcaca agtattttaa taacaggcca gcccacggga gtaaacagca 600
   ccttagcctc gagacctgtt ctctcaactg tgtgtacaga ttttctaagt cgttatgaat 660
   tcagatattc cctacatctc aataaaaaca cctaggagaa caagaaatca ggacatgcat 720
   ctcctgtaag cagaagagaa accgagcacc tgctctcttg cgggggggca gcttcgtgtc 780
   actttccaac tcagcctcca caggagcatg ggcatattgg ttcaaatgaa gtgtaaagga 840
   cacatctctg tccacagtgt aaatcctatt tccttaaggt ggtcaaccga ttgagaccag 900
   ttccacactt ccttaaagaa ggaaaaactc agcagccagc gccaaggccc agcgatcaga 960
   gaggacccgt gctcgccgcg accccgctcg ccctgtggcc ccgcgacccc ggcgcaggtg 1020
   ttggggggac ggcccttccc ttccggtgtg aggggctggc ttctcttcag gttgggggct 1080
   ggcaaactcg aacctcgccg gcgcctagca aaggcttcct ccccggcgcc ttcggcccgc 1140
   gtttccaacg cacattccaa gcgcgcgcat tcgacccaaa cttcagagac gcttctgtgg 1200
   aacggaacgg atggtcgctg acacctcgcc gttcaaatat caggaagtta aaaataacac 1260
   gaccatcgtc atcattccaa acttcggaat agcttttcta aaaatggaaa agacggtggc 1320
   taaacttcaa tcaaaatcgc tgtggtcaag aaagtcctgg aagctgctct atcttaactt 1380
   tgggcggttt gcggttaaca gacgcgcgac ctagaaaccc cagaggcatc gccggttttc 1440
   ccgtcctcct cctcccacca ccgcccagct cttgagaggg gagggtgctg ccggacaggt 1500
   gggtccccgg gtactcactg ctgcccgccc ggccgcggcg ccccgtcccg aggctgccca 1560
   ggaagaggaa ggcgcgctgc cccgccccgc ggacaaggag gccccagcga gggcgtacct 1620
   gcggcaggtg acgaaggagg cggcgcaaaa cacccgccgt gtacgtttcg cgggacaaaa 1680
   accacgcgcc cgccgggccg cgctcaggct tcgcctcagg acttcggaac cgccccgtcc 1740
   tcaagatcga aaagcccaga gccgcgcgct caagcacggt gttgggggtg gggtctcagg 1800
   agcgcccagc aaggcgcccc tgtcggcgtg gacccgcggg ctcaaaggca gttccccggt 1860
   gacccgccca gcccctctat gcgaactcga acgacagcac cacagcccgc cagcgtcgag 1920
   actcgcgctg tgccccaacc caggtgggcg ccgcggagcc gcgagcctga gcccgccctg 1980
   caggtgaccc gcggcccttc ctcctccagg taccccttct cctgcggccc cgtcccctat 2040
   aggtaacctt tacctcccgc ggctccttcc cctccaagcg acccgcagcg cccgggccct 2100
   ccaggtgact gcgcccccgc caacccccgc cccaccacac acacacaccc cctcgccttc 2160
   ttccgcccta ctccctccag gtgaccccac ccccgcagct cctcctcctc cggacaacct 2220
   gcagccccgc ccctgcaggt gaacggcggc ccagtccctg caggtgaccc gcggaccctc 2280
   cccgcccgtc ctactaccgt cataggacgc ctccgcaggc gcactggagc cgattgcgca 2340
   ggagccgatt gcgcaggcgt ggctctcaca cgcgctgccc tgttggcgtt ggtgcgggac 2400
   tgcgcaggcg cgcgggggca agactggcag tgcgcctgcg cgcgtcggcg tgcggaacgc 2460
   cgcggtgtct cggcgcctct gcgcgcggga agatggcgga acaggctacc aagtccgtgc 2520
   tgtttgtgtg tctgggtaag agggcgccga cttactcata gttctgacgt cctctggaga 2580
   gttggatcgg gcttgtgcgc tgtaggttgt gccgccgcgc taggaaccat gagggggagg 2640
   aggccaggga ctggaggcct agggtgttct aggagtgtgc cgcagcgccc ctgttcccca 2700
   tccgccccgt gcacccgccc agcctgcccg ctaaacctgg gtcccctcgc gcctgccata 2760
   tatccgggcc tcttggcatc tgcagccaca gcccctacaa gccaaggtac tttctttgcg 2820
   actactagaa tccctctcgt tcccctccaa tgggcccagg taggcctgga ggttgtcgga 2880
   ctgatgtggt ctcttagggg cctcctgagt cctatgacaa cattgttttg tttgctagtt 2940
   tccttagccc atgcttagat agggatctga gttaaaggga gatgaaagtt ttaccgtttc 3000
   gcggtttctt gcattgtaaa agccaagcaa aggagacctg gaggtcctat ataactttgc 3060
   ccatgaaaat gaaagtgtat ttatatgctt tcaggaactt ccagcttaaa ttattttcac 3120
   tcctagaatt tcaacactta gcttgcctta cttcaaacat aatttatttt cgtttggtct 3180
   tcagggatct tcctttggtg tagcactaca aggttatatt cctcgtgcct ccaccctgag 3240
   aggaaggcct aaattgtcag tagtccggtt acttaaaacc ttctgtgagt ttgcatttcc 3300
   tgcagtttct gtttattttt acagactcct actttatctc ctgtaccttt tcctgctcac 3360
   accctcggga cactgccttc cagaaccttt cgttgttcgg tagcacttca catgcttact 3420
   gagccttcat cgctgggtgc ccattatgca ttttgcctgg aaagcctttt tattttataa 3480
   agtcaaacct aaatatttcc tcctttgctt tcccagatca tgagttgctt cttatcgcat 3540
   cttttattac acaaatttac tataattatt tgatagtctg ttgaaatccc agctgagttt 3600
   aattttgtcg atggcaataa ctgtggctta tttttcattt tgttttcagc tttaggacaa 3660
   tatatgataa acgctgacta ggttttgtag aagttaactt ctgtgaaaga cgaattagaa 3720
   ggcttacctt gtttcacatt ggtagtgggc atcatttgat ttttaagact cattgaaggc 3780
   tgaatgatga aatcaataag tttattaaac agtagtttgg ttaaggatgg ttactaaata 3840
   atattccatt cccttgtata gttttggttg gtattgtcga ttatgctggc ataattgcat 3900
   catggttgct gttaatgggg aagtgtatcc ttgcatatac agtagtccca ctttagctgt 3960
   aggggataca ttccaagact ccccagtgga tcccgaaact gtgggtagta ccaaacccaa 4020
   ttcctgtcaa ttggaacatg tgtctgctcg tttcttacac ccacaaatgt aataccgttg 4080
   ccaccttaac taacatttaa aactgtggtc gcaacttttg cagtttgagg caagacagca 4140
   gacgcatcag gaatttcttt cctgtttaca ttttcatgga tagaagattt gttcgtactg 4200
   tagatcttag taaccttgtt tacattttca tggatagaag atttgttcgt actgtagatc 4260
   ttagtaacct cggtaataag tttttttttt ctttccataa gaagtcgaaa aagctttcac 4320
   ctttttactt aagggtagta gcttatggct tctctttgcc gtatctgaat tgctagcttc 4380
   actactctta tgatttcggg ccattattag taaactaagg gtgactcgaa ctcaggcact 4440
   gtgatagcat cacaatggat ctgataagct agagggcaat aggtggatag acagcatgga 4500
   tatgctggac aaagacgtgt ttcacttctg gggcaggacg gaatgggatg gcatgagaat 4560
   ttatcctgct actcagaaca gtgcacaatt taaaacttag gaattatttg tggaattctt 4620
   catttaatat tttccaactg aagctgacat ggggtaacga tggaagcaaa accttggaaa 4680
   agaggggact attgtatttc aatacaattc agtgattttt tgttgttgtt gagcatctac 4740
   tatatggcat gcattcctct aagtggtgtg gtgaaccaaa cagacaaaaa tcccagcctc 4800
   tctggagctc acgttctctc agtcaccagc aagtattaat tgatgtgctc aatcggttga 4860
   tgtgaagatt aagaaaatag agcagaaagg ggaatgggag tggagtgcag ttttgagtgg 4920
   gatggtcagg gaaggcccca cctgatagat gacttttgag taaagacttg aaggaggtga 4980
   ggaagaagcc taggatattt tggagaagca tcctgaaccc agaggaaagc aggtaaaagt 5040
   tttgaaggga gaatactggc catggtcatg aaccgcagga agctgcagtg gctgggctag 5100
   tgcagcgata gtttttctca gtcaggattc ctcatctata cctgaaaatt tagcagataa 5160
   tttgagcgcc tgttttctca gtgcttccat tgtagatgta tagggagaag ttaattcctt 5220
   atatacactg gtcctttagg gcaggagtct gcaaactctt tgtttaaaga accagattat 5280
   aattatttta gattggcttc acatgacata cagaaagggt gtagcacaaa gtagccatcc 5340
   atatgatgaa cctggctgtg ttctagtgga tcttcatttg tggacatcag aattggatgg 5400
   aactctgtat aattttcaca tgtcacaagg tactctactt ttagattttt tccaactact 5460
   caacaatgta aaaattattc ttaccgctgt gggctgtaca taaaggcagt ggtccatgag 5520
   ccgattttca gacccctgtt ttaggtccag atcttgggag gccatgtgaa gacttgggct 5580
   tactctgaat aaacattgga gggttttgag cagaggtggg aagtgatcaa atttaagttt 5640
   aaaaagaaga gtttcagctg ttgttttgag atgaaactgg aggtggagca tgcctttact 5700
   ggttgcattc tgagggaatt tgagcggtaa atggcatttt acttggtagc catatagagg 5760
   acagtaagag ttgtatgctt ttgaaatgta aaaaactttg ttcattcaaa gcatttgttt 5820
   ggaccctggt gagattgaag tgtagaaaag catcagaggt ttttcattgg tgaagctaat 5880
   gatggaaata taattgagct ccttactaca gcacaattat gaagggtgct ttatgttttt 5940
   gcaatagaga cttattagtg aagatgatgc aggagccatg acccagccag catggttggt 6000
   ggagatagca catgctggtg gagtcacttt acttctggag ctcacatact gggtgctggg 6060
   tctcttacca ggtatagcag acacagcccc accctcccag agcctatctg ccttttatga 6120
   gtctccatcc ttgaattgaa gatgctgaag aagggtataa aatagtctgc ctaatttgga 6180
   aaagacaaac ttgagacttt acagattgaa acatttttta aaggcatttg aagatggaat 6240
   gttctgtttt tagaacttgt ttgataggcg aaacctgaag tacatgcagt gtttcactga 6300
   tgaccggggt gctccatttt ttcataacaa aatttggtgg gcatcaactt tcgaaatcaa 6360
   cagggcaagc tagccattta gacagttaat aatgcaagtt ttcatattgt tgtggggaaa 6420
   gtaagctgtt attttacagt taaagataat tatttgtaaa gctgtggggc tagtctttat 6480
   tatattttta gatgctgaga tactaggcat ctgttgttct acagatccaa aataataatt 6540
   gtcttaattt ggaatacaat gtttgctgtt aaaaatattc ccagggtata tggtgacaag 6600
   taaaagagtt acacacccat ggaagccttt caataattaa aatagtgcct gatgacaata 6660
   cctgtttaga aactaaacaa aatttagaat gtcgaaagaa aacttcacaa cacaaattag 6720
   catatagagg tattcaagct atttgtgtct ttccctttat cggttgtagg tagattttta 6780
   ctgctgtgaa actacagtct ctgtgggaaa aaagtacgta atatgcttag gtcttacaat 6840
   gtagaaactg aaggctgtag taaggcagtg agttggtttt ctgaactagg gaattagctg 6900
   ttttacaaca ctagaagatc cttcattgta tttgtttatc ataaacagaa gaccctaata 6960
   gtgataaaga gctaaactct tagttagtgc tgcctattct cacagttcct ctttttcgta 7020
   tctgtttcct aaattcagct cctaatgtct ggactcagtg tggtgctttg gcattaagga 7080
   gactggaagg cctcagagca gccccagaag caaggtcgta gaaaccatca tttatcttcc 7140
   ccagcgcaga tcatagaaac tagaactcct ctcctgcaaa gcaagccata aaacctagaa 7200
   agatcactct ctcccttttg ccttctcttg aagacagcat ttcagagggg ccctgctcat 7260
   tcctggggga ggaaatgctg tggtcctgct gggtttccct cagtctgcta ccattggatc 7320
   atttcctttt gtccagtgcc atttctacac agctgtgcat tggtcatcta agcatcaaaa 7380
   ccgtttccct gggtctttgg gtcttcattt ctgaaggctc ttgttaacct gtcttttgtt 7440
   acaggagtat cttccatgac ctttatgatg ggtgaggaaa gctgccacac ctttccactc 7500
   tgacactgtc ttgtatttgc tccttcatac gagcctctta caaggtctct ccaattcagt 7560
   catgtgctct tctagtgttt ccttagctgt gccggtaatt ttggtaactt gtaggaaggg 7620
   tctgtgtttt aaacaacttt ctgtcttcag gttagggcac agcttggcat agagttggca 7680
   tacaaaatat gtttgttgaa taagtcatat tatccctttt ccataggact ttctatttct 7740
   cctcatagtc ggctacgcaa agttcaaatg ctggcccagt gtttagggct cctgtatacc 7800
   agcccgtcag tttcagacac tggctctctg tacctgaagc atcgcagcct caacctgctt 7860
   agtgccacaa tccctctgag ttaaatgcct ttcccttcct ctgtccatta agatcctgat 7920
   catcctttta atatcatctc taatctttct tttttctggt aagaaatttt cttatatctt 7980
   gtttttgctt tgtagcatcg ttctttttct gtgctgctgt agtgtattgt ttatcagcat 8040
   tcattttccc acttttatta taataaatat aataaataaa gtggagatag agttcagctg 8100
   aatggccagg tgattcaacc cattgtgtct ccctccgaaa aacccccaat acacacgacg 8160
   cactatgctg ctgaaggagc ttcctgggtt ggcagtgctc tttgaatatt gtctcatgtt 8220
   gatgacagga gagtaaggtg cccagagggc acagtggagc tacctgttag gaacctccca 8280
   gaaatgccct ataggtcact tgctttgggg ctgttttgtt tgtttgtttg tttgtttttg 8340
   tttttgttgc tgttactata atgaaattgt aatggtaagt gttctgcttt cctgagtttg 8400
   gggagtaatt ctaggggatt atcaaacctg agggagccct ggggaacctg gatttctcac 8460
   ttgctggtct aaagtaagga tggccctgat gtcgacatcg tggtctggag agtggtgtcc 8520
   ctgaccttgg gctgctaatg cctggatctg tatccctggg ctcaggcttt gcatgtgatc 8580
   gctcagtaaa tgttggtgtt gaatcgctgt ttatgcttaa atgcatgttt gtgtgtttgt 8640
   gtattgtttt gtgaaacagt agtcatcctt atgtgtgata tacttgaaaa tacatacagt 8700
   tgacccttca gccacagggg tttgaacttt gaaggattta ttcgaggaat ttttttcaat 8760
   caagcacaga tggaaatacc gtgttgtctg aatgcagaaa cccatctgta tgagggcctg 8820
   actttcctat actgggctca cattctcagg gccaactata gaccctgagt atgcagcgtt 8880
   ttggtatact gggtagggtg ggagggtgtc ctgaaccagc tgcctgagtc tactgaggga 8940
   cagaccactg tattttattt ttattctgtt gtatttcatt ttaaaataag ctggttgcaa 9000
   cccacttcat tttcgtgatt cactcctgga tcattagtag caactcttta aacaataatt 9060
   ttgttgaaat tgtttctgat atctttgtca tctgtacttg gagtacttgg ctggttggtg 9120
   tcaaatcact gaacttgagc ctgataggct gttctccagc agggtctacc ctcactggtt 9180
   ggacaaaggc gtcaaaggat agtgttggcc tttgtttttc ctgagggata gcacagcccc 9240
   cgtgtgtcag gtgtgtaaag aaggggagct ggcatgtgcc cttccatcca acctaaccct 9300
   gtttccccac ccctcccttt ttttaaaggt aacatttgtc gatcacccat tgcagaagca 9360
   gttttcagga aacttgtaac cgatcaaaac atctcagaga atgtaagtac cattcaggat 9420
   cttaaagagg ccaacctgaa ctcctctggg caggaaattg caaaaaaaaa aaaaaattcc 9480
   atgtttcttc cctgcagtgg agggtagaca gcgcggcaac ttccgggtat gagataggga 9540
   acccccctga ctaccgaggg cagagctgca tgaagaggca cggcattccc atgagccacg 9600
   ttgcccggca ggtaccgtcc ttggacttga agttgtgtgt tttgtgtttc agtgggtcat 9660
   tgacagcggt gctgtttctg actggaacgt gggccggtcc ccagacccaa gagctgtgag 9720
   ctgcctaaag aaatcatggc attcacacag cccataaagc aagacaggta gacaagctct 9780
   tgttcaattt ctaatatata gagtccagta acttgagaag tagcgaaagg attaaccaga 9840
   cttgtatatt aatgaatgtg tttatttagg gtgagcttac cagctatgtg tgtccatttt 9900
   gtttcacttc tggttgcacg gtgttgaaag rcttgcctga ctttggaatt tacttattaa 9960
   aatgcacata aaagctaggt aatttataat gagagagcct gactgtgcgc tggggctgag 10020
   cggtgctctg tcttctgttc cttcctgcat aatttttatt aaacatttag gccatagtaa 10080
   tcatcctgct gatattgcaa gtttgttgct agaatgaggt tatataatat atacaaaaac 10140
   attttttcaa ctgtaaagtg ccttagtaat atagggtaat accagcaaca t 10191

<210> 8
<211> 769
<212> DNA
<213> Homo sapiens

<220>
<221> misc_feature
<222> 43
<223> n = A,T,C or G

<400> 8
tatggatata taattatagt ctattgggcc acacttaagt ttnttgagaa tagtactcat 60
tactacagta ccctaaatta ttatgttatt agtgagtaac aggctcaata cagcagtttt 120
tttttctttt tagtatgctt tgcatcctct aggcttgaat ggtataaaca ctgtgtttcg 180
acctcttatt caattttaga ttaccaaaga agattttgcc acatttgatt atatactatg 240
tatggatgaa agcaatctga ggtaatcctg tttttgaaga atatttctgt tcaactctca 300
gttcagcagt gggccaagta tttgttgtcc agatttactt tttctatttt aaaggtttta 360
atagtcagtg atggtcacca tatgtagaat tattttattt agaacagcaa aaacttagta 420
atctagaatt gtctcttagg tatttacaag gaaatacacc ttagaaaagg gaaagtctag 480
ttgttaatag catgatttaa ttggaaaact agcttggctg ttatagtttt gtatcaaagc 540
atattgtttc tgcatgagaa acgattttga tatttttgct gtaatgattc ctgggcaggg 600
cagtgtttta ttgattctca ggtcaaacgt ctgcaccata ggttcctatt attatcccat 660
cttgtaaaag aaggaagtga gaaacggtaa tgttaggtta tgtccccgag ttcacacagc 720
tagccatgtg tggatgtcat gggatttaag ccctaggact tcagtagga 769

<210> 9
<211> 2053
<212> DNA
<213> Homo sapiens

<400> 9
ctttatctga aacttgattg tcttaaatgt atttgtggag aaatataatt attgtatatt 60
ttgtgtaaca gaatcagtga gaataagctg gtcgcaaact gtcttgccta gagagagggc 120

agtggcatac agctgcagaa gcgcgacacg ggcctgctga gtgttccgtt tcatttcaaa 180
ttaggtcatt ctgtcttgat tttgatatgg atgtttcaga agaccctagc agatgtccct 240
gtttaacttg aaaccataga tcagaaaact aagttcatat ttcaatttta cagagatttg 300
aatagaaaaa gtaatcaagt taaaacctgc aaagctaaaa ttgaactact tgggagctat 360
gatccacaaa aacaacttat tattgaagat ccctattatg taagtacagt tcacgtttta 420
gggctaatat gaagacccaa cacatttgta tcctgccata ttaaataaca gatgagattg 480
tgttaaggat gtttttgtta tgcaggtttt gccattttct tctttttcct gtccatttag 540
gggaatgact ctgactttga gacggtgtac cagcagtgtg tcaggtgctg cagagcgttc 600
ttggagaagg cccactgagg caggttcgtg ccctgctgcg gccagcctga ctagacccca 660
ccctgaggtc ctgcatttct cagtcggtgt gtaatcacgt tccagggccc aaagccagct 720
ctttgttcag ttgacttact gtttcttacc ttaaaaagta attgtagatg gaaatcagtt 780
gtgtttggca ggagaatcaa taaaaatgtt tgattcagac agcttatggg gtattttaag 840
cattcttaga ctagttgaac atctcacttt gccccagtta caaaaatagt agaacaagca 900
acataaaaca atgaaggaaa acctcacttg aaggcccagg tcaacatcta agcctgttga 960
gacttagata atcgagtcta cctcttcagt aggtttgtgt ggatggcctg gaggcaggtg 1020
cttgctcccc agtgctacct ctctcttccc tagggccttt tgtggattga cagtagtccc 1080
ctccgtagag ctcacagtct agattagaag tgttttaatt tctacacacc catagtgcac 1140
acttgtatat tgaaaagata gggaagagag aaacatttat ggaatcagtc gttggcacct 1200
tcaatacttc atgatttttg tcgagtttac ttcatgagga ggtcagccca ttggctccca 1260
tctgaaccac tttgcctctg aaacttaatt acatccagaa agaaggacac ttgtatgcta 1320
gtctatggtc agttgaggaa tatgactgtt tttatatgca catgtaaccc aaatgtccaa 1380
tataaattgg cttatttttt aaaataattt taaaagttgg gaaaagtgtt attatttggc 1440
atgcttaaat attgaataag tattcttcat cagcatttaa taaatgtata ggcagatgta 1500
aggtaatttc tgtgtatttt gagataatgt caaaatcatg aatatttcaa aataaactgg 1560
ggagttataa aaatacaact agagatataa atctgtgtct gtcctgttgt tgattgacag 1620
gtaggaagca tttgcaaaac attgtattgt cctttttaca tgatctgtga tgttaggcgt 1680
gcgtgccata ttttagtctt caggggataa ggaagtaaga catgatgaaa agtcagcacg 1740
actgctgccc catgtcatca tgtctagttt ctgaaggaag tttgaggttc tacagctgga 1800
actgtccttg cccagtacca tcctctggac atgttgcctc tacgctgtgc cctcctgtgc 1860
agtgatcgac tgcctggcca ggacatctgc actcctgacc tcagtatcat gagggtccca 1920
ggcagtgcat gggaggagac acgtttgatc ctttgactcc ctagtatctt tgaggataag 1980
cttgtttgcc cagatgaaaa gtgagtagtt tcccactgta cccatgtgga agtgcccagg 2040
gctgtcttca tag 2053

<210> 10
<211> 577
<212> DNA
<213> Homo sapiens

<400> 10
atggcggaac aggctaccaa gtccgtgctg tttgtgtgtc tgggtaacat ttgtcgatca 60
cccattgcag aagcagtttt caggaaactt gtaaccgatc aaaacatctc agagaattgg 120
agggtagaca gcgcggcaac ttccggtggg tcattgatag cggtgctgtt tctgactgga 180
acgtgggccg gtccccagac caagagctgt ggagctgcct aagaaatcat ggcattcaca 240
cagcccataa agcaagacag attaccaaag aagattttgc cacatttgat tatatactat 300
gtatggatga aagcaatctg agagatttga atagaaaaag taatcaagtt aaaacctgca 360
aagctaaaat tgaactactt gggagctatg atccacaaaa acaacttatt attgaagatc 420
cctattatgg gaatgactct gactttgaga cggtgtacca gcagtgtgtc aggtgctgca 480
gagcgttctt ggagaaggcc cactgaggca ggttcgtgcc ctgctgcggc cagcctgact 540
agaccccacc ctgaggtcct gcatttctca gtcggtg 577

<210> 11
<211> 1004
<212> DNA
<213> Mus musculus

<400> 11
cagcaatgcc ttaggtgctg caaggccttc ctggagaaga cttactagct ggtcctaagc 60
cccaccattg agcagctcac tcctcagtgc tgtgcccaag ggtggtggca gtccttagcc 120
ccatacccca cctctctttt cagctgactt actgtatatc tttaaaataa ttgtaggtgg 180
gaattaggca tatgttcaga aggataaaag catttgagtc agacagtttg aggtgtggct 240
aagcattctt agactaacta aacctctgac cttgcggtga ttacaaaaca gtggaacaag 300
caaatatgga acaaaaaaaa aaaaaaaaaa aaaaaaaaaa aaaaaaaaaa aaaaaaaaaa 360
aaaaaaaaaa aaaaacaaaa aaaagggggg ggggccccaa aacccccgag ggaggagccg 420
ccccagataa atctcacccc accccccatt ttttttgaaa aaacggggcc cccctaagag 480

gaggagcgag catacataaa accgcagacg gagcgagcgc gccggacaca acacaacacg 540
acgcgggagg cgacaaccaa cgtcagtcct agccggacta catgtggcag gagcacccca 600
catgcggggc cggggaaaca actggcggca acccctccca cagaaaataa gggcccgagc 660
agaatacaca cacgcacgcg gccgtcgcgg tcgtaccacc cccgccgcat ttacgggggc 720
gcgaaaacat ttggcccccc gacggagtaa cacgaacacc ttcgggccgc gccgtgaaca 780
aacaaccccc cacccacccc ccaccacgcg acccccccac caccagcgac gacgcgccaa 840
cgccacacac gaccagccag accgcacgca acgaccccac ccgccactac cacccaaccc 900
acgacgcgac ccccacccac cgcacgcccg acacgacacc acccagccac cccacccgcc 960
gcccaccccc cccacccacc cacacctcaa cagagcacca caac 1004

<210> 12
<211> 738
<212> DNA
<213> Mus musculus

<400> 12
aggtcagtta tagactaagc aatctccatc atttagcata tttctggtgc acaggaagca 60
acgtgaggat cactcttcat ctactcccat tacaaagtct accattataa ttttgatatg 120
cttgcattct ctattaattg aaataatata caactcatgt gtatatgtca ttacaaagag 180
ttttgcttca tggaagcatg aaagtatcac atatttatgg cagagataag aattagagaa 240
attgagggtc tgcaccgaaa catggcagag gttgggtcca agtcagtgct gttcgtgtgt 300
ctcggtaaca tttgccggtc acccattgca gaagcagtat tcaggaaact ggtaactgat 360
gaaaaggttt cagataattg ggccattgac agcagcgccg tttccgactg gaacgtgggc 420
cggcccccag acccaagagc tgtcagctgc ctaagaaatc atggcattag cacagcccat 480
aaggcaagac agattacaaa agaagacttt gccacattcg attatatact atgtatggat 540
gaaagcaatc tgagagatct caatagaaaa agtaatcaag ttaaaaactg caaagctaaa 600
attgagctac ttgggagcta tgatccacag aaacagctca tcattgaaga tccctattat 660
ggcaatgact ctgacttcga ggtggtgtac cagcaatgcc ttaggtgctg caaggccttc 720
ctggagaaga cttactag 738

<210> 13
<211> 19031
<212> DNA
<213> Mus musculus

<400> 13
   accgtggccc ggcctggggt cggtggtcca aaggggtaac ttagcttgga agggcgggta 60
   gctccgatcg gaggagccac cctaggaggc tctgcaagcc gctatccttg agttcattgg 120
   ctgccctggc acatcctaat cctccctgct caacaccgtg ccacccaagc tgctctgggg 180
   gtcgccccgc gttcaccccg ccctcactcc ttagacgtgc gacctgggca gagctcagag 240
   tgaacgctag cccgggctgc caacccgaac gcctaagccc cgccctctag gcgacttctg 300
   gccccgcctc tggccgcgta cgaacgtggg gcggcggact gcgcatgtcc acggtcagaa 360
   ccctggcagt gcgcatgcgc ctcgtcccga cgcggtttct gggcgccagg gtctgcaccg 420
   aaacatggca gaggttgggt ccaagtcagt gctgttcgtg tgtctcggta aggactcgcc 480
   gacttaaggg tctttttctt tgggaccctg aagtgctctc cctgttaggc ttccgaacaa 540
   gagcgctggg cggaggaagc agatcagaag gccttggctg ggcccgcgtc tgagcacata 600
   ctcgtttgtc tgcctttttc atatgcatcc cccatcatct ggtactcctc tgagcccgag 660
   atctgtgtaa tttttgtccc agattatgcc cttccaaagc ccatgtcacc cttccgagtg 720
   cccttcaacc acaaatcttt gtgcgagttc tctctctcaa gcccgtagac ccctctcact 780
   tgctcctcct cttttaagtc tgagagtcgc ccatcatctg ctttggcagt tacttccaaa 840
   tctctgtgct gaaaaatatc caaccaaaac aactttaggg agaacagatt tgttttgact 900
   cagtttcaga ggttcaggcc ccttttgaat ggttgtagat tctgggcttg tggtgaggca 960
   aggcatcatg gtccttcaag gtggccagag catgagtcag ggactatttg ctcctgtgga 1020
   cgggaatcac agaaggggag agtagagagc aagacaagcg ttagccccaa gagcacgcat 1080
   cgggatgtat tttcttctgc tcagccacac ttgtatcttt cattacctcc agtcatttcc 1140
   tcatattttg aatctactgg gttaaaccat tgactaggtc agtgccctta tgatcacatc 1200
   ctcgggggat aagccatcac cgacgtcctg agagttgtct ttttctaatc agttaaggtc 1260
   tcgatgacga ttaatcatca tccctgttat tccctcatgc tcttgtccgt cacctaactt 1320
   gcttttcttc ctttgaaccc tctcacctgc taactggccc aggtggcttc atatctgtag 1380
   tgtgactctc taagctggcc ccttctatta gaacagctgg agttcttgcc ggaaaccccc 1440
   acccccaccc ctgttcccca ttgtgccaac gtgagcctag gttacaggaa gaccattctg 1500
   tgacaggttt gtttgatgtc atccttgggt cacagttgct atggttctca gccagtctct 1560
   tttctttagg tctctctgaa accttggttt tcagtcccta ggacaattac tcatgccatg 1620
   tgaaaaacag tttgttttta aatgaataat cttttaagta ccttctaaca cagaatttca 1680
   accttgattc tcctttacat aatatgcaat tctttctttt ttcagagttc ttgcctcctt 1740
   gactccaccc tgaggggcaa gggttaaatt gtcaacagtc cagctatttg tcttttgtcc 1800
   tctgcttttc ctacagagtg acactcctct ttaggctaga agtctgccaa gactttctgt 1860
   ctacctcgct ttatctttcc cacccacctg tggggactgc ccttaagaaa ctgttgctgt 1920
   cctatgcctt gatggtatgg ttaactttac tgggaacctt aatgctgggt tttgcttgaa 1980
   aagccatctg ttttataaag tcaaaatgat gaaaaaaaag tcaaggattt tttataccca 2040
   gaccattggt accccccccc cgagatattt tattccataa gttgcatttt tatttgacag 2100
   cctgtggagc tcttaggaag agttatctgt cttgtgggca atagcttttt tccctttatt 2160
   attccacttt tcagtacaag gtactgtgtg tgatagttgc aagatatctg tactaggtaa 2220
   tttctatgaa gaacaaagta aaactctggc cttaattcac attgcttatt atagttatct 2280
   gtgctggcat attgtaccaa tatgactttt aaaggaagta catgcttgca tatgtttgac 2340
   gtcaatataa ttcagtattt tgtttttgcc actggaggtg catatagtgg tgaacaccac 2400
   agatgaagtc cagctgttct gaagccggta ctttgcagtc accatcagtc aaggaatgcc 2460
   agtggcttca tacagttggt gaggtgttca ctatgagcca aagcagagtg ggagcagggt 2520
   gtaggctgta gtcaggactc atctgaaggt gagcagagac atgaaggtgg caaggggcct 2580
   gtctctactc caaagacaga gcatgaggtt ttcaggtaaa agttgtaacc agagaagtca 2640
   tggttttgga gaccaggcag cctttctcag ccacgatttc tttgcctgtg ggtaccaaac 2700
   ttggatcatt ctaaatacga gaaatgagat ccttatgtac agcagatact ttagggcagg 2760
   gatctacatc ctctgtaaag agctggagtg aatccttaaa cttcatgtgc cgaaaactta 2820
   gtgtagccat agcttggttc tgttcagtag agctcgttcc tggataccag ccttgggact 2880
   tgatagagtt ttcacgtatt acaagatttt ctgcatttgc attatttttt aatgacctaa 2940
   aactaaaaat ttggttaacc ctttgggctg tatagaaatg tggtaggcca agaatggtta 3000
   tttctgggcc cctgtttgga gtctagatac cataggaagc tgtatgaaga cttgggtttg 3060
   ctctgagtga gataagccat gggaggtctg gagcagaaat ggggagggat ataatttgtt 3120
   ccacctgcat tgtggagatt aggctacagg aggccctgga agaagcaggg atagcttcag 3180
   aagttagatg tgttgtagtc agttgctagc ctcggaaaat ttgaggatgg tacagatgaa 3240
   ttctcccaag agcccctttg gagcacagtg aaagctcttg tgtttgaaat gcaaaagttt 3300
   tgttccttca gaacctttct tcaaactttt taagattgaa gttgttttag aaagatgagg 3360
   agaggagcgt tttgctctgt gaagctggat gggacccagc aaaggcaggt gtgatctcag 3420
   ccctagtcac agtggtcata agagaaataa ggccatgttt acatttccag gacttgtgga 3480
   agtcaaacca taaagtgttt ttcttatggc cagtgaactc actcttaatg tatatatgta 3540
   tgtttttatt tattcatttt tgatggggtc agaggtctaa cctagccctg tgttttttct 3600
   aataacatta ataacataaa ttggcaagct agaggcaagt tattagtatt gagagttccc 3660
   ttctattaat cttgtaggtt gtatgatttg tagtctctgt agagataggg tacttttaat 3720
   ctatacctac atacctttaa atacagctat ctgggggcag ggtaggagtg aatatgctca 3780
   aatgatgaaa tggcccacag ttttacaaag aaacaagact gtttcttcgt atttaatgat 3840
   aatttttctt atatagatgt cttgcctttt atttttacat gctaaaatat tacactttgg 3900
   taacttctaa acttccagaa taataatgtt tttcctaacc tgctcaccac tattggctgt 3960
   tttgcctgga tatgtggtga tgtgccagaa gccttttggt tagtgaaaca gtgctgatgg 4020
   tggtctttac aaaaacaaaa caaaacaaaa cagggctagg aagatcttaa tcagtgaagt 4080
   tcttaccttg taaacatgag ggacctgggt tttattccaa gaacctatgc ttaaataaaa 4140
   gaaaaaaaac aataacaaca acaaacaaaa attgtacatg gtaacatact tttatcatcc 4200
   ccacactagg tagatagaag ttgttggttt cctgagcctc ctggccagcc agcctatcct 4260
   gcctactaag gttactctta aactcgtaag aaaccctctc tcaaaaaaca aagtggacaa 4320
   cttctgagga atatcacctt tcttctctgt atacatctgc acgcttacac ccacacaaag 4380
   acatagaaaa accagacaga aattagaatg tggaaaggaa actttcacaa ccgggactat 4440
   catatggagg acatttaaaa tgagttcctg tcttagcttt aatggttaat tgatgaaact 4500
   gttattgcta ttaaacatac acacacacat agtctctctc tctctctctc tctctctctc 4560
   acacacacac acacagtctc tctctctctc tctctctctc tgtcacacac acacacacac 4620
   acacacacac aaacacacac acaaagaagc caagtacata gttttgtaga tatggaagga 4680
   tgtgcagtca tggcaggaac tggtccaggg accagagcat ccatgtttca tcacatctta 4740
   gaatgtctgg ttatgcatca gaagccagga aattttgacc tctcacatac agtttcttgc 4800
   ttgctttttt tttttttttt tcttttctta atgttaaggc ttaggtgata cacccaaata 4860
   cagtgttttg ttttgctgta taccttaaac aaagtctctc ttagcccatg ctttcccacc 4920
   tcttgcccct taagccaagc acagctcccc tgagaggaca tagaacctaa aactcctcta 4980
   gtttcctgtt agaaatctga aacctaaaaa tattatccct cttcttaaaa gcctgtgtgg 5040
   cagagtcgat cccacacaaa gaggaaagtg tgagacacag acctcacgag cctcctccag 5100
   gtctccctgc atttgtttca ttaggtcaag ctgctttgtt cagttgcagt tcttatcaga 5160
   cttagtcata aaactgtata tgctaacttt gatttgacag atggcagtag gatagttttt 5220
   tccaactcaa atcttctgtg cttttctcat ataaacctgc ttatcagagc tgtctggggg 5280
   tggtttaagc ataggactca caccttagac catttcccat tggctttttc atgtaacctt 5340
   ccagagtgtc actgtcttcc accctgagct tttctgacct tcccaccttg taagaggagg 5400
   ttgtatctta aacagctttc tatctgcagg cctaccatag tatttggctc aaaagtagta 5460
   ggcatacaag ataaatgtgt ttggtaagcc acattgccgg aaccttcagg ttctcccagg 5520
   atgcaccaag tacagacttg gcgtgggatt agggctccat gtcccaggcc ctgccttctg 5580
   tgtctagacg agtaccgcct tcaggtcctc ccacggtgct atttaaactt cttatcctgt 5640
   ggacatcagc tcaagcttag gttccctggc atctcactta gctttgcgga atgtttacgt 5700
   ttctctggtc tcataccatc acagcactag tttatcagca ttcattgttg ttgttgttat 5760
   tatcttgtgt cttgagtctt ttcagtgtat gagcctgtct ctttgtttat ctgcttgcag 5820
   agctaaggta tcatttgtct ttcttggtgt agcaaggaat ttggttcttg atcagatggg 5880
   ctcttagaaa gccatccttg atttccctgg tattttcttg gagtgatgga gtatctgtta 5940
   acacctggag gccccatagt ttgtgttttt gggggactca tttgggttct ctcacatttt 6000
   actaggagct ctgaatatca gtactctgct gagcttccta gcttggcagt aatactctca 6060
   gcatattgtc aacgacagga gggctaggtg tccccaggga caagaagact tcgtattcgg 6120
   ttcccccaga atacattttc ccttcttccg cctctctcca atctgtatct ttttatgagt 6180
   tgtgactata ctgcttatct gaattctatg gtcattttaa ggaatcacca aacccgacag 6240
   ttgtgggaag ggaaggcaga tttgtagcca actagtatgg gactcctgac ctatctgttg 6300
   atgtcagaag ttggggcagg tggggccgac aagaggaacc tccctgttcc tccagctggc 6360
   taactcgtgg ggaccatagg cttcttggga caggctttta cgtatgatta ctcaataaag 6420
   cttgtattga attgccttgt gttttgtttt gaagtattac tagtctttat tatgtatgta 6480
   tacttaaaca tagctatttc tgttctatta tactttgttt taaatgtagc tgattgcaac 6540
   ttactttgtt gatttcaaga tttggtcttg gttcattgct agtgaaaatt gatttgtgtt 6600
   ttacatgctt tcttgctgtt tagttacctc agccattctg tcaggaccat gaatctggtt 6660
   ctctcaggga attctgtact gtcttgccaa ccttagatgc aggcactggg catccacttt 6720
   gggagtagtc cactcaatct ttgggccaga aatctgtatc tgcccatctg ccacagctaa 6780
   taacctctat atgtcttctc cctctctttt taaggtaaca tttgccggtc acccattgca 6840
   gaagcagtat tcaggaaact ggtaactgat gaaaaggttt cagataatgt gagtaccatt 6900
   cagtagtgtg acgagtccag actgaatccc tttgggcagc aaggtgccag aacacttctt 6960
   ttctctcctg cagtggagga tagacagtgc ggctacatcc acctatgaag tggggaaccc 7020
   tcctgactat cgagggcaga actgcatgag aaaacatggc atccacatgc agcacattgc 7080
   acggcaggta ccgtactgga gcttgacata tgttttgtgt tacagtgggc cattgacagc 7140
   agcgccgttt ccgactggaa cgtgggccgg cccccagacc caagagctgt cagctgccta 7200
   agaaatcatg gcattagcac agcccataag gcaagacagg tagaaaagat ctcgtttaat 7260
   ttctactaca tagaatccca ttacttgaga gtctgaccac cctgtgtgtt gcagcatgta 7320
   tttggttggg ggcgactgac ttgctgcctg tgtagttgtg gaagatttgg atggccttgg 7380
   gtaacgtgtt aaagtacaga taaaagacat aagccatagt gatacaatgt ttgctgtgag 7440
   ccagacttgg ctgtggtctt cattttgtta ccatgtacag actctctggc tagacatttc 7500
   tgcttatgct actagtttgt tagtacacat gtctttttct actttaaagt accttaaaca 7560
   tacagctcta gtgccttgtt aacacttgtc tgtctggaag actggaatta aaaacaaacc 7620
   ctttagttac agacaaagat agttttgtat ccatattacc agaagatggc agcctgatgt 7680
   atttctttga gttccttatt gaaattaagt tgttctttta tgttgttttt aacaaaccgt 7740
   tctcaactgt tgtttttttt attatttttt ggctttttag ttccaaagca ctagtaaaga 7800
   atactttgta cgtcagtgtc acattttcct tatgttcttt ctctttacca attgccacac 7860
   tttatgcaaa taactatttt agtacagttc ttaacatgtt atttttctaa tctttcaact 7920
   taggttgtaa atggcatatg gttagggtgc tagctcttga actcaaagca atgtcttggg 7980
   ataggaactt ttagcatggc cgactggaga accgcccctg ctggtcaagt gtgctcacgt 8040
   gtccccgttt tcagtgtcat cagcttctcc atggactgag tccatcatca aggttgcttg 8100
   gccgcttctc ccctcttgtg cagtggtccc tcccaaagtt tcactgtgcg ttctaacatt 8160
   gtaggagtag aatcatcttg cttgtgttgt tgagtcaggg tcacactggc acttgaggca 8220
   gtcctcgtgg ttcagcctcc tcaggctgga attgcaggtc tgccccatgt ccctggcaca 8280
   gcagtggagt gcacgcacac gtagcatctt ttccatgtcg ctgctgaggg ttacagcagt 8340
   ttgccctggg cagggttaag tgctgtattg ttccttttgt acctctgctg gacgtgtggg 8400
   tggcttctgt tgtgagaatc tgtgggcagt ggcaccccag atggagccat ctaatgggtt 8460
   catagccagc atctgtacct atacctcacc aaatgcacag agcatgtctt cttgtctttt 8520
   aagaagtgta tgtgtctatt gcatatgcca gcaagatttt gctgacagaa ccctgacata 8580
   gctctctctt gtgaggctat gccagtgcct ggcaaatact aaagtggatg ctcacagtca 8640
   tttattagat ggaacacagg gcccccaatg aagaagctag agaaagtacc caaggagctg 8700
   aaggggtctg caaccctata ggaggaacaa caatatgaac taaccagtac ccccagagct 8760
   tgtgtctcta gctgcgtggc agaggatggc ctagtaggcc atcaatggga ggagaggcac 8820
   ctggtcttgt gaagattata tgccccaaat acaggggaat gccagggcca ggaagcggga 8880
   gtgggtgggt tggggagcag cgaagggggg ggggggaagg tatagggaat tttcgggata 8940
   gcacttaaaa tgtaaatgaa gaaaatatct aattaaaaaa aagaagtgtg tgtaaaaaat 9000
   tgatggtatc tctattttgt ttggaaaatt gatctgattg attgcttttg ctgatttttt 9060
   tttaattttt ttaccaattt attgacattg atatttatgt ttccatactt taaaaaattt 9120
   tttaaaattc attcatttat ttatttacac cccacatttt attcccctcc tagtccaccc 9180
   tttgactgtt ccacatctca tacctcctcc ccgcccttct gtctccatga ggatgtcccc 9240
   attcccaccc cctcacccca cctgacctct aaactcctgg gtcctccagt ctcttggggg 9300
   ttatttgcgt catccctgat tgaacctaga cctgacagtc ctctgctata tatgtgttgg 9360
   gggcctcaga tcagctggtg tatgctgcca ggttggtggt ctagtgtttg agagatctcc 9420
   agggtctagg ttaattgaga ctgctggtct tcctacaggg ttgccctcct cctcagcttc 9480
   tttcagcttt tccctaattc aactactggg gtcaacagct tctgtctcca ttggttgagt 9540
   gcaaatatct gcatctgact ctttcagctg cttgttgggt ctttctgagg gcagtcatgc 9600
   taggttcctt tttgtgaacg ctccatagcc tcagtaatag tgtcagacct tgggtcctcc 9660
   ccttgagctg gatcccactt tgggcctgtt gctggacctt cttttcctca ggctcctctc 9720
   cctttccatc tctgcagttc tttcagacga gattaattat gggtcagaga tgtgtctgtg 9780
   ggatggcaac tccatccctc acttgatgcc ctgttttttg ctggaggtgg gctctacaag 9840
   ttccctctcc cctctgtagg gcatttcatc taaggtccct ccctttgaat ccttagagtc 9900
   tcacctccca ggtctctggt acattctgga ggatcctgca acctcctgtc tcctgaggtc 9960
   caaggttgcc tgtttccatt ctttctgctg accctcaggg cttcagtcct tttcccctca 10020
   cccaacacca catcatgatt ccccgccccc catgtccact ttccctctca gtccctccct 10080
   ctgcccttgt gattgctttc ttctctctcc ctagtaggac tgaggtgtcc tcacttgggc 10140
   ccttcagctt gttgaccttt ttgagttctg aggactgtat cttggttatt ctgtactttt 10200
   ttttctcttc ttttttcttt tggctaatat ccacttatta gtgagtacgt accatgcatg 10260
   tccttttggg actgagttac atcattcagg ataatatttt ctagttccat ccatttgctt 10320
   gcaaaactca tgatgtcctc gttcttaata gctgagtagt tatcccactg ggtaaatgtg 10380
   taaatacgtt ttctgtatcc attcttctgt tgtgggatat ctgggtcgtt tccagcttct 10440
   ggctatcaca aataaggctg ctatgaacat agtggaacac atgcccctgt ggcatggtgg 10500
   ggcatctttt gggtatattc ctaagattgg tattgctggg tgttaaggta gatctattac 10560
   agtttcctga ggaacctcca aaagtttcca gaacttttga cttccagagt ggttgtacta 10620
   gtttgcaatc ccactagcaa tggaggagtg tgataatatc tctttttttt ctggctgttg 10680
   gttttctctc tggtctccag ttcatcagct ttgctgtggg agaccaacgc atgctttttg 10740
   aactgggact tgatgctttt taaccagttt tttggaaatt aggctttttc tttttgtttg 10800
   tttggttttt ttttggagag gcagctttca tatatattgg acataaaaat ctttctgttc 10860
   tttcattccg ccgagtttaa ttagcattat tttcatgccg cctatgatgt ggggctgttt 10920
   gctggacagt aggtaactta ccattggcag ggaagaaaaa ttatcccctc acataccctc 10980
   cctcattctc accagaattt tgagggctcc atgttttgta gagaacaaaa gctgttgtga 11040
   atttacaagt gaacatattc tttgtatttt aagtaaggtc tgctaatgcc tttctgggtc 11100
   atttctgagc ctgctctttc ctcttttttg gagaactggg aattacaccc taggtagagc 11160
   ctggtacgag cttatattgc atgtgatctg ctactgagct gtttccccag cagtatttag 11220
   tttttatttg gacagtttca ccctagcctg agctagcctg agctagcctg agctagcctg 11280
   agctagcctg agctagcctt gaacatgatt cctgcttctc tttcataagg agctgagatt 11340
   acaggccagt atcactaggc ctggcaagtt ggctttcttc ttgatttttc tgtgtggtgc 11400
   ccttttggtg ctgtgtaaat aatcatagtt cctttgaaag atcacatttt taccactgtt 11460
   gctgtgagga tgctggtaga tgttgtcaga tacagggtct cagtaggatt gcagactggc 11520
   agtacctctg agtacccttc tgctgctggg cctcaggatg gcatggactg ctcacctgca 11580
   gggcaggagc aggtcacagc atagaatgtg ggcttttctt tactgagcgc acataacttc 11640
   tcattggtct cttgagcttt ttgctctggc aggagagggc ttggtaggtc aggtggaaca 11700
   acctacactg actcttattt tctgggcctg tgctcctcca agcttcactg tcctgccagg 11760
   cctaacccca gtttcttctc cattgtccca ggcatccagg caggtagggc cctgcttttc 11820
   caccatggtc tttgcctggc tttctttgct cttgaggaag cttctaggcc cttgagatta 11880
   ttggcgtctc catcctggtg gccctttgtg caggactcag tgtgtgtgac tgctagtctc 11940
   tagtccttgc ctactcagat gtggtctagt atcaagcact agtactcaag agtgatccac 12000
   gggtcctttg gaagcattcc taaattgcac atagcttcac cttacatagt gtatcctaat 12060
   ataacccagt acagaggcaa gtaagaaaat ctgtctgaca atgccatttc ctgaagtgaa 12120
   aattaaaaat atctgtgggc tagagcgatg tcacagtggc taagagtact ttctcttcca 12180
   taggacaaga gttcacacat acaatacaca cacacacaca cacagagaga gagagagaga 12240
   gagagagaga gagagaatga gagaaaaata aaaacatctt aacaaaaagc ctatagctat 12300
   aatggatttt ttttttaaaa atcatttatt tttattttat gtgtatgagt attttacctg 12360
   caagcatgca taccacattt gtgcagtgct tgcttgcaga ggctaaaaga gggtgttgtg 12420
   agacatcgtg tgggtgctgg gaactgaacc tgggtccttt gcaatagcga cacatacctt 12480
   taaccactga gtcatttctc tagtcctggg ttttcatctt tttaaattaa gaaaaaattg 12540
   attttgtttt agttttagca gtgataaaac ctcctaaatg ggctgtttgg aaatggactt 12600
   gaatgcctcg ggggttaaag agctcttgcc tataatgact tgagagagaa ctctgggagg 12660
   gtccaggcag ggctcactca gatcagcagg taaggtccac cataggaact caacgggagg 12720
   tgattgttat ctgtggctgt aatcctgtca tgctgctcta gtgagccagg gttctcagaa 12780
   tcttggagaa gaatcttggt ttctgtgttg cttttctaat aataggatta gtttgtcctg 12840
   agtttcaact gtgcattttc catgacataa agttctagct gctcttcttt ctcagttatt 12900
   tatttattta tttaaggtat agaatagagt ttattagggg tatgggaaga ggagttaaga 12960
   gggtagtaga gaaaggggga gggggagaga agaaagaaga gaagtagagg aggagaggcc 13020
   agccatgagc atgtggagag aggagagaag gggatgggga cagcgggagg aagggaaaag 13080
   caagagctag agaaagcaaa atagcaagaa ctttctcagt tattttaaaa tccaaaaatc 13140
   ctgtgtggtt tgactctgta agtcattaag cagtacattt tttaggacaa attaataata 13200
   ataagccaaa attaatgcta agatattctt cataacttga aaaaataaca tattttatat 13260
   gatagtattg gtttaatgtc tttgaaataa attatgagtc ttgtgtcttg gggaattaaa 13320
   acagtacagt cctttgtgat tatttaaatg ctgtgctaat agtatatgga tgtgcatact 13380
   aaaagctaaa atgtctcatt tttaatataa ttaatttagt ataatataat ttaatataat 13440
   taatttaata taataatttt agtaacaagc aagtgaccat ttttattact gtttggttat 13500
   agtcattgtt cttttaaccc aggtgtttcc tgggtagtca aaatctagaa ggtaaatttc 13560
   ttcaactaca tttaagggag cttatgtctt aaactatgta gtgccagaaa caagtacatg 13620
   atgaagaccc ccaggatctt catgttagct ttagagggac ttgcactcag ggagtcttta 13680
   ccctctgaga atcagttaat ctccctcttc tgaggccctg tgtctcttct ctgcctcact 13740
   ctcctggaga gtatttactc ttcttcatag ggtggcctca cgagaacaaa gatctgttcc 13800
   ttaattaatc tgtattccca aggcctaagg cagtcatgga atgttgaata cagtggaggt 13860
   gactttatga tgccaattga gattacatct tactgaggga ccaaaactaa caacaagctt 13920
   ttttatgtcc atgttttata tttatgatta tttcaaagaa ccttttataa aattattttg 13980
   caataaagtt ttggacttga aatataccct aaattgtgta ttttgttaaa atttcagttg 14040
   ataagtgaac atagacttag gttaatgagt taacatataa ttacatgttt tttttttcaa 14100
   ctacagtttt gtatggatat aatcactgct tttgactttt ttaattgtag attacaaaag 14160
   aagactttgc cacattcgat tatatactat gtatggatga aagcaatctg aggtaatcat 14220
   atatatttgt ttttttgata aagggtttct ctgtctgccc tataactcac tctgtagacc 14280
   tggccttgaa ctcgggtcta cctatcactg cctcctgagt gctaggatta gaaatgacac 14340
   taccatgtcc agtgtatgca gtactcatac tttacacagt agctctgtcc tcctctcagt 14400
   tgtgttctag gcccagcaag tgcccggaat gactttctag tgtttttaca gttttttttt 14460
   ttcttcttcc attttttatt aggtatttag ctcatttaca tttccaatgc tataccaaaa 14520
   gtcccccata cccacccacc cccactcccc tacccaccca ctcccccttt ttggccctgg 14580
   cattcccctg tactggggca cataaagttt gcgtgtccaa tgggccgtct ttacagtttt 14640
   tatggacaag gttgataaca acctacagag ctattcagga gggcaggaag accaagtgtg 14700
   cttatctcag agctctgttt acaaaggtgt gtcttctaaa agacacagtg cagccagtag 14760
   atgaatggcc atggagacca gcgggtttgg ggctctaggt gtggaggtga tgcgttggtc 14820
   actgattcct ttactgctgt aacagatgag gagtctcttg tatcctattg ctatatagtt 14880
   gctgcactcc atagcaataa gtctgacaag acagccctca ctgagtttat ggtttttggg 14940
   ctaagcaaca ctgatgggat aggggttaag tagcaatgat tgagtctgta aaactgaggt 15000
   ggtctgcagg tccatacccc tcatcagtaa atttacttta ttaccaagga agaaattgag 15060
   gaactatgga cttaaatcac ttccctaggc tcatgggacg aatgggtggg ggtatagaag 15120
   agctgctgcc actgtggcct gcatgctctc cactaaactt acttgtccag ccacacttgg 15180
   tcctggcctc gctcagctgt cctgtactga gtccatcttc ctcctgtctt cacttgccct 15240
   gaacattgtc cagcctgcct tgggatgtgg tacacatggt cttttgagtt cttttctggc 15300
   atttttatgg atctgaagtg ttatcatttt tgtcatatca aaatacattt tttttttttt 15360
   tttggttttt tgagacaggg tttctctgtg tagccctggc tgtcctggaa ctgactttgt 15420
   agactatgct ggcctcgaac tcagaaatcc gcctgcctct gcctcccaag tgctgggatt 15480
   aaaggcgtgc gccacatttc acagaagaaa actttattat ttgagttaaa gatacacatt 15540
   ttgagtatca gaatcagtac actgtggcat acatacagct gcaggactcc cataggtctg 15600
   cagagtgttc tgtttcattt caaatcaggt tgcactgttg taatttgata ggatatttca 15660
   taaaccctga gcagatgtcc ctgtttaact taaaaccata gatcagaaat ctaagttcat 15720
   gtttcgattt tacagagatc tcaatagaaa aagtaatcaa gttaaaaact gcaaagctaa 15780
   aattgagcta cttgggagct atgatccaca gaaacagctc atcattgaag atccctatta 15840
   tgtaagtccc tgtcaggttt ggagcctctg ttaagatggg caggatgtat tcggtggtat 15900
   taaatgaaca ggatggattc tgttgtatta aatctgtgtt tgggtcttcg gaggacactg 15960
   tcatcataaa ccattgctgg tttttctttt tctgtccttg cagggcaatg actctgactt 16020
   cgaggtggtg taccagcaat gccttaggtg ctgcaaggcc ttcctggaga agacttacta 16080
   gctggtccta agccccacca ttgagcagct cactcatcag tgctgtgccc aagggtggtg 16140
   gcagtcctta gccccatacc ccacctctct tttcagctga cttactgtat atctttaaaa 16200
   taattgtagg tgggaattag gcatatgttc agaaggataa aagcatttga gtcagacagt 16260
   ttgaggtgtg gctaagcatt cttagactaa ctaaacctct gaccttgcgg tgattacaaa 16320
   acagtggaac aagcaaatat ggaacaaaag aaaacaaaaa caaaaacaaa aacccagaaa 16380
   gtaagagtga cctagaaggt ccatatcagc ctctgagctc ggcaagcctg ggtcgtctgg 16440
   tctaagtgga gtgtgtgcat gacccgcacc cagtgtgccg tttgcttgcc gtgtactctc 16500
   ttctctaggc tctcgttgtg acaatagctc cattcacggc agccttccag ttaacactgg 16560
   cagtttaagc tcagacacac tgagggtgtt gagggatttg agagaggaga cgctggatgt 16620
   gctgatgggc ctgggactcc agcaggccgt cctgggctgg acagtgacac cctgtctaaa 16680
   aggggaacaa aaataacaac ggatgggagc agcagatgga taaagttacc atcttcagtc 16740
   ttacttggtt tttgtctact ttcctgagct ttgttcttgt ttgagccact ttgctttaaa 16800
   aaaaaagtaa gtgtgcttaa atactggtat gtgtttgtaa gtgttctctc atgggcaatt 16860
   tacaaagtta taggcaagca agagtaattt ttgtgtattt tcagaaaaga gacctcaaat 16920
   ttatatgaat gtatgtcaga aaggaacatg aattcaactg ggaagttatc aaatacaact 16980
   gagatacaaa tccagtgtct gcctgcttct tactgacaag tgaacaagat acctaaatgt 17040
   tgcctccatg tgccttttta tttgcttgag tgtgaagttt gggctctcag cctctgtgtt 17100
   agaaagtaaa gtgatgagat ggatacacac aatgctgtgt tggatggcga tgggtgtcta 17160
   attgagagac tccaggcact atccttatcc ttgggccttc ttcatgtagc tggtgctccc 17220
   tacccgcttc ccagctgaaa aggtggtaac tgtctgtctt agcctgtctt agctcaggtc 17280
   actactggtg agctccagtc acgcagaact tgtagttaga agagccatcc tgacgtctga 17340
   aaggaaggaa gcccaggggc ctaggaatat gcagtctctt cttggccagg gctcctctct 17400
   cgaaggagga aagcttacac ctgactcttc cagaagaaag cagctatccc agcaccctct 17460
   cacagaaagg ccatgatgtc ccagcaaagg cagacacttc agctgccttt ttgggctcct 17520
   gtgggggttt ggtcagagtc ttcacaaaat taaaaccgag gaagggagag gacaggtagt 17580
   cagccacaag gaggaaatgg attaagtcaa agctctccac cctacttcag tgcttgcttc 17640
   agaatctcag atttctcttt caggtcataa gatcctattg ttttcttgaa attttacctg 17700
   tgtatcttac acgcagatcc ttggaatgtc aggttctgct ttgttctacg gctgtctcaa 17760
   acagcacagt gtgtttataa gggctcatga acagagaaat taacttttta cattcaatca 17820
   aattacattt gtttaaaata cagccattaa attttaaaac tgttctcagg gtgcttcttg 17880
   gtttgacatt ctcttacaat caggtagaga tggtagatgt ggagtattga tggctaaatg 17940
   agaccaagcg atcaggagtt tatgttattc agtacttagt cacatctaca cacacacaca 18000
   cgcacacgtg tatatatata gtgaacaatg tgtatgtatg tttatatata catatattct 18060
   gagccttgac attgttattt aagagtcatt ctgaagactg ttcattattc caaacacttc 18120
   atttatttcc ttgtatttat tcacagtatt tacatattgt acaatacctg gaatgtactt 18180
   ttacatttac agaggacaag tgggactggc cttggcatag ccacagatac atatgccact 18240
   gttttcaaca caactgaggt ttttttggtt ttcattaaaa tttttcatgc agttccaaac 18300
   tatactttgg gattttaatt gtagaataca aaatgtcaaa tcatactata tgctctatga 18360
   aaatacagtt taatgttctg cctatgtttc taaagaaata ttccttgtgg ttccacctaa 18420
   tcttaaaaag aaaatacctc attttacaga acaatacatc aaatgtggaa tgttgtctgt 18480
   ttttacaatc ataagagtgg caaatctcac tgacagatac actgattcac tgaatgcata 18540
   tttgtaaact gtcagcaaca tagaaaatgc aaaggaatta tggaagagtg caaaaataaa 18600
   tctctgtcca caggaagtgg gcatgaagat ggttctgttc tgttggtttc caaggcactt 18660
   ttcaaggttc ctggtgtgtc gctcctttgc agctgaaaac aattacattt ggtgtcatat 18720
   agctactcag gtcttattac taattctaga tgatgtggta caagtttagc aatataacaa 18780
   atttataaac aaggaacctg tatttctctt aacgttcaaa gtctaggtcc ttcccccact 18840
   cccacctgaa aacaacccag ttctatagtt accatgtgct cacggtgcct aagggtgaca 18900
   gccaccatgc acacctgtga ataaacactt gctcaagtca agtttggtgc ttggtcttag 18960
   gtgtgatggg gcactttggg aatcctgtca agagatgtag tttgtgcctc ctctggggaa 19020
   gagtttgctt g 19031

<210> 14
<211> 477
<212> DNA
<213> Mus musculus

<400> 14
atggcagagg ttgggtccaa gtcagtgctg ttcgtgtgtc ctggtaacat ttgccggtca 60
cccattgcag aagcagtatt caggaaactg gtaactgatg aaaaggtttc agataattgg 120
gccattgaca gcagcgccgt ttccgactgg aacgtgggcc ggcccccaga cccaagagct 180
gtcagctgcc taagaaatcg tggcattagc acagcccata aggcaagaca gattacaaaa 240
gaagactttg ccacattcga ttatatacta tgtatggatg aaagcaatct gagagatctc 300
aatagaaaaa gtaatcaagt taaaaactgc aaagctaaaa ttgagctact tgggagctat 360
gatccacaga aacagctcat cattgaagat ccctattatg gcaatgactc tgacttcgag 420
gtggtgtacc agcaatgcct taggtgctgc aaggccttcc tggagaagcc ttactag 477

<210> 15
<211> 477
<212> DNA
<213> Mus musculus

<400> 15
atggcagagg ttgggtccaa gtcagtgctg ttcgtgtgtc tcggtaacat ttgccggtca 60
cccattgcag aagcagtatt caggaaactg gtaactgatg aaaaggtttc agataattgg 120
aggatagaca gtgcggctac atccacctat gaagtgggga accctcctga ctatcgaggg 180
cagaactgca tgagaaaaca tggcatccac atgcagcaca ttgcacggca gattacaaaa 240
gaagactttg ccacattcga ttatatacta tgtatggatg aaagcaatct gagagatctc 300
aatagaaaaa gtaatcaagt taaaaactgc aaagctaaaa ttgagctact tgggagctat 360
gatccacaga aacagctcat cattgaagat ccctattatg gcaatgactc tgacttcgag 420
gtggtgtacc agcaatgcct taggtgctgc aaggccttcc tggagaagac ttactag 477

<210> 16
<211> 1436
<212> DNA
<213> Rattus norvegicus

<400> 16
gggggcggtt tctgggcgcc agggtctgca ccgaaacatg gcagaggttg ggtccaagtc 60
agtgctgttc gtgtgtctcg gtaacatttg ccggtcaccc attgcagaag cagtgttcag 120
aaaattggta actgatgaaa acgtttcaga taactggagg atagacagtg cggctacatc 180
cacctatgaa gtggggaacc cccctgacta tcgagggcag aactgcatga aaaaacatgg 240
catccacatg caacacattg cacggcagat tacaagagaa gactttgcca cattcgatta 300
tatactgtgt atggatgaaa gcaatctgag agatctgaat agaaaaagta atcaagttaa 360
aaactgcaaa gctaaaatcg agctacttgg gagctatgat ccacagaagc agctcattat 420
tgaagatccc tattatggca atgactctga cttcgaggtg gtgtaccagc aatgccttag 480
gtgctgcaag gccttcctgg agaagactca ctagctggtc ctaaccacca ccactgagca 540
acccactcct cagtgctgtg cccaagggtg gtggcagtcc ttagccccat accccacctc 600
tcttttcagc tgacttactg tatatcttta aaataattgt aggtggaaat caggcatttg 660
ttcagaagga taaaaacatt tgaggcagac atttgaggtg tggctcagta ttcttagact 720
aacaaaagct ctggcctcgc cataattaca aaatagtgga acgagcaact gtggaacaaa 780
agaaaaccca ataaagtaag aatgacccac aaggtccaga tcagcctttg agcccagcga 840
gcctgggtag tctggtctaa ctggagtgtg agcatggcca gcacccagtg tgctgtttgc 900
ttgccttaca ctctctattc tatattgtct cttattgtga caatatctcc atccatggca 960
gccttccatt taacactggg agagtttaaa cccagacaca ccgagggttc agatatttga 1020
gagaggagaa cctggatgtg atgatgggcc tggaactcca gcaggccatc ctgggctgta 1080
cagtgactcc ctgtctgaaa gataaaaatg ctaacagatg ggagcaacag gttggtgaag 1140
ttaacacctt taatctcact tggtttttgt gtagtttctt gagcttggtt cctatttgag 1200
ccactttgct tctttaaaga gaaagtaagt gtgcttaaat agtggtgtgc gtttgtaagt 1260
gttctctcat caacatttta caaagttaca ggcaagcatg agtaatttta gtgtattttc 1320
agaaaaggga cctcaaattt atgtggatat atgtcagaaa agattgtgaa ttaaactggg 1380
atgttatcaa atacaactaa aaatacaaaa aaaaaaaaaa aaaaaaaaaa aaaaaa 1436

<210> 17
<211> 16001
<212> DNA
<213> Rattus norvegicus

<220>
<221> misc_feature
<222> 10056, 10057, 10058, 10059, 10060, 10061, 10062, 10063, 10064,
10065, 10066, 10067, 10068, 10069, 10070, 10071, 10072,
10073, 10074, 10075, 10076, 10077, 10078, 10079, 10080,
10081, 10082, 10083, 10084, 10085, 10086, 10087, 10088
<223> n = A,T,C or G

<220>
<221> misc_feature
<222> 10089, 10090, 10091, 10092, 10093, 10094, 10095, 10096, 10097,
10098, 10099, 10100, 10101, 10102, 10103, 10104, 10105,
10106, 10107, 10108, 10109, 10110, 10111, 10112, 10113,
10114, 10115, 10116, 10117, 10118, 10119, 10120, 10121
<223> n = A,T,C or G

<220>
<221> misc_feature
<222> 10122, 10123, 10124, 10125, 10126, 10127, 10128, 10129, 10130,
10131, 10132, 10133, 10134, 10135, 10136, 10137, 10138,
10139, 10140, 10141, 10142, 10143, 10144, 10145, 10146,
10147, 10148, 10149, 10150, 10151, 10152, 10153, 10154
<223> n = A,T,C or G

<220>
<221> misc_feature
<222> 10155, 10156, 10157, 10158, 10159, 10160, 10161, 10162, 10163,
10164, 10165, 10166, 10167, 10168, 10169, 10170, 10171,
10172, 10173, 10174, 10175, 10176, 10177, 10178, 10179,
10180, 10181, 10182, 10183, 10184, 10185, 10186, 10187
<223> n = A,T,C or G

<220>
<221> misc_feature

<222> 10188, 10189, 10190, 10191, 10192, 10193, 10194, 10195, 10196,
10197, 10198, 10199, 10200, 10201, 10202, 10203, 10204,
10205, 10206, 10207, 10208, 10209, 10210, 10211, 10212,
10213, 10214, 10215, 10216, 10217, 10218, 10219, 10220
<223> n = A,T,C or G

<220>
<221> misc_feature
<222> 10221, 10222, 10223, 10224, 10225, 10226, 10227, 10228, 10229,
10230, 10231, 10232, 10233, 10234, 10235, 10236, 10237,
10238, 10239, 10240, 10241, 10242, 10243, 10244, 10245,
10246, 10247, 10248, 10249, 10250, 10251, 10252, 10253
<223> n = A,T,C or G

<400> 17
   aaacatttat gatcaggagt gtagaaacta atcttgagtt ctgaggattt gcaaaagagg 60
   atccgagcag tatgatcttg gtagatgcgc ccagacaaac ccaggaactt cactatttct 120
   ctcctctttc acaacgccct tcgcaagagg aagagttgcc ggggtaccga cacgccccgg 180
   ctgctcagac ccttactcac tggtgccagc ggctccgctc cgtgcccaag ctcctcactc 240
   cagtccagga agaggacggt gttctccttg gtccgcctcc ccgaccggac ccgcccagcg 300
   ggcgcacacc tgacccaggt gagggaatag gtgtgtgccc tgtttttgcc aatgagatca 360
   ctggtcctat acacttgctc agaacatagc tcaactgcgt ctacccgcct cattcctttg 420
   gaaccttgca aatcaccccc tcttcttaat cgggaaaacc caccgctagt accgtggcgc 480
   ggcgtagggt cggtggtcca actgggtaac ttagcttgga agggagggta gctccgatcg 540
   gaggagcccc cctaggaggg tctgcaagcc gctctctttg agctcattgg cagccctggc 600
   acgttctgct taacactgcg ccacccaccc tgctctgggg gtcgccccgc gttcaccctg 660
   ccctcgcact ttagcggtgg gatcaaagca gagcccagag tgaacgctag acccggttgc 720
   caaccacaac tccctagccc agccctttag gcagcttctg gccccgcctt gggcggagtg 780
   cgaacgtggg gcggcggact gcgcatgtcc gcgggcagaa cctggcagtg cgcatgcgcc 840
   acgtctctac gcggtttctg ggcgccaggg tctgcaccga aacatggcag aggttgggtc 900
   caagtcagtg ctgttcgtgt gtctcggtaa ggactcgccg acttaagggt ctttttcttt 960
   gggaccccga agtgctctcc gtgttaagct tctaaacaag agccctgggc ggagggaaga 1020
   agagcagaag gccttggctg ggcccgcgcc taaaaacatt catttgcctg gctttttcat 1080
   acgcatcccc cattttctgg tactcccctg aacctaaggt ctgtgtagtt ttgtcccaga 1140
   ttatgccccg gtctccttcc tgcccttccg aagcacgtgt caccctccca agtgcccttc 1200
   caccacaaat ctttgtccta ctcccgtctc tctcccaagc ctgtaggccc ttttcacttg 1260
   cttctcctaa gtctgagagt cgcccatcac ctgctgtggt agttactttc atatctctgt 1320
   gctaacactg tctaaccaga acaactttag ggagaacaga tttgttttga ttcagtttcg 1380
   gagggttcag tctacttttg aatgattgca gactctgggc ttgtggtgag gcatcacggt 1440
   catcagggcg tccagagcat gagtcaggct acctgctcct tgtggaccgg aatcacggaa 1500
   ggggagacat ggaggtagag aacaagacaa gcggttagcc ccaagagcaa gcatcgtgat 1560
   gtatttattt tcttctgtgt agccccactt gtgttttttc atcacctccc agtagtttgg 1620
   ttatatttct aatctaatgg agtaaaccat tgactaggtc agagtcctta cgatcaaatc 1680
   ctctcaggct atgcctttac cgacgtcctg aaagttgttt tttctaacca gtcaaggtca 1740
   caatgaagat aaatcatcat ccctgtcatt tcctcaagct cttgtccctc accaacgtgc 1800
   ttttcttatt ctgaaccgtc tcacctgcta actggcaggt ggcttcatat ttgtagtatg 1860
   actctctaag ctggcccctt ctattagaac agctggagtt cttgccttcc ccagaacccc 1920
   accccacacc tctctcattc tgccaaggtg agcctcagtt acaggaagac cattctgtga 1980
   cagcagtttc ccaaacaggt ttgttcgatg tcatctttgg gtcccagttg ctgtggttct 2040
   cagccagtct ctattttttt tttaagttgt tctttttttg ttctttcatt tttttattgg 2100
   atatttatct acatttcaaa tgctatcccc ttctcagttt ttcccaccag aaacccccta 2160
   tccaatccca ccatcccctg cctctataag ggtgctcccc cacccataca cccacccact 2220
   tcttcctgcc ttctgccctg gaattttcct acacgggggc atccagcctt cacagaacca 2280
   agtgcctccc ctcccattga tgcctgacaa ggccatcctc ttctacatat gcagctggag 2340
   ctataggtcc caccatgtgt actctttggt tggtggttta gttcctggga gctcggggcc 2400
   ctgggtgggg ggttgggggg tggggtgtga tatctggttg gttgatattg ttgttgttcc 2460
   tataggggtg caaaccccct cagccacctc ggtcctttct ctaactcctc caccactggg 2520
   gaccctgttc tcagacgaat ggttggctgc gagcattctt tagttctctc tgagaccttg 2580
   gttgttttca atccctggga caattactca gttttgctac atgaaaaata gtttttcttt 2640
   ttataagatg aatagtcttt caaatacttc taacacagaa ttttcattct tagaatttca 2700
   acattgattc tcctttaagt aatatgtaat tcttttcttt tttcagagtt cttgcctcat 2760
   tgcctcggtt cagcaatcat tggtcttttg gcctttgctt ttcctacaga gtagcgctcc 2820
   tcctttttcg gctagaagtc tgccaagact ttctgtccac ctcactttat ctttcccacc 2880
   cacctgtggg gaccgccctc aagaaactgt tgctgtcctt tgccttgatg tgtggttaat 2940
   tttactgggt accatgctgg gtttttcttg aaaagccata tattttataa agacaaaata 3000
   aagaaaaaaa agtcaggggt tttcatcccc agactgttag tcacttcctt ttatccccct 3060
   atagagatat tttatcgcat aaattgcatt tttaattgac agcctgttga gttcttaaga 3120
   agagctatct ttctagtggg caattgcttt tttttccttt tatttttcca ctttcagtac 3180
   aatgtagtgt ggtgtttgca aggtatctgt actaggtaat ttctatgaag agaaaagtaa 3240
   aactgtggcc ttatttcatg ttatctgtgc tggcatatta cacccacatg actttttttt 3300
   ttattttttt atttattttt ttcagagcgg gggaccgaac ccagggcctc gctctaccac 3360
   tgagctaaat ccccaacccc cccacatgac ttttaaaggg aagtacatgc ttgcttatgt 3420
   ttgacttcaa tataattcag cattttgtct ttgccactgg agatgcatgt ggtggtgaac 3480
   accgcaggta aagtccaact gttgtgaagc tggttctctg ccggtctcca tcagtcaagg 3540
   aacgccagtg gcctcgtcca gtgggtgaag tgtgcagcca aaacagagtg ggagctgggt 3600
   gtaatttgta gtcaggactc tcttgaaggt gagcagagac aaaggtggca agagacctgt 3660
   ctgtctgggc tcagaaggca gagcatgagg atttcaggta aaagttgtaa ccggagaaat 3720
   cactattttg gagatcaggc agctttctga accaagattt ctttgcctgt gggtaccaaa 3780
   cttgtttcat tctaaatacc tggggagaaa ttgggtcctt atatacagca gatactgtag 3840
   ggcagggatc tgcatcctct gtaaagagct gggatgaatc cttaaacttc atgtggtgat 3900
   aacttagtgt agccatagct tggctctgtt cagtagaact cgttcctgga tgccagcatt 3960
   ggaacttgat gcagttttca catgtcacaa ggttttctgt atttgcatta tttttaatga 4020
   gctaaaacta aaaatttggt taacccttta gacagcatgt ggatgtggta gtccaggagt 4080
   ggtcatttct ggaccctatt tggagtctaa atatcatagg aggctgtatg aggattgggt 4140
   ttactctgcg tgagataagc cttgggaggt ccagaacaga aatgagaggg acataatttg 4200
   ttccatctgc agtgtggaga ttaggctgca ggaggccctg ggagaagcag ggatatcatt 4260
   tgaagtaaga tgtgttatag tcagttgcta gtccaggaaa ctttgaagat ggtatagatg 4320
   cgttctacca agagaccctt tggaggacag taaaagctgt tgtgtttgaa atgcacaagt 4380
   tttattcctt cagaaccttt tttcgaacta gttaagattg aagttgtttt agcaagatga 4440
   agaaaggagc attttgctct gtgtagctag aagggactcc acaaaggcag gtgtgatctc 4500
   agctttagtc atagtggttg taagaaaacg aggctatgtt taagtttcag ggacttgtgg 4560
   aaatgaaaac ctgaggctct gtgcttttct catggccagt gtactcactc ttattaatgt 4620
   gtgtatgctt ttattttcat ttttgatggg ctcagaggtc taacctcagc tgtctatctt 4680
   taataacatt aattttaata acaaattggt gagctagagg cagagttccc ttctattgat 4740
   cttgtgcgtt gtatgatttg tagtctccag agataggata ctttgaatct atacttacac 4800
   accttacttt acatatggct acctgggggt ggggcatggg tgaatatgct aaaacgatga 4860
   tatgtcctga gtttttacat tctaaaagaa acaagactgt tacttcatag ttaatgataa 4920
   gttttcttat acagacgtct tacattttac ttttacacac taagatatta catttcggtt 4980
   acttctaaac ttctagaata atggtgcttg tcctaaactg cccaccactg ttacctcttt 5040
   tgtctggata tgtggtgatg tgcaaaaact ataaacagag tgaatcagtg ccgagggtgg 5100
   cccttatgaa acacatcagg actagggaga tcttcattgg taaagttctt gccttgtaga 5160
   catcagggac ctgggtttta ctccaagaac ctatgttaaa ataaaagaaa aacaaaacaa 5220
   cactaacaac aacaagaatt gtacatggta acatactttt ataatccctg cactaggtag 5280
   atagaggttg ttgggtccct gagcctcctg gccagccagc ctgtcctact aaggttactt 5340
   aaactagtaa gaaactctct caaaaataaa ggtgggcaac ctctgagaaa taccgcccaa 5400
   tattgtcccc tattctccat ctgcacctgc acgcttgcat tcttgcaccc acatgtactc 5460
   atagaaaaac caaacaaaaa ttagaatgta gaaagggaaa ccttcataac caggactagc 5520
   atatggagga catttaaaat gaattcctgt cttagcttta atgattaatt aataagacta 5580
   ttattgctct taatctttct ttctctctct ctctctctct ctctctctca cacacacaca 5640
   cacacacaca cacacacaca cacacacaca cagtggaggg ggcgggagag gaggggaaga 5700
   agccaagtac ttagttttgt agatatggaa ggatgcacag tcatggcagg aactggtcca 5760
   gggaccagag cattcatatt tcatcacatc ttagaatgtc tggttgtgca tcagaagcca 5820
   ggagcttttg acctcccgca tagtttcttg cttgtttaat tttttcttaa tgtttgggct 5880
   tatgtgataa cacccaaata tggtgttttg tctttccgtg cacctcagag tctctcttat 5940
   cccatgcctt cccacctctt gctccttagg ccaagcataa ctcctctaag ataggacata 6000
   gaacctataa tttctctagt ttcctgtaag aaacctgaag cctagtaata ttacccccct 6060
   tctcccgtaa aagcctgtgt gccagagtga gtcccataca aggaggaaag cgtgaggcac 6120
   agaccaacct ccccaggctc ttccaggtct ccctgcattg gtttcattag gacgggctgc 6180
   ttggttcagt tagagttctt atcagatgta gttgtaaaac tatatatacc gacgttcatt 6240
   taacaaatgg gaagtgggac agtttttcca gctctttgcc tcttcaatct gaaaattttc 6300
   atgttagata aactaattaa gcctcgtgtg tttttctcgt ataaacctgc tcatcagagt 6360
   tgtctggggt gatttaaatg tgggtgaggc taggaatcag accttagact gtttcccatt 6420
   ggctttgtca tgtatcctcc cagagtctca ctgtcttcta ccctgagctc ttctgaactc 6480
   cccacctcgt aagaggaagt tgtatcttaa atagctttct atctgtaggc cgaccataat 6540
   acttggctca aaagtaatag ccatacaaaa taaatgtgtt tggtagccac atggccttca 6600
   ggttgttctc cctgggttca ctaagtaccg acttggcgtg ggattagggc tcctcgtccc 6660
   aggccctgcc ttctgtgtct agactagtac aggttctgcc ttcaagtcct cccacagtgc 6720
   tgcttaactt cttatcttag gaacatcagc tcaagcttag gctccctggc gtcccattta 6780
   gctttgtgga atctttgttt ctctggtctc ataccattat ggcacttgtt tatcagcatt 6840
   ctcctccttc tcctcatcta cctcctcccc cctcctcttc atcctcctct tcctccatca 6900
   tcatcatcat catcatcatc atcatcatca tcatcatcat cttcttgtgt cttgtgtctt 6960
   gtgttttctc agtgtatgag tctgcctctt tgtttatctg cttgcagagc taaggtgtca 7020
   tttgtccttg ttggtatagt aagaatttgg ttcttgtcca agtgggctct taggaagtca 7080
   tccttgattt ccctggtatt ttgatggagt atctgttaac acctggaggc cccatagttt 7140
   gtgtttttgg ggactcattt ggatcctgtg tcattatccc ctcacattta actaggaact 7200
   ctggatatca gtactatgct gagcttcctg gttaggcagt agagctctca gcatattgtc 7260
   agtcaatgac aggagggtga ggtgtcccca aggacaagaa gacttattat ttggttcccc 7320
   agaataaatt ttctcttctt ccatctccaa tctatatctt ttaatgagtt acaatatatt 7380
   acttatctga attccatggt cattctaagg aatcaccaag cctgacggag agggaaggca 7440
   gatgtgtagc ctctagtatg gagctcctga cctatctgtt gacataagaa gttggggtat 7500
   gtgaggcagt caagagaaac ctctccctgc tcctctagct ggctaactct tggggaccac 7560
   agtcttcttg ggacaaggct ttacatatga ttactcagta aagcctgtgt ggaattgcgt 7620
   tgtgttttgc tttgaagtat tactagtctt tattatgtat ggtatactta aaaatgccta 7680
   tgtctgttct attatacttt attttaaatg aagctgactg caactcattt cattgatttc 7740
   aagacttgct cctggttcat tgctagtgaa tattgatttg ggttttatct gcttccttga 7800
   gctttagtta cctcagccat tctgtgagga ccatgaatct agtttgatta actgttctgt 7860
   cagggaattc tgtactgcct tgccaatctt aggtgcgggc actgggcatc cgttttggga 7920
   gtggtccacc cagtcttcgg gccaggaatt tgtacctacc tttctgccac agctaacgac 7980
   ctctgtatgt cttcttcctc tcttttttaa ggtaacattt gccggtcacc cattgcagaa 8040
   gcagtgttca gaaaattggt aactgatgaa aacgtttcag ataacgtgag taccattcag 8100
   tagggtgaag agtccaggct gaatacctgt gggcatcaag gtgccacaac acttcttttc 8160
   tctcccgcag tggaggatag acagtgcggc tacatccacc tatgaagtgg ggaacccccc 8220
   tgactatcga gggcagaact gcatgaaaaa acatggcatc cacatgcaac acattgcacg 8280
   gcaggtaccg tatggagctt gacatatgtt ttgtgttaca gtgggccatt gacagcagcg 8340
   ccgtttccga ctggaacgtg ggccggcccc cagacccaag agctgtcagc tgcctaagaa 8400
   atcatggcat tagcacagcc cataaggcaa gacaggtaga aaagatctcg tttaatttct 8460
   actacctaga attccataac ttgagagtct gaccaccctg tgttttgcag aatgtattag 8520
   gtaggggtcg actaacttgc tgcctgtgta gttgtggaag atttggacct tgggtaactt 8580
   gttaaagtac agatcccagg tataagccgc agtgaggcag tgtggctggg agccaggcct 8640
   ggcggtggtc ttcatatgtt accatgtgca gacagacttc tgctcatgct cctagcttgt 8700
   tagtacatag aaatgtcttt tctactttga agtatcttaa acatacagag gggctttggt 8760
   gttagcacag agggggtggt gttagcactt gtctcacctg aagactatgg aattaaaaca 8820
   aactcttctg ttacagatga aggtagtttt atattcatat tatcagaaaa tggcagctgt 8880
   tgtatttctt tgagttcctt attgaaatta agttgttcat tgaccatttt taattgctga 8940
   tctcattagg cagaagatga aagagtgtat atcattcgtt cacttaggtc gtttcaggct 9000
   tttgcataca tggtccatct gccactgtct ggagaacatg gacttacttt gagtgaccaa 9060
   tcaaaactgt aagacatgtg gttcttttaa tttctgctca tgtatagcaa aagcaaatac 9120
   aatattttac attcctaggc cttgaaaacc aaagccagaa acttcctcca gagcagtttt 9180
   aatgactagc tttaggagta gcatttccgt agaatctttg tttcttttat gttgttgttt 9240
   ttcttttatt gtctttgact tttcagttcc agagcactag taaagaattc ttcgtgtgtc 9300
   agtatcacat tttccttatg tttgtaggtt cgttctcttt actgattgcc acacttcatg 9360
   taaataacta tttagtacag ttcttaacat ttttctagtc tgtcaactta ggtttagtaa 9420
   acggcatgtg gttagggtac cagttctatt gaacaaaaaa aacagtgtct tgggatagga 9480
   gcttttagca aggcagacta gaacagtcct tgctgctcaa gtgtccccat tttcaatgtc 9540
   ctttcatcag cttctccatg gactgagtct aacaccaagg ttatttggcc gtttctccct 9600
   tctcatgcag aggtcacccc caaagtttcg ctcatcattt taacattata ggagtaggat 9660
   cattggctct gttttgctga gtccgggtcc tactctggct cttgaggcag tcctcatggg 9720
   tcagcctccc cagtgctggg agtccgggtg tgcaccatgt ccctggcata gcggaggggg 9780
   ccgtgcacat gcagcttctt ttccatgttg ctgccgagtg tcagagaagt tgcgttggtc 9840
   agggttaagt gctgtgttgt tctactgttc tacctctgct ggatggtggg cagcttctgt 9900
   gtgagagtat gtaggcagtg gcagcacaga aggcgccatc taatgggttc atagccagtg 9960
   tctgtatcta tgcctcacca aatgcacggg gcatggcttc ttgaatttag gaagtgcgta 10020
   tgtaaaaaat tgatagtatc gctacttagt ttggannnnn nnnnnnnnnn nnnnnnnnnn 10080
   nnnnnnnnnn nnnnnnnnnn nnnnnnnnnn nnnnnnnnnn nnnnnnnnnn nnnnnnnnnn 10140
   nnnnnnnnnn nnnnnnnnnn nnnnnnnnnn nnnnnnnnnn nnnnnnnnnn nnnnnnnnnn 10200
   nnnnnnnnnn nnnnnnnnnn nnnnnnnnnn nnnnnnnnnn nnnnnnnnnn nnnnnnnnnn 10260
   nnnnnnnnnn nnnnnnnnnn nnnnnnnnnn nnnnnnnnnn nnnnnnnnnn nnnnnnnnnn 10320
   nnnnnnnnnn nnnnnnnnnn nnnnnnnnnn nnnnnnnnnn nnnnnnnnnn nnnnnnnnnn 10380
   nnnnnnnnnn nnnnnnnnnn nnnnnnnnnn nnnnnnnnnn nnnnnnnnnn nnnnnnnnnn 10440
   nnnnnnnnnn nnnccgagct accccagcat tatttagttt tttattttga cagtttcact 10500
   ctgtagccta ggctagccgt gaacatgatt ttcctgcttc tctttcacag taggagctga 10560
   aattccaggc ctgtacacct aggcttggcc agttggcttt cttcttgctt ttgctgtgtg 10620
   gtgccctttt ggtgctgtgt aaataatcac agtgcctgaa agatcatagt tttaccgctg 10680
   ttgctgtgag gatgctggta gatgttgtca gatacagcat ctcagtagga ttgcaggctt 10740
   gtagtgcctc tcagtaccct ctgctgctgg gcctgaagat ggcatggatt gcttacttgc 10800
   agggtgggaa cagctcagag tgtggtttct ctttactgag tgcacataac tctccttggt 10860
   ctcgtgagct ttctgctcgg gcaggagagg acttggtcag gtcaagtggg acagcctaca 10920
   ctaactcttt tcctcggcct gtgctcttcc aaggttcact gtcccgccat gcctgggccc 10980
   agcttcttct ctatggtccc aggcatcagg gcaggtaggg cctgcttttc caccatggcc 11040
   tttgcctggc tttctttgct cttgaggagg cttctaggcc cttgacacca ttggcatctc 11100
   tgtcctggtg gctctttgtg caggactcgg tgtgtgttac agctaacctc taaccattgc 11160
   gtactgggtt gtggtttagt atcaagcact aatacttaag agtgatccac aggtcctttg 11220
   gaagcattcc tttttttttt tttttttttt tttttctttt tttcggggct ggggaccgaa 11280
   cccaggacct tgcgcttcct aggcaaacgc tctaccgctg agccaaatcc ccaacccttg 11340
   gaagcattcc taaattgcac ataacttcac cttacatagt gtatcctaat ataacccagt 11400
   gcagaggcaa gtaaaaatat ctgtctgaca atgccgtttc ctgacctgaa aattaaaaac 11460
   acctgtgggc taaagaaata tcttggtgtc ttaagactac ttcctcctct tccataggac 11520
   cagagtgtgt acacacaaac acacaaacac acacacacac acacacacag acacagagag 11580
   agagagagag agagagagag agagagagag agagagagag agagatagac aaaaaacatc 11640
   ttaacagaaa acccatagct ataatggatt tttaagtttg aaagcgttcg tttgtttttg 11700
   ttttatgtgt gtgagtatct tacctgagag tatgcatagc acatttgtgc agggcttgca 11760
   gaggctagaa gagggtgtca ccgtctctga aataggagtt acaggtgtga ggcattatgt 11820
   gggtgctggg aactgaacct gggtcctttg caatagtaac atacctttaa ccaccgagcc 11880
   atttttctag tcctggattt gcattttttc aaattaagaa aaaattgatt ttgttttagt 11940
   tttagcattg ataaaacctc ctaaatgggc tgtttggaaa tggacttgac taccttgggg 12000
   gttaaagagc tcttgggtat aatgacttgg gagagaactc tgggaggatc caagcagtac 12060
   tcactgggat gagcagttaa ggtccacaca tagggactca actggaggtg atgtgttatc 12120
   tgtggctata ttcctgtttg tcacactgct ctagtgggcc aggtattctt aggagcgtgt 12180
   tcatagtctt ggagaagagt cttggtttct gtgtaggctc taataatggg attaatttgt 12240
   cctgtgcttc actgtgcatt ttccatgacg taagttctag ctgctcttct ttctcagttc 12300
   gttttaaaat acaaaaatcc tgtgtggctt gactctataa atcactaagc agtacatttt 12360
   ttaggataaa ttatttataa taagtccaaa actaatgcta agatattctt cataacttga 12420
   aaaaataaca ttttatgata gtatttgttt aatgtctttg aaataagtta taagtgttgg 12480
   gtattgggga tatcaaaaca atacagtcct ttgattattt aagtgctatg ctattagtat 12540
   atggacgtgc atactaaaag ctaaaattcc tcatttttaa tagagtgaca agcaagtgac 12600
   catttttatt actgtttggt tatagtcatt gttcttttaa cccaggtgtt tcctgggtag 12660
   tcaaaatcta gaagataaac ttcttcagct gcactgaagg gagcttacgt gtcttaaatt 12720
   atgtggtgcc agaatagagc acattgatga ggacctgcgg gatcctccct gttagcttca 12780
   gacagacttg cactcgtgga tccttgtccc tggtgggcct tctacctttt gagaatccat 12840
   tagttcccct gttctgaggc tctgtgtctt ctctctttct tgctttcctt gagagtattt 12900
   attctccttc acagggatgg cctcacaaga acaaagaact gttccttaat taatctggag 12960
   tggttcccaa ggcctaaggc atggaatgat ggatgtggtg ggggtgactg catggagtca 13020
   tttcagatta catcttacag agggaccaaa actaacaaca acaaacttta cgtccatgtt 13080
   ttatatttct gattatttca aagaaccttt tacaaaacta ttttgtaata aagttttgga 13140
   aatacaatga ttttgtttta acttcaaatg tactctaaat tgtgtgtttt tttttttttt 13200
   aattttagtg gataagtgaa catagaattt agttaatgag ttaacatgta attacatggt 13260
   tttttcagct ataatgttgt atggatgtaa tcactgtttt ttgatttctt aattgtagat 13320
   tacaagagaa gactttgcca cattcgatta tatactgtgt atggatgaaa gcaatctgag 13380
   gtaatcatat attttgtttt ttttgataaa gggtttccct gtgtagccct gtctgccttg 13440
   taactcactt tgtagatttg accttgaact caggtatact tatccctgct tcctgagtgt 13500
   taggttaaaa ggtaagcact gccatgtcca gttcatgcag taatcatact ttacacagta 13560
   tttctttcct cctctcagtt gtgttatagg cccaacaagt tgtccagaat gacttgctag 13620
   tgtcttacag tttttgtgta caaggttgat tacaatctac agagtgttca ggagggcagg 13680
   aggatcaagg tatggttatc tcagatcttt acagaagtgt gtcttctgag agaccgtcca 13740
   gccactacag gaatggacag ggagagcagc tggttcgggg ctccaggtgt ggaggtgatt 13800
   gatgccttgg tcactggttc ttttgctcct gtcacagatg aggaggcgct tgtatcctat 13860
   ttccgtatag ttgctgctct gtagagtaag tgacaagaca gaccgcactg ggtttatggt 13920
   ttttggctaa ccagcacgga tgggctcagg gttaagatgt ggcctgcagg tccacatccc 13980
   tcatcaataa atttgtttta ttatcaaaga agaaatcaag gaaccatgaa cttaaattac 14040
   ttccctaggt tcataggatg gatgggtggg ctatagggag cataaaacca tagctgctgc 14100
   cactgtggcc tgctttccac taaacttgcc caaccacact tggtcctgac ctcactcagc 14160
   tctcctacac tgagtccatc ttccctgcct tccttgccct gaacgttgtc caaactgttt 14220
   ctttgtcctg ggatatggta tacatggtct tttgagttct tttctgccat tttatgtatc 14280
   tgaaatgtta tcatttttat tgtatcaaaa tacatttcac agaggaaaac tttattatat 14340
   aaattaaaga tacacatttt gggtgtcaga atcagtgcac tgtggcatac agctgcagaa 14400
   gtccataggt ctgcagagta ttctgtttca ttccaaatga ggttgtactg ttttgatagg 14460
   atatttcata aaccctgagc agatgtccct gtttaactta aaaccataga tcagaaatct 14520
   aagttcatgt ttcgattttc cagagatctg aatagaaaaa gtaatcaagt taaaaactgc 14580
   aaagctaaaa tcgagctact tgggagctat gatccacaga agcagctcat tattgaagat 14640
   ccctattatg taagtacatt tcaggtttgc agcctctgtt aagatgagca ggatgtattt 14700
   attctgtgga attaaatgag caggatgtat tctgttgtat taaatgacag gtttgggtct 14760
   tctgaggaca ctgtcatcat aaaccattgc tagtttttct ttttgtgtcc ttgcagggca 14820
   atgactctga cttcgaggtg gtgtaccagc aatgccttag gtgctgcaag gccttcctgg 14880
   agaagactca ctagctggtc ctaaccacca ccactgagca acccactcct cagtgctgtg 14940
   cccaagggtg gtggcagtcc ttagccccat accccacctc tcttttcagc tgacttactg 15000
   tatatcttta aaataattgt aggtggaaat caggcatttg ttcagaagga taaaaacatt 15060
   tgaggcagac atttgaggtg tggctcagta ttcttagact aacaaaagct ctggcctcgc 15120
   cataattaca aaatagtgga acgagcaact gtggaacaaa agaaaaccca ataaagtaag 15180
   aatgacccac aaggtccaga tcagcctttg agcccagcga gcctgggtag tctggtctaa 15240
   ctggagtgtg agcatggcca gcacccagtg tgctgtttgc ttgccttaca ctctctattc 15300
   tatattgtct cttattgtga caatatctcc atccatggca gccttccatt taacactggg 15360
   agagtttaaa cccagacaca ccgagggttc agatatttga gagaggagaa cctggatgtg 15420
   atgatgggcc tggaactccn nnnnnnnnnn nnnnnnnnnn nnnnnnnnnn nnnnnnnnnn 15480
   nnnnnnnnng gcgccagggt ctgcaccgaa acatagcaga ggttgggtcc aagtcagtgc 15540
   tgttcgtgtg tctcggtaac atttgccggt cacccattgc agaagcagtg ttcagaaaat 15600
   tggtaactga tgaaaacgtt tcagataact gggccattga cagcagcgcc gtttccgact 15660
   ggaacgtggg ccggccccca gactcaagag ctgtcagctg cctaagaaat catggcatta 15720
   gcacagccca taaggcaaga cagattacaa gagaagactt tgccacattc gattatatac 15780
   tgtgtatgga tgaaagcaat ctgagagatc tgaatagaaa aagtaatcaa gttaaaaact 15840
   gcaaagctaa aatcgagcta cttgggagct atgatccaca gaagcagctc attattgaag 15900
   atccctatta tggcaatgac tctgacttcg aggtggtgta ccagcaatgc cttaggtgct 15960
   gcaaggcctt cctggagaag actcactagc tggtcctaac c 16001

<210> 18
<211> 459
<212> DNA
<213> Rattus norvegicus

<400> 18
atggcagcct tccatttaac actgggagag tttaaaccca gacacaccga ggaagcagtg 60
ttcagaaaat tggtaactga tgaaaacgtt tcagataact gggccattga cagcagcgcc 120
gtttccgact ggaacgtggg ccggccccca gactcaagag ctgtcagctg cctaagaaat 180
catggcatta gcacagccca taaggcaaga cagattacaa gagaagactt tgccacattc 240
gattatatac tgtgtatgga tgaaagcaat ctgagagatc tgaatagaaa aagtaatcaa 300
gttaaaaact gcaaagctaa aatcgagcta cttgggagct atgatccaca gaagcagctc 360
attattgaag atccctatta tggcaatgac tctgacttcg aggtggtgta ccagcaatgc 420
cttaggtgct gcaaggcctt cctggagaag actcactag 459

<210> 19
<211> 20
<212> DNA
<213> Artificial Sequence

<220>
<223> Oligomeric Compound

<400> 19
cgtgtgtctg tgctagtccc 20

<210> 20
<211> 20
<212> DNA
<213> Artificial Sequence

<220>
<223> Oligomeric Compound

<400> 20
ggcaacgtga acaggtccaa 20

<210> 21
<211> 18
<212> DNA
<213> Artificial Sequence

<220>
<223> Oligomeric Compound

<400> 21
gcccattgct ggacatgc                                                    18

<210> 22
<211> 20
<212> DNA
<213> Artificial Sequence

<220>
<223> Oligomeric Compound

<400> 22
agcccattgc tggacatgca                                                  20

<210> 23
<211> 20
<212> DNA
<213> Artificial Sequence

<220>
<223> Oligomeric Compound

<400> 23
ttgtcccagt cccaggcctc                                                  20

<210> 24
<211> 20
<212> DNA
<213> Artificial Sequence

<220>
<223> Oligomeric Compound

<400> 24
ctttccgttg gacccctggg                                                  20

<210> 25
<211> 20
<212> DNA
<213> Artificial Sequence

<220>
<223> Oligomeric Compound

<400> 25
gtgcgcgcga gcccgaaatc                                                  20

<210> 26
<211> 20
<212> DNA
<213> Artificial Sequence

<220>
<223> Oligomeric Compound

<400> 26
atccaagtgc tactgtagta                                                  20

<210> 27
<211> 20
<212> DNA
<213> Artificial Sequence

<220>
<223> Oligomeric Compound

<220>
<221> misc_feature
<222> 1, 2, 3, 4, 5, 6, 7, 8, 9, 10, 11, 12, 13, 14, 15, 16, 17,
18, 19, 20
<223> n = A,T,C or G

<400> 27
nnnnnnnnnn nnnnnnnnnn 20

<210> 28
<211> 20
<212> DNA
<213> Artificial Sequence

<220>
<223> Oligomeric Compound

<400> 28
gccctccatg ctggcacagg 20

<210> 29
<211> 20
<212> DNA
<213> Artificial Sequence

<220>
<223> Oligomeric Compound

<400> 29
agcaaaagat caatccgtta 20

<210> 30
<211> 20
<212> DNA
<213> Artificial Sequence

<220>
<223> Oligomeric Compound

<400> 30
tacagaaggc tgggccttga 20

<210> 31
<211> 20
<212> DNA
<213> Artificial Sequence

<220>
<223> Oligomeric Compound

<400> 31
atgcattctg cccccaagga 20

<210> 32
<211> 21
<212> DNA
<213> Artificial Sequence

<220>
<223> Oligomeric Compound

<400> 32
caacggattt ggtcgtattg g                                                21

<210> 33
<211> 26
<212> DNA
<213> Artificial Sequence

<220>
<223> Oligomeric Compound

<400> 33
ggcaacaata tccactttac cagagt                                           26

<210> 34
<211> 21
<212> DNA
<213> Artificial Sequence

<220>
<223> Oligomeric Compound

<400> 34
cgcctggtca ccagggctgc t                                                21

<210> 35
<211> 19
<212> DNA
<213> Artificial Sequence

<220>
<223> Oligomeric Compound

<400> 35
gaaggtgaag gtcggagtc                                                   19

<210> 36
<211> 20
<212> DNA
<213> Artificial Sequence

<220>
<223> Oligomeric Compound

<400> 36
gaagatggtg atgggatttc                                                  20

<210> 37
<211> 20
<212> DNA
<213> Artificial Sequence

<220>
<223> Oligomeric Compound

<400> 37
caagcttccc gttctcagcc                                                  20

<210> 38
<211> 20
<212> DNA
<213> Artificial Sequence

<220>
<223> Oligomeric Compound

<400> 38
tggaatcata ttggaacatg                                                    20

<210> 39
<211> 20
<212> DNA
<213> Artificial Sequence

<220>
<223> Oligomeric Compound

<400> 39
ggcaaattca acggcacagt                                                    20

<210> 40
<211> 20
<212> DNA
<213> Artificial Sequence

<220>
<223> Oligomeric Compound

<400> 40
gggtctcgct cctggaagat                                                    20

<210> 41
<211> 27
<212> DNA
<213> Artificial Sequence

<220>
<223> Oligomeric Compound

<400> 41
aaggccgaga atgggaagct tgtcatc                                            27

<210> 42
<211> 23
<212> DNA
<213> Artificial Sequence

<220>
<223> Oligomeric Compound

<400> 42
tgttctagag acagccgcat ctt                                                23

<210> 43
<211> 21
<212> DNA
<213> Artificial Sequence

<220>
<223> Oligomeric Compound

<400> 43
caccgacctt caccatcttg t                                                  21

<210> 44
<211> 24
<212> DNA
<213> Artificial Sequence

<220>

<223> Oligomeric Compound

<400> 44
ttgtgcagtg ccagcctcgt ctca                                              24

<210> 45
<211> 18
<212> DNA
<213> Artificial Sequence

<220>
<223> Primer


<400> 45
tgcggccagc ctgactag                                                     18

<210> 46
<211> 23
<212> DNA
<213> Artificial Sequence

<220>
<223> Primer

<400> 46
cgtgattaca caccgactga gaa                                               23

<210> 47
<211> 20
<212> DNA
<213> Artificial Sequence

<220>
<223> Probe

<400> 47
ccccaccctg aggtcctgca                                                   20

<210> 48
<211> 21
<212> DNA
<213> Artificial Sequence

<220>
<223> Primer

<400> 48
gggtccaagt cagtgctgtt c                                                 21

<210> 49
<211> 23
<212> DNA
<213> Artificial Sequence

<220>
<223> Primer

<400> 49
cctgaatact gcttctgcaa tgg                                               23

<210> 50
<211> 26
<212> DNA
<213> Artificial Sequence

<220>
<223> Probe

<400> 50
tgtgtctcgg taacatttgc cggtca                                            26

<210> 51
<211> 23
<212> DNA
<213> Artificial Sequence

<220>
<223> Primer

<400> 51
ggcctcgcca taattacaaa ata                                               23

<210> 52
<211> 24
<212> DNA
<213> Artificial Sequence

<220>
<223> Primer

<400> 52
ggaccttgtg ggtcattctt actt                                              24

<210> 53
<211> 30
<212> DNA
<213> Artificial Sequence

<220>
<223> Probe

<400> 53
aacgagcaac tgtggaacaa aagaaaaccc                                        30

<210> 54
<211> 20
<212> DNA
<213> Artificial Sequence

<220>
<223> Oligomeric Compound

<400> 54
ctttggtaat ctgccgggca                                                   20

<210> 55
<211> 20
<212> DNA
<213> Artificial Sequence

<220>
<223> Oligomeric Compound

<400> 55
actgcttctg caatgggtga                                                   20

<210> 56
<211> 20
<212> DNA

<213> Artificial Sequence

<220>
<223> Oligomeric Compound

<400> 56
caatgaccca attctctgag                                                    20

<210> 57
<211> 20
<212> DNA
<213> Artificial Sequence

<220>
<223> Oligomeric Compound

<400> 57
tccatacata gtatataatc                                                    20

<210> 58
<211> 20
<212> DNA
<213> Artificial Sequence

<220>
<223> Oligomeric Compound

<400> 58
tttcatccat acatagtata                                                    20

<210> 59
<211> 20
<212> DNA
<213> Artificial Sequence

<220>
<223> Oligomeric Compound

<400> 59
attgctttca tccatacata                                                    20

<210> 60
<211> 20
<212> DNA
<213> Artificial Sequence

<220>
<223> Oligomeric Compound

<400> 60
agattgcttt catccataca                                                    20

<210> 61
<211> 20
<212> DNA
<213> Artificial Sequence

<220>
<223> Oligomeric Compound

<400> 61
ctcagattgc tttcatccat                                                    20

<210> 62
<211> 20

<212> DNA
<213> Artificial Sequence

<220>
<223> Oligomeric Compound

<400> 62
ctctcagatt gctttcatcc                                                    20

<210> 63
<211> 20
<212> DNA
<213> Artificial Sequence

<220>
<223> Oligomeric Compound

<400> 63
agcctgttcc gccatcttcc                                                    20

<210> 64
<211> 20
<212> DNA
<213> Artificial Sequence

<220>
<223> Oligomeric Compound

<400> 64
gacttggtag cctgttccgc                                                    20

<210> 65
<211> 20
<212> DNA
<213> Artificial Sequence

<220>
<223> Oligomeric Compound

<400> 65
gcacggactt ggtagcctgt                                                    20

<210> 66
<211> 20
<212> DNA
<213> Artificial Sequence

<220>
<223> Oligomeric Compound

<400> 66
acacaaacag cacggacttg                                                    20

<210> 67
<211> 20
<212> DNA
<213> Artificial Sequence

<220>
<223> Oligomeric Compound

<400> 67
caaatgttac ccagacacac                                                    20

<210> 68

<211> 20
<212> DNA
<213> Artificial Sequence

<220>
<223> Oligomeric Compound

<400> 68
caatgggtga tcgacaaatg                                                    20

<210> 69
<211> 20
<212> DNA
<213> Artificial Sequence

<220>
<223> Oligomeric Compound

<400> 69
tgaaaactgc ttctgcaatg                                                    20

<210> 70
<211> 20
<212> DNA
<213> Artificial Sequence

<220>
<223> Oligomeric Compound

<400> 70
tcggttacaa gtttcctgaa                                                    20

<210> 71
<211> 20
<212> DNA
<213> Artificial Sequence

<220>
<223> Oligomeric Compound

<400> 71
ccaattctct gagatgtttt                                                    20

<210> 72
<211> 20
<212> DNA
<213> Artificial Sequence

<220>
<223> Oligomeric Compound

<400> 72
gttgccgcgc tgtctaccct                                                    20

<210> 73
<211> 20
<212> DNA
<213> Artificial Sequence

<220>
<223> Oligomeric Compound

<400> 73
atgacccacc ggaagttgcc                                                    20

<210> 74
<211> 20
<212> DNA
<213> Artificial Sequence

<220>
<223> Oligomeric Compound

<400> 74
tccagtcaga aacagcaccg                                                    20

<210> 75
<211> 20
<212> DNA
<213> Artificial Sequence

<220>
<223> Oligomeric Compound

<400> 75
taggcagctc acagctcttg                                                    20

<210> 76
<211> 20
<212> DNA
<213> Artificial Sequence

<220>
<223> Oligomeric Compound

<400> 76
ccatgatttc ttaggcagct                                                    20

<210> 77
<211> 20
<212> DNA
<213> Artificial Sequence

<220>
<223> Oligomeric Compound

<400> 77
tttatgggct gtgtgaatgc                                                    20

<210> 78
<211> 20
<212> DNA
<213> Artificial Sequence

<220>
<223> Oligomeric Compound

<400> 78
cttgctttat gggctgtgtg                                                    20

<210> 79
<211> 20
<212> DNA
<213> Artificial Sequence

<220>
<223> Oligomeric Compound

<400> 79
aatcaaatgt ggcaaaatct                                                    20

<210> 80
<211> 20
<212> DNA
<213> Artificial Sequence

<220>
<223> Oligomeric Compound

<400> 80
attcaaatct ctcagattgc                                                    20

<210> 81
<211> 20
<212> DNA
<213> Artificial Sequence

<220>
<223> Oligomeric Compound

<400> 81
tgcaggtttt aacttgatta                                                    20

<210> 82
<211> 20
<212> DNA
<213> Artificial Sequence

<220>
<223> Oligomeric Compound

<400> 82
ggatcatagc tcccaagtag                                                    20

<210> 83
<211> 20
<212> DNA
<213> Artificial Sequence

<220>
<223> Oligomeric Compound

<400> 83
gatcttcaat aataagttgt                                                    20

<210> 84
<211> 20
<212> DNA
<213> Artificial Sequence

<220>
<223> Oligomeric Compound

<400> 84
ccataatagg gatcttcaat                                                    20

<210> 85
<211> 20
<212> DNA
<213> Artificial Sequence

<220>
<223> Oligomeric Compound

<400> 85

agtcattccc ataataggga                                                    20

<210> 86
<211> 20
<212> DNA
<213> Artificial Sequence

<220>
<223> Oligomeric Compound

<400> 86
gtcagagtca ttcccataat                                                    20

<210> 87
<211> 20
<212> DNA
<213> Artificial Sequence

<220>
<223> Oligomeric Compound

<400> 87
cgtctcaaag tcagagtcat                                                    20

<210> 88
<211> 20
<212> DNA
<213> Artificial Sequence

<220>
<223> Oligomeric Compound

<400> 88
cacactgctg gtacaccgtc                                                    20

<210> 89
<211> 20
<212> DNA
<213> Artificial Sequence

<220>
<223> Oligomeric Compound

<400> 89
gtgggccttc tccaagaacg                                                    20

<210> 90
<211> 20
<212> DNA
<213> Artificial Sequence

<220>
<223> Oligomeric Compound

<400> 90
gaacctgcct cagtgggcct                                                    20

<210> 91
<211> 20
<212> DNA
<213> Artificial Sequence

<220>
<223> Oligomeric Compound

<400> 91
cagcagggca cgaacctgcc                                                    20

<210> 92
<211> 20
<212> DNA
<213> Artificial Sequence

<220>
<223> Oligomeric Compound

<400> 92
gggtctagtc aggctggccg                                                    20

<210> 93
<211> 20
<212> DNA
<213> Artificial Sequence

<220>
<223> Oligomeric Compound

<400> 93
tgagaaatgc aggacctcag                                                    20

<210> 94
<211> 20
<212> DNA
<213> Artificial Sequence

<220>
<223> Oligomeric Compound

<400> 94
acacaccgac tgagaaatgc                                                    20

<210> 95
<211> 20
<212> DNA
<213> Artificial Sequence

<220>
<223> Oligomeric Compound

<400> 95
gggccctgga acgtgattac                                                    20

<210> 96
<211> 20
<212> DNA
<213> Artificial Sequence

<220>
<223> Oligomeric Compound

<400> 96
aacaaagagc tgggctttgg                                                    20

<210> 97
<211> 20
<212> DNA
<213> Artificial Sequence

<220>
<223> Oligomeric Compound

<400> 97
ctttttaagg taagaaacag                                                    20

<210> 98
<211> 20
<212> DNA
<213> Artificial Sequence

<220>
<223> Oligomeric Compound

<400> 98
tgaatcaaag atttttattg                                                    20

<210> 99
<211> 20
<212> DNA
<213> Artificial Sequence

<220>
<223> Oligomeric Compound

<400> 99
aaatacccca taagctgtct                                                    20

<210> 100
<211> 20
<212> DNA
<213> Artificial Sequence

<220>
<223> Oligomeric Compound

<400> 100
gcttaaaata ccccataagc                                                    20

<210> 101
<211> 20
<212> DNA
<213> Artificial Sequence

<220>
<223> Oligomeric Compound

<400> 101
aagaatgctt aaaatacccc                                                    20

<210> 102
<211> 20
<212> DNA
<213> Artificial Sequence

<220>
<223> Oligomeric Compound

<400> 102
caagtgaggt tttccttcat                                                    20

<210> 103
<211> 20
<212> DNA
<213> Artificial Sequence

<220>

<223> Oligomeric Compound

<400> 103
tagatgttga cctgggcctt                                                    20

<210> 104
<211> 20
<212> DNA
<213> Artificial Sequence

<220>
<223> Oligomeric Compound

<400> 104
gtctcaacag gcttagatgt                                                    20

<210> 105
<211> 20
<212> DNA
<213> Artificial Sequence

<220>
<223> Oligomeric Compound

<400> 105
gactcgatta tctaagtctc                                                    20

<210> 106
<211> 20
<212> DNA
<213> Artificial Sequence

<220>
<223> Oligomeric Compound

<400> 106
aacctactga agaggtagac                                                    20

<210> 107
<211> 20
<212> DNA
<213> Artificial Sequence

<220>
<223> Oligomeric Compound

<400> 107
aagagagagg tagcactggg                                                    20

<210> 108
<211> 20
<212> DNA
<213> Artificial Sequence

<220>
<223> Oligomeric Compound

<400> 108
ctaatctaga ctgtgagctc                                                    20

<210> 109
<211> 20
<212> DNA
<213> Artificial Sequence

<220>
<223> Oligomeric Compound

<400> 109
aaacacttct aatctagact                                                    20

<210> 110
<211> 20
<212> DNA
<213> Artificial Sequence

<220>
<223> Oligomeric Compound

<400> 110
ctatgggtgt gtagaaatta                                                    20

<210> 111
<211> 20
<212> DNA
<213> Artificial Sequence

<220>
<223> Oligomeric Compound

<400> 111
agtgtgcact atgggtgtgt                                                    20

<210> 112
<211> 20
<212> DNA
<213> Artificial Sequence

<220>
<223> Oligomeric Compound

<400> 112
aaatgtttct ctcttcccta                                                    20

<210> 113
<211> 20
<212> DNA
<213> Artificial Sequence

<220>
<223> Oligomeric Compound

<400> 113
gccaacgact gattccataa                                                    20

<210> 114
<211> 20
<212> DNA
<213> Artificial Sequence

<220>
<223> Oligomeric Compound

<400> 114
tgaaggtgcc aacgactgat                                                    20

<210> 115
<211> 20
<212> DNA
<213> Artificial Sequence

<220>
<223> Oligomeric Compound

<400> 115
gaagtattga aggtgccaac                                                    20

<210> 116
<211> 20
<212> DNA
<213> Artificial Sequence

<220>
<223> Oligomeric Compound

<400> 116
gccaatgggc tgacctcctc                                                    20

<210> 117
<211> 20
<212> DNA
<213> Artificial Sequence

<220>
<223> Oligomeric Compound

<400> 117
tggttcagat gggagccaat                                                    20

<210> 118
<211> 20
<212> DNA
<213> Artificial Sequence

<220>
<223> Oligomeric Compound

<400> 118
aagtgtcctt ctttctggat                                                    20

<210> 119
<211> 20
<212> DNA
<213> Artificial Sequence

<220>
<223> Oligomeric Compound

<400> 119
catattcctc aactgaccat                                                    20

<210> 120
<211> 20
<212> DNA
<213> Artificial Sequence

<220>
<223> Oligomeric Compound

<400> 120
tttgggttac atgtgcatat                                                    20

<210> 121
<211> 20
<212> DNA

<213> Artificial Sequence

<220>
<223> Oligomeric Compound

<400> 121
tgatgaagaa tacttattca                                                    20

<210> 122
<211> 20
<212> DNA
<213> Artificial Sequence

<220>
<223> Oligomeric Compound

<400> 122
acatctgcct atacatttat                                                    20

<210> 123
<211> 20
<212> DNA
<213> Artificial Sequence

<220>
<223> Oligomeric Compound

<400> 123
tccccagttt attttgaaat                                                    20

<210> 124
<211> 20
<212> DNA
<213> Artificial Sequence

<220>
<223> Oligomeric Compound

<400> 124
ggaagcaact catgatctgg                                                    20

<210> 125
<211> 20
<212> DNA
<213> Artificial Sequence

<220>
<223> Oligomeric Compound

<400> 125
aatgcatgcc atatagtaga                                                    20

<210> 126
<211> 20
<212> DNA
<213> Artificial Sequence

<220>
<223> Oligomeric Compound

<400> 126
ctaatgatcc aggagtgaat                                                    20

<210> 127
<211> 20

<212> DNA
<213> Artificial Sequence

<220>
<223> Oligomeric Compound

<400> 127
tggtacttac attctctgag                                                    20

<210> 128
<211> 20
<212> DNA
<213> Artificial Sequence

<220>
<223> Oligomeric Compound

<400> 128
ctttggtaat ctaaaattga                                                    20

<210> 129
<211> 20
<212> DNA
<213> Artificial Sequence

<220>
<223> Oligomeric Compound

<400> 129
acaggattac ctcagattgc                                                    20

<210> 130
<211> 20
<212> DNA
<213> Artificial Sequence

<220>
<223> Oligomeric Compound

<400> 130
gttgaacaga aatattcttc                                                    20

<210> 131
<211> 20
<212> DNA
<213> Artificial Sequence

<220>
<223> Oligomeric Compound

<400> 131
attcaaatct ctgtaaaatt                                                    20

<210> 132
<211> 20
<212> DNA
<213> Artificial Sequence

<220>
<223> Oligomeric Compound

<400> 132
ctgtctgact caaatgcttt                                                    20

<210> 133

<211> 20
<212> DNA
<213> Artificial Sequence

<220>
<223> Oligomeric Compound

<400> 133
ggtcagaggt ttagttagtc                                                    20

<210> 134
<211> 20
<212> DNA
<213> Artificial Sequence

<220>
<223> Oligomeric Compound

<400> 134
tccgtctgcg gttttatgta                                                    20

<210> 135
<211> 20
<212> DNA
<213> Artificial Sequence

<220>
<223> Oligomeric Compound

<400> 135
gtggtgctct gttgaggtgt                                                    20

<210> 136
<211> 20
<212> DNA
<213> Artificial Sequence

<220>
<223> Oligomeric Compound

<400> 136
ttgcttagtc tataactgac                                                    20

<210> 137
<211> 20
<212> DNA
<213> Artificial Sequence

<220>
<223> Oligomeric Compound

<400> 137
atgatggaga ttgcttagtc                                                    20

<210> 138
<211> 20
<212> DNA
<213> Artificial Sequence

<220>
<223> Oligomeric Compound

<400> 138
aaatatgcta aatgatggag                                                    20

<210> 139
<211> 20
<212> DNA
<213> Artificial Sequence

<220>
<223> Oligomeric Compound

<400> 139
gcttcctgtg caccagaaat                                                    20

<210> 140
<211> 20
<212> DNA
<213> Artificial Sequence

<220>
<223> Oligomeric Compound

<400> 140
ctcacgttgc ttcctgtgca                                                    20

<210> 141
<211> 20
<212> DNA
<213> Artificial Sequence

<220>
<223> Oligomeric Compound

<400> 141
actttgtaat gggagtagat                                                    20

<210> 142
<211> 20
<212> DNA
<213> Artificial Sequence

<220>
<223> Oligomeric Compound

<400> 142
tataatggta gactttgtaa                                                    20

<210> 143
<211> 20
<212> DNA
<213> Artificial Sequence

<220>
<223> Oligomeric Compound

<400> 143
tagagaatgc aagcatatca                                                    20

<210> 144
<211> 20
<212> DNA
<213> Artificial Sequence

<220>
<223> Oligomeric Compound

<400> 144
ttcaattaat agagaatgca                                                    20

<210> 145
<211> 20
<212> DNA
<213> Artificial Sequence

<220>
<223> Oligomeric Compound

<400> 145
acatatacac atgagttgta                                                    20

<210> 146
<211> 20
<212> DNA
<213> Artificial Sequence

<220>
<223> Oligomeric Compound

<400> 146
ctttgtaatg acatatacac                                                    20

<210> 147
<211> 20
<212> DNA
<213> Artificial Sequence

<220>
<223> Oligomeric Compound

<400> 147
aaactctttg taatgacata                                                    20

<210> 148
<211> 20
<212> DNA
<213> Artificial Sequence

<220>
<223> Oligomeric Compound

<400> 148
tgcttccatg aagcaaaact                                                    20

<210> 149
<211> 20
<212> DNA
<213> Artificial Sequence

<220>
<223> Oligomeric Compound

<400> 149
gatactttca tgcttccatg                                                    20

<210> 150
<211> 20
<212> DNA
<213> Artificial Sequence

<220>
<223> Oligomeric Compound

<400> 150

aatatgtgat actttcatgc                                                    20

<210> 151
<211> 20
<212> DNA
<213> Artificial Sequence

<220>
<223> Oligomeric Compound

<400> 151
gccataaata tgtgatactt                                                    20

<210> 152
<211> 20
<212> DNA
<213> Artificial Sequence

<220>
<223> Oligomeric Compound

<400> 152
agaccctcaa tttctctaat                                                    20

<210> 153
<211> 20
<212> DNA
<213> Artificial Sequence

<220>
<223> Oligomeric Compound

<400> 153
tgccatgttt cggtgcagac                                                    20

<210> 154
<211> 20
<212> DNA
<213> Artificial Sequence

<220>
<223> Oligomeric Compound

<400> 154
acctctgcca tgtttcggtg                                                    20

<210> 155
<211> 20
<212> DNA
<213> Artificial Sequence

<220>
<223> Oligomeric Compound

<400> 155
tgacttggac ccaacctctg                                                    20

<210> 156
<211> 20
<212> DNA
<213> Artificial Sequence

<220>
<223> Oligomeric Compound

<400> 156
acagcactga cttggaccca                                                    20

<210> 157
<211> 20
<212> DNA
<213> Artificial Sequence

<220>
<223> Oligomeric Compound

<400> 157
acgaacagca ctgacttgga                                                    20

<210> 158
<211> 20
<212> DNA
<213> Artificial Sequence

<220>
<223> Oligomeric Compound

<400> 158
acacacgaac agcactgact                                                    20

<210> 159
<211> 20
<212> DNA
<213> Artificial Sequence

<220>
<223> Oligomeric Compound

<400> 159
ttaccgagac acacgaacag                                                    20

<210> 160
<211> 20
<212> DNA
<213> Artificial Sequence

<220>
<223> Oligomeric Compound

<400> 160
aatgttaccg agacacacga                                                    20

<210> 161
<211> 20
<212> DNA
<213> Artificial Sequence

<220>
<223> Oligomeric Compound

<400> 161
gcaaatgtta ccgagacaca                                                    20

<210> 162
<211> 20
<212> DNA
<213> Artificial Sequence

<220>
<223> Oligomeric Compound

<400> 162
ggtgaccggc aaatgttacc                                                    20

<210> 163
<211> 20
<212> DNA
<213> Artificial Sequence

<220>
<223> Oligomeric Compound

<400> 163
tgggtgaccg gcaaatgtta                                                    20

<210> 164
<211> 20
<212> DNA
<213> Artificial Sequence

<220>
<223> Oligomeric Compound

<400> 164
caatgggtga ccggcaaatg                                                    20

<210> 165
<211> 20
<212> DNA
<213> Artificial Sequence

<220>
<223> Oligomeric Compound

<400> 165
gcaatgggtg accggcaaat                                                    20

<210> 166
<211> 20
<212> DNA
<213> Artificial Sequence

<220>
<223> Oligomeric Compound

<400> 166
tgcttctgca atgggtgacc                                                    20

<210> 167
<211> 20
<212> DNA
<213> Artificial Sequence

<220>
<223> Oligomeric Compound

<400> 167
atactgcttc tgcaatgggt                                                    20

<210> 168
<211> 20
<212> DNA
<213> Artificial Sequence

<220>

<223> Oligomeric Compound

<400> 168
gaatactgct tctgcaatgg                                                    20

<210> 169
<211> 20
<212> DNA
<213> Artificial Sequence

<220>
<223> Oligomeric Compound

<400> 169
ctgaatactg cttctgcaat                                                    20

<210> 170
<211> 20
<212> DNA
<213> Artificial Sequence

<220>
<223> Oligomeric Compound

<400> 170
ttcctgaata ctgcttctgc                                                    20

<210> 171
<211> 20
<212> DNA
<213> Artificial Sequence

<220>
<223> Oligomeric Compound

<400> 171
accagtttcc tgaatactgc                                                    20

<210> 172
<211> 20
<212> DNA
<213> Artificial Sequence

<220>
<223> Oligomeric Compound

<400> 172
agttaccagt ttcctgaata                                                    20

<210> 173
<211> 20
<212> DNA
<213> Artificial Sequence

<220>
<223> Oligomeric Compound

<400> 173
tcatcagtta ccagtttcct                                                    20

<210> 174
<211> 20
<212> DNA
<213> Artificial Sequence

<220>
<223> Oligomeric Compound

<400> 174
cttttcatca gttaccagtt                                                    20

<210> 175
<211> 20
<212> DNA
<213> Artificial Sequence

<220>
<223> Oligomeric Compound

<400> 175
aaccttttca tcagttacca                                                    20

<210> 176
<211> 20
<212> DNA
<213> Artificial Sequence

<220>
<223> Oligomeric Compound

<400> 176
ttatctgaaa ccttttcatc                                                    20

<210> 177
<211> 20
<212> DNA
<213> Artificial Sequence

<220>
<223> Oligomeric Compound

<400> 177
aattatctga aaccttttca                                                    20

<210> 178
<211> 20
<212> DNA
<213> Artificial Sequence

<220>
<223> Oligomeric Compound

<400> 178
ggcccaatta tctgaaacct                                                    20

<210> 179
<211> 20
<212> DNA
<213> Artificial Sequence

<220>
<223> Oligomeric Compound

<400> 179
atggcccaat tatctgaaac                                                    20

<210> 180
<211> 20
<212> DNA
<213> Artificial Sequence

<220>
<223> Oligomeric Compound

<400> 180
tgctgtcaat ggcccaatta                                                    20

<210> 181
<211> 20
<212> DNA
<213> Artificial Sequence

<220>
<223> Oligomeric Compound

<400> 181
gcgctgctgt caatggccca                                                    20

<210> 182
<211> 20
<212> DNA
<213> Artificial Sequence

<220>
<223> Oligomeric Compound

<400> 182
ggccggccca cgttccagtc                                                    20

<210> 183
<211> 20
<212> DNA
<213> Artificial Sequence

<220>
<223> Oligomeric Compound

<400> 183
gcaaagtctt cttttgtaat                                                    20

<210> 184
<211> 20
<212> DNA
<213> Artificial Sequence

<220>
<223> Oligomeric Compound

<400> 184
aatgtggcaa agtcttcttt                                                    20

<210> 185
<211> 20
<212> DNA
<213> Artificial Sequence

<220>
<223> Oligomeric Compound

<400> 185
taatcgaatg tggcaaagtc                                                    20

<210> 186
<211> 20
<212> DNA

<213> Artificial Sequence

<220>
<223> Oligomeric Compound

<400> 186
gtatataatc gaatgtggca                                                    20

<210> 187
<211> 20
<212> DNA
<213> Artificial Sequence

<220>
<223> Oligomeric Compound

<400> 187
agtatataat cgaatgtggc                                                    20

<210> 188
<211> 20
<212> DNA
<213> Artificial Sequence

<220>
<223> Oligomeric Compound

<400> 188
catacatagt atataatcga                                                    20

<210> 189
<211> 20
<212> DNA
<213> Artificial Sequence

<220>
<223> Oligomeric Compound

<400> 189
ctttcatcca tacatagtat                                                    20

<210> 190
<211> 20
<212> DNA
<213> Artificial Sequence

<220>
<223> Oligomeric Compound

<400> 190
tctctcagat tgctttcatc                                                    20

<210> 191
<211> 20
<212> DNA
<213> Artificial Sequence

<220>
<223> Oligomeric Compound

<400> 191
gatctctcag attgctttca                                                    20

<210> 192
<211> 20

<212> DNA
<213> Artificial Sequence

<220>
<223> Oligomeric Compound

<400> 192
attgagatct ctcagattgc                                                    20

<210> 193
<211> 20
<212> DNA
<213> Artificial Sequence

<220>
<223> Oligomeric Compound

<400> 193
ttctattgag atctctcaga                                                    20

<210> 194
<211> 20
<212> DNA
<213> Artificial Sequence

<220>
<223> Oligomeric Compound

<400> 194
gcagttttta acttgattac                                                    20

<210> 195
<211> 20
<212> DNA
<213> Artificial Sequence

<220>
<223> Oligomeric Compound

<400> 195
aatgatgagc tgtttctgtg                                                    20

<210> 196
<211> 20
<212> DNA
<213> Artificial Sequence

<220>
<223> Oligomeric Compound

<400> 196
agggatcttc aatgatgagc                                                    20

<210> 197
<211> 20
<212> DNA
<213> Artificial Sequence

<220>
<223> Oligomeric Compound

<400> 197
aatagggatc ttcaatgatg                                                    20

<210> 198

<211> 20
<212> DNA
<213> Artificial Sequence

<220>
<223> Oligomeric Compound

<400> 198
cctcgaagtc agagtcattg                                                    20

<210> 199
<211> 20
<212> DNA
<213> Artificial Sequence

<220>
<223> Oligomeric Compound

<400> 199
caccacctcg aagtcagagt                                                    20

<210> 200
<211> 20
<212> DNA
<213> Artificial Sequence

<220>
<223> Oligomeric Compound

<400> 200
tggtacacca cctcgaagtc                                                    20

<210> 201
<211> 20
<212> DNA
<213> Artificial Sequence

<220>
<223> Oligomeric Compound

<400> 201
attgctggta caccacctcg                                                    20

<210> 202
<211> 20
<212> DNA
<213> Artificial Sequence

<220>
<223> Oligomeric Compound

<400> 202
acctcgaagt cagagtcatt                                                    20

<210> 203
<211> 20
<212> DNA
<213> Artificial Sequence

<220>
<223> Oligomeric Compound

<400> 203
ggacccaacc tctgccatgt                                                    20

<210> 204
<211> 20
<212> DNA
<213> Artificial Sequence

<220>
<223> Oligomeric Compound

<400> 204
ctaaggcatt gctggtacac                                                    20

<210> 205
<211> 20
<212> DNA
<213> Artificial Sequence

<220>
<223> Oligomeric Compound

<400> 205
agcacctaag gcattgctgg                                                    20

<210> 206
<211> 20
<212> DNA
<213> Artificial Sequence

<220>
<223> Oligomeric Compound

<400> 206
cttgcagcac ctaaggcatt                                                    20

<210> 207
<211> 20
<212> DNA
<213> Artificial Sequence

<220>
<223> Oligomeric Compound

<400> 207
aaggccttgc agcacctaag                                                    20

<210> 208
<211> 20
<212> DNA
<213> Artificial Sequence

<220>
<223> Oligomeric Compound

<400> 208
ccaggaaggc cttgcagcac                                                    20

<210> 209
<211> 20
<212> DNA
<213> Artificial Sequence

<220>
<223> Oligomeric Compound

<400> 209
cttctccagg aaggccttgc                                                    20

<210> 210
<211> 20
<212> DNA
<213> Artificial Sequence

<220>
<223> Oligomeric Compound

<400> 210
gtgagtcttc tccaggaagg                                                    20

<210> 211
<211> 20
<212> DNA
<213> Artificial Sequence

<220>
<223> Oligomeric Compound

<400> 211
taggaccagc tagtgagtct                                                    20

<210> 212
<211> 20
<212> DNA
<213> Artificial Sequence

<220>
<223> Oligomeric Compound

<400> 212
ctcagtggtg gtggttagga                                                    20

<210> 213
<211> 20
<212> DNA
<213> Artificial Sequence

<220>
<223> Oligomeric Compound

<400> 213
gccaccaccc ttgggcacag                                                    20

<210> 214
<211> 20
<212> DNA
<213> Artificial Sequence

<220>
<223> Oligomeric Compound

<400> 214
ggctaaggac tgccaccacc                                                    20

<210> 215
<211> 20
<212> DNA
<213> Artificial Sequence

<220>
<223> Oligomeric Compound

<400> 215

gatatacagt aagtcagctg 20

<210> 216
<211> 20
<212> DNA
<213> Artificial Sequence

<220>
<223> Oligomeric Compound

<400> 216
acctacaatt attttaaaga 20

<210> 217
<211> 20
<212> DNA
<213> Artificial Sequence

<220>
<223> Oligomeric Compound

<400> 217
tgatttccac ctacaattat 20

<210> 218
<211> 20
<212> DNA
<213> Artificial Sequence

<220>
<223> Oligomeric Compound

<400> 218
atgcctgatt tccacctaca 20

<210> 219
<211> 20
<212> DNA
<213> Artificial Sequence

<220>
<223> Oligomeric Compound

<400> 219
tctgaacaaa tgcctgattt 20

<210> 220
<211> 20
<212> DNA
<213> Artificial Sequence

<220>
<223> Oligomeric Compound

<400> 220
aatgtctgcc tcaaatgttt 20

<210> 221
<211> 20
<212> DNA
<213> Artificial Sequence

<220>
<223> Oligomeric Compound

<400> 221
acctcaaatg tctgcctcaa                                                    20

<210> 222
<211> 20
<212> DNA
<213> Artificial Sequence

<220>
<223> Oligomeric Compound

<400> 222
gagccacacc tcaaatgtct                                                    20

<210> 223
<211> 20
<212> DNA
<213> Artificial Sequence

<220>
<223> Oligomeric Compound

<400> 223
gtctaagaat actgagccac                                                    20

<210> 224
<211> 20
<212> DNA
<213> Artificial Sequence

<220>
<223> Oligomeric Compound

<400> 224
ttgttagtct aagaatactg                                                    20

<210> 225
<211> 20
<212> DNA
<213> Artificial Sequence

<220>
<223> Oligomeric Compound

<400> 225
tatggcgagg ccagagcttt                                                    20

<210> 226
<211> 20
<212> DNA
<213> Artificial Sequence

<220>
<223> Oligomeric Compound

<400> 226
attttgtaat tatggcgagg                                                    20

<210> 227
<211> 20
<212> DNA
<213> Artificial Sequence

<220>
<223> Oligomeric Compound

<400> 227
acagttgctc gttccactat                                                    20

<210> 228
<211> 20
<212> DNA
<213> Artificial Sequence

<220>
<223> Oligomeric Compound

<400> 228
tgttccacag ttgctcgttc                                                    20

<210> 229
<211> 20
<212> DNA
<213> Artificial Sequence

<220>
<223> Oligomeric Compound

<400> 229
ccttgtgggt cattcttact                                                    20

<210> 230
<211> 20
<212> DNA
<213> Artificial Sequence

<220>
<223> Oligomeric Compound

<400> 230
gctgggctca aaggctgatc                                                    20

<210> 231
<211> 20
<212> DNA
<213> Artificial Sequence

<220>
<223> Oligomeric Compound

<400> 231
ttagaccaga ctacccaggc                                                    20

<210> 232
<211> 20
<212> DNA
<213> Artificial Sequence

<220>
<223> Oligomeric Compound

<400> 232
ctcacactcc agttagacca                                                    20

<210> 233
<211> 20
<212> DNA
<213> Artificial Sequence

<220>

<223> Oligomeric Compound

<400> 233
cactgggtgc tggccatgct                                                    20

<210> 234
<211> 20
<212> DNA
<213> Artificial Sequence

<220>
<223> Oligomeric Compound

<400> 234
gtaaggcaag caaacagcac                                                    20

<210> 235
<211> 20
<212> DNA
<213> Artificial Sequence

<220>
<223> Oligomeric Compound

<400> 235
ttgtcacaat aagagacaat                                                    20

<210> 236
<211> 20
<212> DNA
<213> Artificial Sequence

<220>
<223> Oligomeric Compound

<400> 236
ggagatattg tcacaataag                                                    20

<210> 237
<211> 20
<212> DNA
<213> Artificial Sequence

<220>
<223> Oligomeric Compound

<400> 237
ccatggatgg agatattgtc                                                    20

<210> 238
<211> 20
<212> DNA
<213> Artificial Sequence

<220>
<223> Oligomeric Compound

<400> 238
ggctgccatg gatggagata                                                    20

<210> 239
<211> 20
<212> DNA
<213> Artificial Sequence

<220>
<223> Oligomeric Compound

<400> 239
aaatggaagg ctgccatgga                                                    20

<210> 240
<211> 20
<212> DNA
<213> Artificial Sequence

<220>
<223> Oligomeric Compound

<400> 240
agtgttaaat ggaaggctgc                                                    20

<210> 241
<211> 20
<212> DNA
<213> Artificial Sequence

<220>
<223> Oligomeric Compound

<400> 241
ttaaactctc ccagtgttaa                                                    20

<210> 242
<211> 20
<212> DNA
<213> Artificial Sequence

<220>
<223> Oligomeric Compound

<400> 242
ctgggtttaa actctcccag                                                    20

<210> 243
<211> 20
<212> DNA
<213> Artificial Sequence

<220>
<223> Oligomeric Compound

<400> 243
ggttctcctc tctcaaatat                                                    20

<210> 244
<211> 20
<212> DNA
<213> Artificial Sequence

<220>
<223> Oligomeric Compound

<400> 244
agttccaggc ccatcatcac                                                    20

<210> 245
<211> 20
<212> DNA
<213> Artificial Sequence

<220>
<223> Oligomeric Compound

<400> 245
atggcctgct ggagttccag                                                    20

<210> 246
<211> 20
<212> DNA
<213> Artificial Sequence

<220>
<223> Oligomeric Compound

<400> 246
tttttatctt tcagacaggg                                                    20

<210> 247
<211> 20
<212> DNA
<213> Artificial Sequence

<220>
<223> Oligomeric Compound

<400> 247
cccatctgtt agcattttta                                                    20

<210> 248
<211> 20
<212> DNA
<213> Artificial Sequence

<220>
<223> Oligomeric Compound

<400> 248
ctgttgctcc catctgttag                                                    20

<210> 249
<211> 20
<212> DNA
<213> Artificial Sequence

<220>
<223> Oligomeric Compound

<400> 249
ttaacttcac caacctgttg                                                    20

<210> 250
<211> 20
<212> DNA
<213> Artificial Sequence

<220>
<223> Oligomeric Compound

<400> 250
ccaagctcaa gaaactacac                                                    20

<210> 251
<211> 20
<212> DNA

<213> Artificial Sequence

<220>
<223> Oligomeric Compound

<400> 251
aagtggctca aataggaacc                                                    20

<210> 252
<211> 20
<212> DNA
<213> Artificial Sequence

<220>
<223> Oligomeric Compound

<400> 252
ctttctcttt aaagaagcaa                                                    20

<210> 253
<211> 20
<212> DNA
<213> Artificial Sequence

<220>
<223> Oligomeric Compound

<400> 253
gcacacttac tttctcttta                                                    20

<210> 254
<211> 20
<212> DNA
<213> Artificial Sequence

<220>
<223> Oligomeric Compound

<400> 254
caccactatt taagcacact                                                    20

<210> 255
<211> 20
<212> DNA
<213> Artificial Sequence

<220>
<223> Oligomeric Compound

<400> 255
acaaacgcac accactattt                                                    20

<210> 256
<211> 20
<212> DNA
<213> Artificial Sequence

<220>
<223> Oligomeric Compound

<400> 256
gttgatgaga gaacacttac                                                    20

<210> 257
<211> 20

<212> DNA
<213> Artificial Sequence

<220>
<223> Oligomeric Compound

<400> 257
gtaactttgt aaaatgttga                                                    20

<210> 258
<211> 20
<212> DNA
<213> Artificial Sequence

<220>
<223> Oligomeric Compound

<400> 258
ttactcatgc ttgcctgtaa                                                    20

<210> 259
<211> 20
<212> DNA
<213> Artificial Sequence

<220>
<223> Oligomeric Compound

<400> 259
   gtcccttttc tgaaaataca                                                     20

<210> 260
<211> 20
<212> DNA
<213> Artificial Sequence

<220>
<223> Oligomeric Compound

<400> 260
ataaatttga ggtccctttt                                                    20

<210> 261
<211> 20
<212> DNA
<213> Artificial Sequence

<220>
<223> Oligomeric Compound

<400> 261
atatccacat aaatttgagg                                                    20

<210> 262
<211> 20
<212> DNA
<213> Artificial Sequence

<220>
<223> Oligomeric Compound

<400> 262
atcttttctg acatatatcc                                                    20

<210> 263

<211> 20
<212> DNA
<213> Artificial Sequence

<220>
<223> Oligomeric Compound

<400> 263
tctccagtgg caaagacaaa                                                    20

<210> 264
<211> 20
<212> DNA
<213> Artificial Sequence

<220>
<223> Oligomeric Compound

<400> 264
aagcaagaaa ctatgcggga                                                    20

<210> 265
<211> 20
<212> DNA
<213> Artificial Sequence

<220>
<223> Oligomeric Compound

<400> 265
catacggtac ctgccgtgca                                                    20

<210> 266
<211> 20
<212> DNA
<213> Artificial Sequence

<220>
<223> Oligomeric Compound

<400> 266
caatggccca ctgtaacaca                                                    20

<210> 267
<211> 20
<212> DNA
<213> Artificial Sequence

<220>
<223> Oligomeric Compound

<400> 267
gagtacattt gaagttaaaa                                                    20

<210> 268
<211> 20
<212> DNA
<213> Artificial Sequence

<220>
<223> Oligomeric Compound

<400> 268
ctcttgtaat ctacaattaa                                                    20

<210> 269
<211> 20
<212> DNA
<213> Artificial Sequence

<220>
<223> Oligomeric Compound

<400> 269
tatacctgag ttcaaggtca                                                    20

<210> 270
<211> 20
<212> DNA
<213> Artificial Sequence

<220>
<223> Oligomeric Compound

<400> 270
ctcttgtaat ctgtcttgcc                                                    20

<210> 271
<211> 20
<212> DNA
<213> Artificial Sequence

<220>
<223> Oligomeric Compound

<400> 271
gaggtctcga cttacccgct                                                    20

<210> 272
<211> 20
<212> DNA
<213> Artificial Sequence

<220>
<223> Oligomeric Compound

<400> 272
ttctacctcg cgcgatttac                                                    20

<210> 273
<211> 20
<212> DNA
<213> Artificial Sequence

<220>
<223> Oligomeric Compound

<400> 273
ttagaatacg tcgcgttatg                                                    20

<210> 274
<211> 20
<212> DNA
<213> Artificial Sequence

<220>
<223> Oligomeric Compound

<400> 274
ccttccctga aggttcctcc                                                    20

<210> 275

<400> 275
 000

<210> 276

<400> 276
 000

<210> 277

<400> 277
 000

<210> 278

<400> 278
 000

<210> 279
<211> 20
<212> DNA
<213> Artificial Sequence

<220>
<223> Oligomeric Compound

<400> 279
accccgttcc gcacgccccc                                                    20

<210> 280
<211> 20
<212> DNA
<213> Artificial Sequence

<220>
<223> Oligomeric Compound

<400> 280
tagcctgttc cgccatcttc                                                    20

<210> 281
<211> 20
<212> DNA
<213> Artificial Sequence

<220>
<223> Oligomeric Compound

<400> 281
gctcatggga atgccgtgcc                                                    20

<210> 282
<211> 20
<212> DNA
<213> Artificial Sequence

<220>
<223> Oligomeric Compound

<400> 282
atcttctttg gtaatctgcc                                                    20

<210> 283
<211> 20
<212> DNA
<213> Artificial Sequence

<220>
<223> Oligomeric Compound

<400> 283
atagggatct tcaataataa                                                    20

<210> 284
<211> 20
<212> DNA
<213> Artificial Sequence

<220>
<223> Oligomeric Compound

<400> 284
caccgtctca aagtcagagt                                                    20

<210> 285
<211> 20
<212> DNA
<213> Artificial Sequence

<220>
<223> Oligomeric Compound

<400> 285
ccgactgaga aatgcaggac                                                    20

<210> 286
<211> 20
<212> DNA
<213> Artificial Sequence

<220>
<223> Oligomeric Compound

<400> 286
aacaaagagc tggctttggg                                                    20

<210> 287
<211> 20
<212> DNA
<213> Artificial Sequence

<220>
<223> Oligomeric Compound

<400> 287
caaacacaac tgatttccat                                                    20

<210> 288
<211> 20
<212> DNA
<213> Artificial Sequence

<220>
<223> Oligomeric Compound

<400> 288
tgaatcaaac atttttattg                                                    20

<210> 289
<211> 20
<212> DNA
<213> Artificial Sequence

<220>
<223> Oligomeric Compound

<400> 289
ttgttctact attttgtaa                                                    20

<210> 290
<211> 20
<212> DNA
<213> Artificial Sequence

<220>
<223> Oligomeric Compound

<400> 290
gtgaggtttt ccttcattgt                                                   20

<210> 291
<211> 20
<212> DNA
<213> Artificial Sequence

<220>
<223> Oligomeric Compound

<400> 291
actactgtca atccacaaaa                                                   20

<210> 292
<211> 20
<212> DNA
<213> Artificial Sequence

<220>
<223> Oligomeric Compound

<400> 292
tcttccctat cttttcaata                                                   20

<210> 293
<211> 20
<212> DNA
<213> Artificial Sequence

<220>
<223> Oligomeric Compound

<400> 293
gtattgaagg tgccaacgac                                                   20

<210> 294
<211> 20
<212> DNA
<213> Artificial Sequence

<220>
<223> Oligomeric Compound

<400> 294

taagtttcag aggcaaagtg                                                    20

<210> 295
<211> 20
<212> DNA
<213> Artificial Sequence

<220>
<223> Oligomeric Compound

<400> 295
catacaagtg tccttctttc                                                    20

<210> 296
<211> 20
<212> DNA
<213> Artificial Sequence

<220>
<223> Oligomeric Compound

<400> 296
ttattttaaa aaataagcca                                                    20

<210> 297
<211> 20
<212> DNA
<213> Artificial Sequence

<220>
<223> Oligomeric Compound

<400> 297
aaataataac acttttccca                                                    20

<210> 298
<211> 20
<212> DNA
<213> Artificial Sequence

<220>
<223> Oligomeric Compound

<400> 298
aggtttagtt agtctaagaa                                                    20

<210> 299
<211> 20
<212> DNA
<213> Artificial Sequence

<220>
<223> Oligomeric Compound

<400> 299
agaggtttag ttagtctaag                                                    20

<210> 300
<211> 20
<212> DNA
<213> Artificial Sequence

<220>
<223> Oligomeric Compound

<400> 300
tcagaggttt agttagtcta                                                    20

<210> 301
<211> 20
<212> DNA
<213> Artificial Sequence

<220>
<223> Oligomeric Compound

<400> 301
aaggtcagag gtttagttag                                                    20

<210> 302
<211> 20
<212> DNA
<213> Artificial Sequence

<220>
<223> Oligomeric Compound

<400> 302
gcaaggtcag aggtttagtt                                                    20

<210> 303
<211> 20
<212> DNA
<213> Artificial Sequence

<220>
<223> Oligomeric Compound

<400> 303
ccgcaaggtc agaggtttag                                                    20

<210> 304
<211> 20
<212> DNA
<213> Artificial Sequence

<220>
<223> Oligomeric Compound

<400> 304
tttgttccat atttgcttgt                                                    20

<210> 305
<211> 20
<212> DNA
<213> Artificial Sequence

<220>
<223> Oligomeric Compound

<400> 305
atgggtgacc ggcaaatgtt                                                    20

<210> 306
<211> 20
<212> DNA
<213> Artificial Sequence

<220>
<223> Oligomeric Compound

<400> 306
aatgggtgac cggcaaatgt                                                    20

<210> 307
<211> 20
<212> DNA
<213> Artificial Sequence

<220>
<223> Oligomeric Compound

<400> 307
tgcaatgggt gaccggcaaa                                                    20

<210> 308
<211> 20
<212> DNA
<213> Artificial Sequence

<220>
<223> Oligomeric Compound

<400> 308
ctgcaatggg tgaccggcaa                                                    20

<210> 309
<211> 20
<212> DNA
<213> Artificial Sequence

<220>
<223> Oligomeric Compound

<400> 309
tctgcaatgg gtgaccggca                                                    20

<210> 310
<211> 20
<212> DNA
<213> Artificial Sequence

<220>
<223> Oligomeric Compound

<400> 310
ttctgcaatg ggtgaccggc                                                    20

<210> 311
<211> 20
<212> DNA
<213> Artificial Sequence

<220>
<223> Oligomeric Compound

<400> 311
cttctgcaat gggtgaccgg                                                    20

<210> 312
<211> 20
<212> DNA
<213> Artificial Sequence

<220>

<223> Oligomeric Compound

<400> 312
gcttctgcaa tgggtgaccg                                                    20

<210> 313
<211> 20
<212> DNA
<213> Artificial Sequence

<220>
<223> Oligomeric Compound

<400> 313
ctgcttctgc aatgggtgac                                                    20

<210> 314
<211> 20
<212> DNA
<213> Artificial Sequence

<220>
<223> Oligomeric Compound

<400> 314
tactgcttct gcaatgggtg                                                    20

<210> 315
<211> 20
<212> DNA
<213> Artificial Sequence

<220>
<223> Oligomeric Compound

<400> 315
aatactgctt ctgcaatggg                                                    20

<210> 316
<211> 20
<212> DNA
<213> Artificial Sequence

<220>
<223> Oligomeric Compound

<400> 316
tcctgaatac tgcttctgca                                                    20

<210> 317
<211> 20
<212> DNA
<213> Artificial Sequence

<220>
<223> Oligomeric Compound

<400> 317
gtttcctgaa tactgcttct                                                    20

<210> 318
<211> 20
<212> DNA
<213> Artificial Sequence

<220>
<223> Oligomeric Compound

<400> 318
cagtttcctg aatactgctt                                                    20

<210> 319
<211> 20
<212> DNA
<213> Artificial Sequence

<220>
<223> Oligomeric Compound

<400> 319
taccagtttc ctgaatactg                                                    20

<210> 320
<211> 20
<212> DNA
<213> Artificial Sequence

<220>
<223> Oligomeric Compound

<400> 320
gttaccagtt tcctgaatac                                                    20

<210> 321
<211> 20
<212> DNA
<213> Artificial Sequence

<220>
<223> Oligomeric Compound

<400> 321
cagttaccag tttcctgaat                                                    20

<210> 322
<211> 20
<212> DNA
<213> Artificial Sequence

<220>
<223> Oligomeric Compound

<400> 322
tgtatgctcg ctcctcctct                                                    20

<210> 323
<211> 20
<212> DNA
<213> Artificial Sequence

<220>
<223> Oligomeric Compound

<400> 323
atcgaatgtg gcaaagtctt                                                    20

<210> 324
<211> 20
<212> DNA
<213> Artificial Sequence

<220>
<223> Oligomeric Compound

<400> 324
ccggcgcgct cgctccgtct                                                    20

<210> 325
<211> 20
<212> DNA
<213> Artificial Sequence

<220>
<223> Oligomeric Compound

<400> 325
tataatcgaa tgtggcaaag                                                    20

<210> 326
<211> 20
<212> DNA
<213> Artificial Sequence

<220>
<223> Oligomeric Compound

<400> 326
tatataatcg aatgtggcaa                                                    20

<210> 327
<211> 20
<212> DNA
<213> Artificial Sequence

<220>
<223> Oligomeric Compound

<400> 327
tagtatataa tcgaatgtgg                                                    20

<210> 328
<211> 20
<212> DNA
<213> Artificial Sequence

<220>
<223> Oligomeric Compound

<400> 328
atagtatata atcgaatgtg                                                    20

<210> 329
<211> 20
<212> DNA
<213> Artificial Sequence

<220>
<223> Oligomeric Compound

<400> 329
acatagtata taatcgaatg                                                    20

<210> 330
<211> 20
<212> DNA

<213> Artificial Sequence

<220>
<223> Oligomeric Compound

<400> 330
atacatagta tataatcgaa                                                    20

<210> 331
<211> 20
<212> DNA
<213> Artificial Sequence

<220>
<223> Oligomeric Compound

<400> 331
gattgctttc atccatacat                                                    20

<210> 332
<211> 20
<212> DNA
<213> Artificial Sequence

<220>
<223> Oligomeric Compound

<400> 332
cagattgctt tcatccatac                                                    20

<210> 333
<211> 20
<212> DNA
<213> Artificial Sequence

<220>
<223> Oligomeric Compound

<400> 333
tctcagattg ctttcatcca                                                    20

<210> 334
<211> 20
<212> DNA
<213> Artificial Sequence

<220>
<223> Oligomeric Compound

<400> 334
ttgtcgcctc ccgcgtcgtg                                                    20

<210> 335
<211> 20
<212> DNA
<213> Artificial Sequence

<220>
<223> Oligomeric Compound

<400> 335
atctctcaga ttgctttcat                                                    20

<210> 336
<211> 20

<212> DNA
<213> Artificial Sequence

<220>
<223> Oligomeric Compound

<400> 336
agatctctca gattgctttc                                                    20

<210> 337
<211> 20
<212> DNA
<213> Artificial Sequence

<220>
<223> Oligomeric Compound

<400> 337
tgagatctct cagattgctt                                                    20

<210> 338
<211> 20
<212> DNA
<213> Artificial Sequence

<220>
<223> Oligomeric Compound

<400> 338
ctcctgccac atgtagtccg                                                    20

<210> 339
<211> 20
<212> DNA
<213> Artificial Sequence

<220>
<223> Oligomeric Compound

<400> 339
ctgctcgggc ccttattttc                                                    20

<210> 340
<211> 20
<212> DNA
<213> Artificial Sequence

<220>
<223> Oligomeric Compound

<400> 340
cacctcgaag tcagagtcat                                                    20

<210> 341
<211> 20
<212> DNA
<213> Artificial Sequence

<220>
<223> Oligomeric Compound

<400> 341
accacctcga agtcagagtc                                                    20

<210> 342

<211> 20
<212> DNA
<213> Artificial Sequence

<220>
<223> Oligomeric Compound

<400> 342
acaccacctc gaagtcagag                                                    20

<210> 343
<211> 20
<212> DNA
<213> Artificial Sequence

<220>
<223> Oligomeric Compound

<400> 343
tacaccacct cgaagtcaga                                                    20

<210> 344
<211> 20
<212> DNA
<213> Artificial Sequence

<220>
<223> Oligomeric Compound

<400> 344
gtacaccacc tcgaagtcag                                                    20

<210> 345
<211> 20
<212> DNA
<213> Artificial Sequence

<220>
<223> Oligomeric Compound

<400> 345
ggtacaccac ctcgaagtca                                                    20

<210> 346
<211> 20
<212> DNA
<213> Artificial Sequence

<220>
<223> Oligomeric Compound

<400> 346
ctggtacacc acctcgaagt                                                    20

<210> 347
<211> 20
<212> DNA
<213> Artificial Sequence

<220>
<223> Oligomeric Compound

<400> 347
gctggtacac cacctcgaag                                                    20

<210> 348
<211> 20
<212> DNA
<213> Artificial Sequence

<220>
<223> Oligomeric Compound

<400> 348
tgctggtaca ccacctcgaa                                                    20

<210> 349
<211> 20
<212> DNA
<213> Artificial Sequence

<220>
<223> Oligomeric Compound

<400> 349
tacgaccgcg acggccgcgt                                                    20

<210> 350
<211> 20
<212> DNA
<213> Artificial Sequence

<220>
<223> Oligomeric Compound

<400> 350
ttgctggtac accacctcga                                                    20

<210> 351
<211> 20
<212> DNA
<213> Artificial Sequence

<220>
<223> Oligomeric Compound

<400> 351
cgaaggtgtt cgtgttactc                                                    20

<210> 352
<211> 20
<212> DNA
<213> Artificial Sequence

<220>
<223> Oligomeric Compound

<400> 352
gcgttggcgc gtcgtcgctg                                                    20

<210> 353
<211> 20
<212> DNA
<213> Artificial Sequence

<220>
<223> Oligomeric Compound

<400> 353
gggtggtagt ggcgggtggg                                                    20

<210> 354
<211> 20
<212> DNA
<213> Artificial Sequence

<220>
<223> Oligomeric Compound

<400> 354
gtggctgggt ggtgtcgtgt                                                    20

<210> 355
<211> 738
<212> DNA
<213> Mus musculus

<400> 355
aggtcagtta tagactaagc aatctccatc atttagcata tttctggtgc acaggaagca 60
acgtgaggat cactcttcat ctactcccat tacaaagtct accattataa ttttgatatg 120
cttgcattct ctattaattg aaataatata caactcatgt gtatatgtca ttacaaagag 180
ttttgcttca tggaagcatg aaagtatcac atatttatgg cagagataag aattagagaa 240
attgagggtc tgcaccgaaa catggcagag gttgggtcca agtcagtgct gttcgtgtgt 300
ctcggtaaca tttgccggtc acccattgca gaagcagtat tcaggaaact ggtaactgat 360
gaaaaggttt cagataattg ggccattgac agcagcgccg tttccgactg gaacgtgggc 420
cggcccccag acccaagagc tgtcagctgc ctaagaaatc atggcattag cacagcccat 480
aaggcaagac agattacaaa agaagacttt gccacattcg attatatact atgtatggat 540
gaaagcaatc tgagagatct caatagaaaa agtaatcaag ttaaaaactg caaagctaaa 600
attgagctac ttgggagcta tgatccacag aaacagctca tcattgaaga tccctattat 660
ggcaatgact ctgacttcga ggtggtgtac cagcaatgcc ttaggtgctg caaggccttc 720
ctggagaaga cttactag                                                      738

<210> 356
<211> 15
<212> DNA
<213> Artificial Sequence

<220>
<223> Oligomeric Compound

<400> 356
aatgccatga tttct                                                         15

<210> 357
<211> 15
<212> DNA
<213> Artificial Sequence

<220>
<223> Oligomeric Compound

<400> 357
gccatgattt cttag                                                         15

<210> 358
<211> 13
<212> DNA
<213> Artificial Sequence

<220>
<223> Oligomeric Compound

<400> 358
atgccatgat ttc                                                           13

<210> 359
<211> 25
<212> DNA
<213> Artificial Sequence

<220>
<223> Oligomeric Compound

<400> 359
aatgccatga tttcttaggc agctc                                          25

<210> 360
<211> 14
<212> DNA
<213> Artificial Sequence

<220>
<223> Oligomeric Compound

<400> 360
tttcttaggc agct                                                      14

<210> 361
<211> 35
<212> DNA
<213> Artificial Sequence

<220>
<223> Oligomeric Compound

<400> 361
tgtgaatgcc atgatttctt aggcagctca cagct                               35

<210> 362
<211> 27
<212> DNA
<213> Artificial Sequence

<220>
<223> Oligomeric Compound

<400> 362
ccatgatttc ttaggcagct cacagct                                        27

<210> 363
<211> 23
<212> DNA
<213> Artificial Sequence

<220>
<223> Oligomeric Compound

<400> 363
gatttcttag gcagctcaca gct                                            23